# EUROPEAN PATENT SPECIFICATION

(11) **EP 3 478 831 B1**
(45) Date of publication and mention of the grant of the patent: **20.01.2021**
(21) Application number: 17737114.3
(22) Date of filing: 22.06.2017
(51) Int. Cl.: C12N 9/64

(54) **ASPARTIC PROTEASES**
ASPARTAT-PROTEASEN
PROTÉASES ASPARTIQUES

(30) Priority: 30.06.2016 WO PCT/CN2016/087852
(43) Date of publication of application: 08.05.2019
(60) Divisional application: 20212694.2
(73) Proprietor: Danisco US Inc., Palo Alto, California 94304 (US)
(72) Inventor: GU, Xiaogang, Shanghai 200335 (CN); ZOU, Zhengzheng, Shanghai 200031 (CN); XIE, Zhiyong, Shanghai 200335 (CN); HAO, Helong, Shanghai 200335 (CN); YU, Shukun, S-212 40 Malmo (SE); SHIPOVSKOV, Stepan, 8250 Egå (DK)
(74) Representative: Mewburn Ellis LLP
(86) International application number: PCT/US2017/038746
(87) International publication number: WO 2018/005225

(56) References cited:
- WO-A1-2013/135732
- WO-A1-2014/138983

## Description

### FIELD

The field relates to novel aspartic proteases, compositions containing these proteases and uses thereof.

### BACKGROUND

Proteases (also called peptidases or proteinases) are enzymes capable of cleaving peptide bonds. Proteases have evolved multiple times, and different classes of proteases can perform the same reaction by completely different catalytic mechanisms. Proteases can be found in animals, plants, bacteria, archaea and viruses.

Proteolysis can be achieved by enzymes currently classified into six broad groups: aspartic proteases, cysteine proteases, serine proteases, threonine proteases, glutamic proteases, and metalloproteases.

Aspartic proteases (EC 3.4.23), also known as aspartic endopeptidases and aspartyl proteases, use an activated water molecule bound to one or more catalytic aspartate residues to hydrolyze a peptide bond in a polypeptide substrate. Unlike serine or cysteine proteases, aspartic proteases do not form a covalent intermediate during cleavage. Proteolysis therefore occurs in a single step. In general, they have two highly conserved aspartates in the active site and are optimally active at acidic pH. Nearly all known aspartyl proteases are inhibited by pepstatin. An up to date classification of protease evolutionary superfamilies is found in the MEROPS database [http://merops.sanger.ac.uk; Rawlings, N.D., Waller, M., Barrett, A.J. & Bateman, A. (2014) MEROPS: the database of proteolytic enzymes, their substrates and inhibitors. Nucleic Acids Res 42, D503-D509]. In this database, proteases are classified firstly by 'clan' (superfamily) based on structure, mechanism and catalytic residue order. Within each 'clan', proteases are classified into families based on sequence similarity. Each family may contain many hundreds of related proteases.

Five superfamilies (clans) of aspartic proteases are known, each representing an independent evolution of the same active site and mechanisms. Each superfamily contains several families with similar sequences. The clans are the following: Clan AA (e.g. a pepsin family), Clan AC (e.g., a signal peptidase family), Clan AD (e.g., a Presenilin family), Clan AE (e.g., a GPR endopeptidase family), and Clan AF (e.g., an Omptin family).

WO 2014/138983 discloses secreted enzymes from *Paecilomyces byssochlamydoides* including a putative aspartic protease.

WO 2013/135732 discloses an aspartic protease from *Rasamsonia emersonii.*

Acid proteases are widely used in industrial applications including, but not limited to, food, animal feed, producing alcohols, wine production, brewing and in fabric and household care.

Yet, there is a continuing need for proteases for many different applications especially in the food and feed industries.

### SUMMARY

In one aspect, the disclosure provides a recombinant construct comprising at least one regulatory sequence functional in a production host operably linked to a nucleotide sequence encoding an aspartic protease selected from the group consisting of
(a) an aspartic protease having at least 90% sequence identity to SEQ ID NO:2 or SEQ ID NO:7; and
(b) an aspartic protease having at least 90% sequence identity to SEQ ID NO:6, or SEQ ID NO:9.

The production host can be selected from the group consisting of bacteria, fungi, yeast and algae. In yet another aspect the recombinant construct carrying the aspartic protease gene can be chromosomally or extrachromosomally expressed in the production host.

In another aspect, a method for producing an aspartic protease is disclosed which comprises:
(a) transforming a production host with the recombinant construct of described herein; and
(b) culturing the production host of step (a) under conditions whereby the aspartic protease is produced.

In addition, the aspartic protease is optionally recovered from the production host.

In yet another aspect, an aspartic protease-containing culture supernatant can be obtained by the methods provided herein

Also disclosed is a recombinant microbial production host for expressing an aspartic protease wherein the recombinant microbial production host comprises the recombinant construct provided herein. The microbial production host can be selected from the group consisting of bacteria, fungi, yeast and algae.

In another aspect, there is provided the use of an aspartic protease in feed, feedstuff, a feed additive composition or premix wherein the aspartic protease is selected from the group consisting of
(a) an aspartic protease having at least 90% sequence identity to SEQ ID NO:2 or SEQ ID NO:7; and
(b) an aspartic protease having at least 90% sequence identity to SEQ ID NO:6, or SEQ ID NO:9;
wherein the aspartic protease may be used (i) alone or (ii) in combination with a direct fed microbial comprising at least one bacterial strain or (iii) with at least one other enzyme or (iv) in combination with a direct fed microbial comprising at least one bacterial strain and at least one other enzyme.

Also provided is animal feed comprising an aspartic protease selected from the group consisting of (a) an aspartic protease having at least 90% sequence identity to SEQ ID NO:2 or SEQ ID NO:7; and
(b) an aspartic protease having at least 90% sequence identity to SEQ ID NO:6, or SEQ ID NO:9;
wherein the aspartic protease is present in an amount from 1-20g/ton feed and further wherein the aspartic protease may be used (i) alone or (ii) in combination with a direct fed microbial comprising at least one bacterial strain or (iii) with at least one other enzyme or (iv) in combination with a direct fed microbial comprising at least one bacterial strain and at least one other enzyme.

In another aspect, an isolated polypeptide having protease activity is provided, said polypeptide comprising an amino acid sequence of a protease selected from the group consisting of:
(a) an aspartic protease having at least 90% sequence identity to SEQ ID NO:2 or SEQ ID NO:7; and
(b) an aspartic protease having at least 90% sequence identity to SEQ ID NO:6, or SEQ ID NO:9.

In still another aspect, a polynucleotide sequence encoding the polypeptide described herein is provided.

In yet another embodiment, there is disclosed feed additive composition for use in animal feed comprising the polypeptide of described herein and at least one component selected from the group consisting of a protein, a peptide, sucrose, lactose, sorbitol, glycerol, propylene glycol, sodium chloride, sodium sulfate, sodium acetate, sodium citrate, sodium formate, sodium sorbate, potassium chloride, potassium sulfate, potassium acetate, potassium citrate, potassium formate, potassium acetate, potassium sorbate, magnesium chloride, magnesium sulfate, magnesium acetate, magnesium citrate, magnesium formate, magnesium sorbate, sodium metabisulfite, methyl paraben and propyl paraben.

Furthermore, this composition can also be a granulated feed additive composition for use in animal feed comprising the polypeptide described herein, wherein the granulated feed additive composition comprises particles produced by a process selected from the group consisting of high shear granulation, drum granulation, extrusion, spheronization, fluidized bed agglomeration, fluidized bed spray coating, spray drying, freeze drying, prilling, spray chilling, spinning disk atomization, coacervation, tableting, or any combination of the above processes.

The mean diameter of such particles is greater than 50 microns and less than 2000 microns

In addition, the enzyme composition can be in a liquid form and, more specifically in a liquid form suitable for spray-drying on a feed pellet.

### BRIEF DESCRIPTION OF THE DRAWINGS AND SEQUENCES

Figure 1 is a plasmid map of pGXT-RcyPro2.
Figure 2 is a dose response curve of purified RcyPro2, RcoPro2 and GcyPro1.
Figure 3 is a pH profile of purified RcyPro2, RcoPro2 and GcyPro1.
Figure 4 is temperature profile of purified RcyPro2, RcoPro2 and GcyPro1.
Figure 5 is a soycorn meal hydrolysis of purified fungal aspartic proteases at pH3 using OPA assay.
Figure 6 is a soycorn meal hydrolysis of purified fungal aspartic proteases at pH3 using BCA assay.
Figure 7 shows pepsin stability of purified RcyPro2, RcoPro2 and GcyPro1.
Figure 8 shows haze reduction performance of purified RcyPro2, RcoPro2 and GcyPro1.
Figure 9 is a Clustal W multiple sequence alignment of mature regions of RcyPro2, RcoPro2, GcyPro1 and various homologous aspartic fungal proteases.
Figure 10 is a phylogenetic tree displayingRcyPro2, RcoPro2 and GcyPro1 and various aspartic fungal proteases.

The following sequences comply with 37 C.F.R. §§ 1.821-1.825 ("Requirements for Patent Applications Containing Nucleotide Sequences and/or Amino Acid Sequence Disclosures - the Sequence Rules") and are consistent with World Intellectual Property Organization (WIPO) Standard ST.25 (2009) and the sequence listing requirements of the European Patent Convention (EPC) and the Patent Cooperation Treaty (PCT) Rules 5.2 and 49.5(a-bis), and Section 208 and Annex C of the Administrative Instructions. The symbols and format used for nucleotide and amino acid sequence data comply with the rules set forth in 37 C.F.R. § 1.822.

**Table 1. Summary of Nucleotide and Amino Acid SEQ ID Numbers**

| Description | Source Organism for Gene Sequence | Nucleotide SEQ ID NO. | Full length Amino Acid SEQ ID NO. | Mature protease Amino Acid (isolated from *Trichoderma reesei* culture) SEQ ID NO. |
|---|---|---|---|---|
| RcyPro2 | *Rasamsonia cylindrospora* CBS549.62 | 1 | 2 | 7 |
| RcoPro2 | *Rasamsonia composticola* CBS549.92 | 3 | 4 | 8 |
| GcyPro1 | *Geosmithia cylindrospora* NRRL2673 | 5 | 6 | 9 |

### DETAILED DESCRIPTION

In this disclosure, a number of terms and abbreviations are used. The following definitions apply unless specifically stated otherwise.

The articles "a", "an", and "the" preceding an element or component are intended to be nonrestrictive regarding the number of instances (i.e., occurrences) of the element or component. Therefore "a", "an", and "the" should be read to include one or at least one, and the singular word form of the element or component also includes the plural unless the number is obviously meant to be singular.

The term "comprising" means the presence of the stated features, integers, steps, or components as referred to in the claims, but that it does not preclude the presence or addition of one or more other features, integers, steps, components or groups thereof. The term "comprising" is intended to include embodiments encompassed by the terms "consisting essentially of" and "consisting of". Similarly, the term "consisting essentially of" is intended to include embodiments encompassed by the term "consisting of".

Where present, all ranges are inclusive and combinable. For example, when a range of "1 to 5" is recited, the recited range should be construed as including ranges "1 to 4", "1 to 3", "1-2", "1-2 & 4-5", "1-3 & 5", and the like.

The term "protease" means a protein or polypeptide domain of derived from a microorganism, e.g., a fungus, bacterium, or from a plant or animal, and that has the ability to catalyze cleavage of peptide bonds at one or more of various positions of a protein backbone (e.g., E.C. 3.4).

The term "acid protease" means a protease having the ability to hydrolyze proteins under acidic conditions.

The terms "aspartic protease" and "aspartic acid protease" are used interchangeably herein and are a type of acid protease. Aspartic proteases (EC 3.4.23), also known as aspartyl proteases, use an activated water molecule bound to one or more catalytic aspartate residues to hydrolyze a peptide bond in a polypeptide substrates. Generally, they have two highly conserved aspartates in the active site and are optimally active at acidic pH.

The abbreviation "AFP" refers to an aspartic fungal protease, that is, an aspartic protease from a fungal organism source.

The term "direct-fed microbial" ("DFM") as used herein is source of live (viable) naturally occurring microorganisms. A DFM can comprise one or more of such naturally occurring microorganisms such as bacterial strains. Categories of DFMs include Bacillus, Lactic Acid Bacteria and Yeasts. Thus, the term DFM encompasses one or more of the following: direct fed bacteria, direct fed yeast, direct fed yeast and combinations thereof.

Bacilli are unique, gram-positive rods that form spores. These spores are very stable and can withstand environmental conditions such as heat, moisture and a range of pH. These spores germinate into active vegetative cells when ingested by an animal and can be used in meal and pelleted diets. Lactic Acid Bacteria are gram-positive cocci that produce lactic acid which are antagonistic to pathogens. Since Lactic Acid Bacteria appear to be somewhat heat-sensitive, they are not used in pelleted diets. Types of Lactic Acid Bacteria include *Bifidobacterium, Lactobacillus* and *Streptococcus.*

The term "prebiotic" means a non-digestible food ingredient that beneficially affects the host by selectively stimulating the growth and/or the activity of one or a limited number of beneficial bacteria.

The term "probiotic culture" as used herein defines live microorganisms (including bacteria or yeasts for example) which, when for example ingested or locally applied in sufficient numbers, beneficially affects the host organism, i.e. by conferring one or more demonstrable health benefits on the host organism. Probiotics may improve the microbial balance in one or more mucosal surfaces. For example, the mucosal surface may be the intestine, the urinary tract, the respiratory tract or the skin. The term "probiotic" as used herein also encompasses live microorganisms that can stimulate the beneficial branches of the immune system and at the same time decrease the inflammatory reactions in a mucosal surface, for example the gut. Whilst there are no lower or upper limits for probiotic intake, it has been suggested that at least 10⁶-10¹², preferably at least 10⁶-10¹⁰, preferably 10⁸-10⁹, cfu as a daily dose will be effective to achieve the beneficial health effects in a subject.

The term "CFU" as used herein means "colony forming units" and is a measure of viable cells in which a colony represents an aggregate of cells derived from a single progenitor cell.

The term "isolated" means a substance in a form or environment that does not occur in nature. Non-limiting examples of isolated substances include (1) any non-naturally occurring substance, (2) any substance including, but not limited to, any host cell, enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated. The terms "isolated nucleic acid molecule", "isolated polynucleotide", and "isolated nucleic acid fragment" will be used interchangeably and refer to a polymer of RNA or DNA that is single- or double-stranded, optionally containing synthetic, non-natural or altered nucleotide bases. An isolated nucleic acid molecule in the form of a polymer of DNA may be comprised of one or more segments of cDNA, genomic DNA or synthetic DNA.

The term "purified" as applied to nucleic acids or polypeptides generally denotes a nucleic acid or polypeptide that is essentially free from other components as determined by analytical techniques well known in the art (e.g., a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (e.g., percent by weight on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than a starting composition.

As used herein, the term "functional assay" refers to an assay that provides an indication of a protein's activity. In some embodiments, the term refers to assay systems in which a protein is analyzed for its ability to function in its usual capacity. For example, in the case of a protease, a functional assay involves determining the effectiveness of the protease to hydrolyze a proteinaceous substrate.

The terms "peptides", "proteins" and "polypeptides are used interchangeably herein and refer to a polymer of amino acids joined together by peptide bonds. A "protein" or "polypeptide" comprises a polymeric sequence of amino acid residues. The single and 3-letter code for amino acids as defined in conformity with the IUPAC-IUB Joint Commission on Biochemical Nomenclature (JCBN) is used throughout this disclosure. The single letter X refers to any of the twenty amino acids. It is also understood that a polypeptide may be coded for by more than one nucleotide sequence due to the degeneracy of the genetic code. Mutations can be named by the one letter code for the parent amino acid, followed by a position number and then the one letter code for the variant amino acid. For example, mutating glycine (G) at position 87 to serine (S) is represented as "G087S" or "G87S". When describing modifications, a position followed by amino acids listed in parentheses indicates a list of substitutions at that position by any of the listed amino acids. For example, 6(L,I) means position 6 can be substituted with a leucine or isoleucine. At times, in a sequence, a slash (/) is used to define substitutions, e.g. F/V, indicates that the particular position may have a phenylalanine or valine at that position.

A "prosequence" or "propeptide sequence" refers to an amino acid sequence between the signal peptide sequence and mature protease sequence that is necessary for the proper folding and secretion of the protease; they are sometimes referred to as intramolecular chaperones. Cleavage of the prosequence or propeptide sequence results in a mature active protease. Proteases are often expressed as pro-enzymes.

The terms "signal sequence" and "signal peptide" refer to a sequence of amino acid residues that may participate in the secretion or direct transport of the mature or precursor form of a protein. The signal sequence is typically located N-terminal to the precursor or mature protein sequence. The signal sequence may be endogenous or exogenous. A signal sequence is normally absent from the mature protein. A signal sequence is typically cleaved from the protein by a signal peptidase after the protein is transported.

The term "mature" form of a protein, polypeptide, or peptide refers to the functional form of the protein, polypeptide, or enzyme without the signal peptide sequence and propeptide sequence.

The term "precursor" form of a protein or peptide refers to a mature form of the protein having a prosequence operably linked to the amino or carbonyl terminus of the protein. The precursor may also have a "signal" sequence operably linked to the amino terminus of the prosequence. The precursor may also have additional polypeptides that are involved in post-translational activity (e.g., polypeptides cleaved therefrom to leave the mature form of a protein or peptide).

The term "wild-type" in reference to an amino acid sequence or nucleic acid sequence indicates that the amino acid sequence or nucleic acid sequence is a native or naturally-occurring sequence. As used herein, the term "naturally-occurring" refers to anything (e.g., proteins, amino acids, or nucleic acid sequences) that is found in nature. Conversely, the term "non-naturally occurring" refers to anything that is not found in nature (e.g., recombinant nucleic acids and protein sequences produced in the laboratory or modification of the wild-type sequence).

As used herein with regard to amino acid residue positions, "corresponding to" or "corresponds to" or "corresponds" refers to an amino acid residue at the enumerated position in a protein or peptide, or an amino acid residue that is analogous, homologous, or equivalent to an enumerated residue in a protein or peptide. As used herein, "corresponding region" generally refers to an analogous position in a related proteins or a reference protein.

The terms "derived from" and "obtained from" refer to not only a protein produced or producible by a strain of the organism in question, but also a protein encoded by a DNA sequence isolated from such strain and produced in a host organism containing such DNA sequence. Additionally, the term refers to a protein which is encoded by a DNA sequence of synthetic and/or cDNA origin and which has the identifying characteristics of the protein in question.

The term "amino acid" refers to the basic chemical structural unit of a protein or polypeptide. The following abbreviations used herein to identify specific amino acids can be found in Table 2.

**Table 2. One and Three Letter Amino Acid Abbreviations**

| Amino Acid | Three-Letter Abbreviation | One-Letter Abbreviation |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Any amino acid or as defined herein | Xaa | X |

It would be recognized by one of ordinary skill in the art that modifications of amino acid sequences disclosed herein can be made while retaining the function associated with the disclosed amino acid sequences. For example, it is well known in the art that alterations in a gene which result in the production of a chemically equivalent amino acid at a given site, but do not affect the functional properties of the encoded protein are common. For example, any particular amino acid in an amino acid sequence disclosed herein may be substituted for another functionally equivalent amino acid. For the purposes of this disclosure, substitutions are defined as exchanges within one of the following five groups:
1. Small aliphatic, nonpolar or slightly polar residues: Ala, Ser, Thr (Pro, Gly);
2. Polar, negatively charged residues and their amides: Asp, Asn, Glu, Gln;
3. Polar, positively charged residues: His, Arg, Lys;
4. Large aliphatic, nonpolar residues: Met, Leu, Ile, Val (Cys); and
5. Large aromatic residues: Phe, Tyr, and Trp.

Thus, a codon for the amino acid alanine, a hydrophobic amino acid, may be substituted by a codon encoding another less hydrophobic residue (such as glycine) or a more hydrophobic residue (such as valine, leucine, or isoleucine). Similarly, changes which result in substitution of one negatively charged residue for another (such as aspartic acid for glutamic acid) or one positively charged residue for another (such as lysine for arginine) can also be expected to produce a functionally equivalent product. In many cases, nucleotide changes which result in alteration of the N-terminal and C-terminal portions of the protein molecule would also not be expected to alter the activity of the protein. Each of the proposed modifications is well within the routine skill in the art, as is determination of retention of biological activity of the encoded products.

The term "codon optimized", as it refers to genes or coding regions of nucleic acid molecules for transformation of various hosts, refers to the alteration of codons in the gene or coding regions of the nucleic acid molecules to reflect the typical codon usage of the host organism without altering the polypeptide for which the DNA codes.

The term "gene" refers to a nucleic acid molecule that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different from that found in nature. "Endogenous gene" refers to a native gene in its natural location in the genome of an organism. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

The term "coding sequence" refers to a nucleotide sequence which codes for a specific amino acid sequence. "Suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, RNA processing site, effector binding sites, and stem-loop structures.

The term "operably linked" refers to the association of nucleic acid sequences on a single nucleic acid molecule so that the function of one is affected by the other. For example, a promoter is operably linked with a coding sequence when it is capable of affecting the expression of that coding sequence, *i.e.,* the coding sequence is under the transcriptional control of the promoter. Coding sequences can be operably linked to regulatory sequences in sense or antisense orientation.

The terms "regulatory sequence" or "control sequence" are used interchangeably herein and refer to a segment of a nucleotide sequence which is capable of increasing or decreasing expression of specific genes within an organism. Examples of regulatory sequences include, but are not limited to, promoters, signal sequence, operators and the like. As noted above, regulatory sequences can be operably linked in sense or antisense orientation to the coding sequence/gene of interest.

"Promoter" or "promoter sequences" refer to DNA sequences that define where transcription of a gene by RNA polymerase begins. Promoter sequences are typically located directly upstream or at the 5' end of the transcription initiation site.. Promoters may be derived in their entirety from a native or naturally occurring sequence, or be composed of different elements derived from different promoters found in nature, or even comprise synthetic DNA segments. It is understood by those skilled in the art that different promoters may direct the expression of a gene in different tissues or cell type or at different stages of development, or in response to different environmental or physiological conditions ("inducible promoters").

The "3' non-coding sequences" refer to DNA sequences located downstream of a coding sequence and include sequences encoding regulatory signals capable of affecting mRNA processing or gene expression, such as termination of transcription.

The term "transformation" as used herein refers to the transfer or introduction of a nucleic acid molecule into a host organism. The nucleic acid molecule may be introduced as a linear or circular form of DNA. The nucleic acid molecule may be a plasmid that replicates autonomously, or it may integrate into the genome of a production host. Production hosts containing the transformed nucleic acid are referred to as "transformed" or "recombinant" or "transgenic" organisms or "transformants".

The term "recombinant" as used herein refers to an artificial combination of two otherwise separated segments of nucleic acid sequences, e.g., by chemical synthesis or by the manipulation of isolated segments of nucleic acids by genetic engineering techniques. For example, DNA in which one or more segments or genes have been inserted, either naturally or by laboratory manipulation, from a different molecule, from another part of the same molecule, or an artificial sequence, resulting in the introduction of a new sequence in a gene and subsequently in an organism. The terms "recombinant", "transgenic", "transformed", "engineered" or "modified for exogenous gene expression" are used interchangeably herein.

The terms "recombinant construct", "expression construct", " recombinant expression construct" and "expression cassette" are used interchangeably herein. A recombinant construct comprises an artificial combination of nucleic acid fragments, e.g., regulatory and coding sequences that are not all found together in nature. For example, a construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells. The skilled artisan will also recognize that different independent transformation events may result in different levels and patterns of expression (Jones et al., (1985) EMBO J 4:2411-2418; De Almeida et al., (1989) Mol Gen Genetics 218:78-86), and thus that multiple events are typically screened in order to obtain lines displaying the desired expression level and pattern. Such screening may be accomplished standard molecular biological, biochemical, and other assays including Southern analysis of DNA, Northern analysis of mRNA expression, PCR, real time quantitative PCR (qPCR), reverse transcription PCR (RT-PCR), immunoblotting analysis of protein expression, enzyme or activity assays, and/or phenotypic analysis.

The terms "production host", "host" and "host cell" are used interchangeably herein and refer to any organism, or cell thereof, whether human or non-human into which a recombinant construct can be stably or transiently introduced in order to express a gene. This term encompasses any progeny of a parent cell, which is not identical to the parent cell due to mutations that occur during propagation.

The term "percent identity" is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the number of matching nucleotides or amino acids between strings of such sequences. "Identity" and "similarity" can be readily calculated by known methods, including but not limited to those described in: Computational Molecular Biology (Lesk, A. M., ed.) Oxford University Press, NY (1988); Biocomputing: Informatics and Genome Projects (Smith, D. W., ed.) Academic Press, NY (1993); Computer Analysis of Sequence Data, Part I(Griffin, A. M., and Griffin, H. G., eds.) Humana Press, NJ (1994); Sequence Analysis in Molecular Biology (von Heinje, G., ed.) Academic Press (1987); and Sequence Analysis Primer (Gribskov, M. and Devereux, J., eds.) Stockton Press, NY (1991). Methods to determine identity and similarity are codified in publicly available computer programs.

As used herein, "% identity" or percent identity" or "PID" refers to protein sequence identity. Percent identity may be determined using standard techniques known in the art. Useful algorithms include the BLAST algorithms (See, Altschul et al., J Mol Biol, 215:403-410, 1990; and Karlin and Altschul, Proc Natl Acad Sci USA, 90:5873-5787, 1993). The BLAST program uses several search parameters, most of which are set to the default values. The NCBI BLAST algorithm finds the most relevant sequences in terms of biological similarity but is not recommended for query sequences of less than 20 residues (Altschul et al., Nucleic Acids Res, 25:3389-3402, 1997; and Schaffer et al., Nucleic Acids Res, 29:2994-3005, 2001). Exemplary default BLAST parameters for a nucleic acid sequence searches include: Neighboring words threshold = 11; E-value cutoff = 10; Scoring Matrix = NUC.3.1 (match = 1, mismatch = -3);Gap Opening = 5; and Gap Extension = 2. Exemplary default BLAST parameters for amino acid sequence searches include: Word size = 3; E-value cutoff = 10; Scoring Matrix = BLOSUM62; Gap Opening = 11; and Gap extension = 1. A percent (%) amino acid sequence identity value is determined by the number of matching identical residues divided by the total number of residues of the "reference" sequence including any gaps created by the program for optimal/maximum alignment. BLAST algorithms refer to the "reference" sequence as the "query" sequence.

As used herein, "homologous proteins" or "homologous proteases" refers to proteins that have distinct similarity in primary, secondary, and/or tertiary structure. Protein homology can refer to the similarity in linear amino acid sequence when proteins are aligned. Homologous search of protein sequences can be done using BLASTP and PSI-BLAST from NCBI BLAST with threshold (E-value cut-off) at 0.001. (Altschul SF, Madde TL, Shaffer AA, Zhang J, Zhang Z, Miller W, Lipman DJ. Gapped BLAST and PSI BLAST a new generation of protein database search programs. Nucleic Acids Res 1997 Set 1;25(17):3389-402). Using this information, proteins sequences can be grouped. A phylogenetic tree can be built using the amino acid sequences.

Sequence alignments and percent identity calculations may be performed using the Megalign program of the LASERGENE bioinformatics computing suite (DNASTAR Inc., Madison, WI), the AlignX program of Vector NTI v. 7.0 (Informax, Inc., Bethesda, MD), or the EMBOSS Open Software Suite (EMBL-EBI; Rice et al., Trends in Genetics 16, (6):276-277 (2000)). Multiple alignment of the sequences can be performed using the CLUSTAL method (such as CLUSTALW; for example version 1.83) of alignment (Higgins and Sharp, CABIOS, 5:151-153 (1989); Higgins et al., Nucleic Acids Res. 22:4673-4680 (1994); and Chenna et al., Nucleic Acids Res 31 (13):3497-500 (2003)), available from the European Molecular Biology Laboratory via the European Bioinformatics Institute) with the default parameters. Suitable parameters for CLUSTALW protein alignments include GAP Existence penalty=15, GAP extension =0.2, matrix = Gonnet (*e.g*., Gonnet250), protein ENDGAP = -1, protein GAPDIST=4, and KTUPLE=1. In one embodiment, a fast or slow alignment is used with the default settings where a slow alignment. Alternatively, the parameters using the CLUSTALW method (*e.g.*, version 1.83) may be modified to also use KTUPLE =1, GAP PENALTY=10, GAP extension =1, matrix = BLOSUM (*e.g.,* BLOSUM64), WINDOW=5, and TOP DIAGONALS SAVED=5.

Various polypeptide amino acid sequences and polynucleotide sequences are disclosed herein as features of certain aspects. Variants of these sequences that are at least 90% identical, e.g. 90%-95% identical to the sequences disclosed herein may be used in certain embodiments. Alternatively, a variant polypeptide sequence or polynucleotide sequence in certain embodiments can have at least 90%, 91 %, 92%, 93%, 94%, 95%, 96%, 97%, 98% or 99% identity with a sequence disclosed herein. The variant amino acid sequence or polynucleotide sequence has the same function of the disclosed sequence, or at least about 85%, 86%, 87%, 88%, 89%, 90%, 91%, 92%, 93%, 94%, 95%, 96%, 97%, 98%, or 99% of the function of the disclosed sequence.

The term "variant", with respect to a polypeptide, refers to a polypeptide that differs from a specified wild-type, parental, or reference polypeptide in that it includes one or more naturally-occurring or man-made substitutions, insertions, or deletions of an amino acid. Similarly, the term "variant," with respect to a polynucleotide, refers to a polynucleotide that differs in nucleotide sequence from a specified wild-type, parental, or reference polynucleotide. The identity of the wild-type, parental, or reference polypeptide or polynucleotide will be apparent from context.

The terms "plasmid", "vector" and "cassette" refer to an extra chromosomal element often carrying genes that are not part of the central metabolism of the cell, and usually in the form of double-stranded DNA. Such elements may be autonomously replicating sequences, genome integrating sequences, phage, or nucleotide sequences, in linear or circular form, of a single- or double-stranded DNA or RNA, derived from any source, in which a number of nucleotide sequences have been joined or recombined into a unique construction which is capable of introducing a polynucleotide of interest into a cell. "Transformation cassette" refers to a specific vector containing a gene and having elements in addition to the gene that facilitates transformation of a particular host cell. The terms "expression cassette" and "expression vector are used interchangeably herein and refer to a specific vector containing a gene and having elements in addition to the gene that allow for expression of that gene in a host.

The term "expression", as used herein, refers to the production of a functional end-product (e.g., an mRNA or a protein) in either precursor or mature form. Expression may also refer to translation of mRNA into a polypeptide.

Expression of a gene involves transcription of the gene and translation of the mRNA into a precursor or mature protein. "Antisense inhibition" refers to the production of antisense RNA transcripts capable of suppressing the expression of the target protein. "Co-suppression" refers to the production of sense RNA transcripts capable of suppressing the expression of identical or substantially similar foreign or endogenous genes (U.S. Patent No. 5,231,020). "Mature" protein refers to a post-translationally processed polypeptide; i.e., one from which any pre- or propeptides present in the primary translation product have been removed. "Precursor" protein refers to the primary product of translation of mRNA; i.e., with pre- and propeptides still present. Pre-and propeptides may be but are not limited to intracellular localization signals. "Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or DNA-containing organelle, of a host organism resulting in gene expression without integration or stable inheritance. Host organisms containing the transformed nucleic acid fragments are referred to as "transgenic" organisms

The expression vector can be one of any number of vectors or cassettes useful for the transformation of suitable production hosts known in the art. Typically, the vector or cassette will include sequences directing transcription and translation of the relevant gene, a selectable marker, and sequences allowing autonomous replication or chromosomal integration. Suitable vectors generally include a region 5' of the gene which harbors transcriptional initiation controls and a region 3' of the DNA fragment which controls transcriptional termination. Both control regions can be derived from homologous genes to genes of a transformed production host cell and/or genes native to the production host, although such control regions need not be so derived.

Possible initiation control regions or promoters that can be included in the expression vector are numerous and familiar to those skilled in the art. Virtually any promoter capable of driving these genes is suitable, including but not limited to, *CYC1, HIS3, GAL1, GAL10, ADH1, PGK, PHO5, GAPDH, ADC1, TRP1, URA3, LEU2, ENO, TPI* (useful for expression in *Saccharomyces*)*; AOX1* (useful for expression in *Pichia*)*;* and *lac, araB, tet, trp, IP_{L}, IP_{R}, T7, tac,* and *trc* (useful for expression in *Escherichia coli*) as well as the *amy, apr, npr* promoters and various phage promoters useful for expression in *Bacillus.* In some embodiments, the promoter is a constitutive or inducible promoter. A "constitutive promoter" is a promoter that is active under most environmental and developmental conditions. An "inducible" or "repressible" promoter is a promoter that is active under environmental or developmental regulation. In some embodiments, promoters are inducible or repressible due to changes in environmental factors including but not limited to, carbon, nitrogen or other nutrient availability, temperature, pH, osmolarity, the presence of heavy metal(s), the concentration of inhibitor(s), stress, or a combination of the foregoing, as is known in the art. In some embodiments, the inducible or repressible promoters are inducible or repressible by metabolic factors, such as the level of certain carbon sources, the level of certain energy sources, the level of certain catabolites, or a combination of the foregoing as is known in the art. In one embodiment, the promoter is one that is native to the host cell. For example, when *T. reesei* is the host, the promoter is a native *T. reesei* promoter such as the *cbh1* promoter which is deposited in GenBank under Accession Number D86235.

Suitable non-limiting examples of promoters include *cbh1, cbh2, egll, egl2, egl3, egl4, egl5, xynl,* and *xyn2,* repressible acid phosphatase gene (phoA) promoter of *P. chrysogenus* (see e.g., Graessle et al., (1997) Appl. Environ. Microbiol., 63 :753-756), glucose repressible PCK1 promoter (see e.g., Leuker et al., (1997), Gene, 192:235-240), maltoseinducible, glucose-repressible MET3 promoter (see Liu et al., (2006), Eukary. Cell, 5:638-649), pKi promoter and cpc1 promoter. Other examples of useful promoters include promoters from *A. awamori* and *A. niger* glucoamylase genes (see e.g., Nunberg et al., (1984) Mol. Cell Biol. 15 4:2306-2315 and Boel et al., (1984) EMBO J. 3:1581-1585). Also, the promoters of the *T. reesei xln1* gene may be useful (see e.g., EPA 137280A1).

DNA fragments which control transcriptional termination may also be derived from various genes native to a preferred production host cell. In certain embodiments, the inclusion of a termination control region is optional. In certain embodiments, the expression vector includes a termination control region derived from the preferred host cell.

The expression vector can be included in the production host, particularly in the cells of microbial production hosts. The production host cells can be microbial hosts found within the fungal or bacterial families and which grow over a wide range of temperature, pH values, and solvent tolerances. For example, it is contemplated that any of bacteria, algae, and fungi such as filamentous fungi and yeast may suitably host the expression vector.

Inclusion of the expression vector in the production host cell may be used to express the protein of interest so that it may reside intracellularly, extracellularly, or a combination of both inside and outside the cell. Extracellular expression renders recovery of the desired protein from a fermentation product more facile than methods for recovery of protein produced by intracellular expression.

Certain embodiments of the present disclosure relate to a recombinant construct comprising at least one regulatory sequence functional in a production host operably linked to a nucleotide sequence encoding an aspartic protease selected from the group consisting of:
(a) an aspartic protease having at least 90% sequence identity to SEQ ID NO:2 or SEQ ID NO:7; and
(b) an aspartic protease having at least 90% sequence identity to SEQ ID NO:6, or SEQ ID NO:9.

Expression will be understood to include any step involved in production of an aspartic protease including, but not limited to, transcription, post-transcriptional modification, translation, post-translation modification and secretion.

Techniques for modifying nucleic acid sequences utilizing cloning methods are well known in the art.

Regulatory sequences are defined above. They include all components, which are necessary or advantageous for the expression of an aspartic protease. Each control sequence may be native or foreign to the nucleotide sequence encoding the aspartic protease. Such regulatory sequences include, but are not limited to, a leader, a polyadenylation sequence, a propeptide sequence, a promoter, a signal sequence and a transcription terminator. Regulatory sequences may be provided with linkers for the purpose of introducing specific restriction sites facilitating ligation or the regulatory sequences with the coding region of the nucleotide sequence encoding an aspartic protease.

A regulatory sequence may be an appropriate promoter sequence which is recognized by the production host for expression of the nucleotide sequence. The promoter sequence contains a transcriptional control sequence that mediates expression of an aspartic protease. The promoter may be any nucleotide sequence showing transcriptional activity in the production host including mutant, truncated, and hybrid promoters and may be obtained from genes encoding extracellular or intracellular aspartic proteases either homologous or heterologous to the production host. A preferred promoter is *cbhl* derived from *T. Reesei.*

The regulatory sequence may be a suitable transcription terminator, a sequence recognized by the production host to terminate transcription. The terminator sequence is operably linked to the 3' terminus of the nucleotide sequence encoding the aspartic protease. Any terminator which is functional in the production host of choice may be used. In some embodiments, the termination sequence and the promoter sequence are derived from the same source. In other embodiments, the termination sequence is homologous to the host cell. A particularly suitable terminator sequence is *cbh*1 derived from a *Trichoderma* strain and particularly *T. reesei.* Other useful fungal terminators include the terminator from *A. niger* or *A. awamori* glucoamylase gene (see e.g., Nunberg *et al.* (1984) *supra,* and Boel *et al., (1984) supra).*

There can also be mentioned a regulatory sequence that is also a suitable leader sequence. The 5' untranslated region (5' UTR) (also known as a Leader Sequence or Leader RNA) is the region of an mRNA that is directly upstream from the initiation codon. This region is important for the regulation of translation of a transcript which is needed for translation by the production host.

The leader sequence is operably linked to the 5'terminus of the nucleotide sequence encoding the aspartic protease. Any leader sequence which is functional in the production host of choice may be used.

The regulatory sequence may also be a polyadenylation sequence, a sequence which is operably linked to the 3' terminus of the nucleotide sequence and which, when transcribed, is recognized by the production host as a signal to add polyadenosine residues to transcribed mRNA. Any polyadenylation sequence which is functional in the production host of choice may be used.

The regulatory sequence may be a signal peptide coding region, which codes for an amino acid sequence linked to the amino terminus of the aspartic protease which can direct the encoded protease into a cell's secretory pathway. Any signal peptide coding region that directs the expressed aspartic protease into the secretory pathway of the production host may be used.

The regulatory sequence may be a propeptide coding region which codes for an amino acid sequence positioned at the amino terminus of a polypeptide. The resultant polypeptide is known as a proenzyme or a zymogen in some cases. A proenzyme is generally inactive and can be converted to a mature active polypeptide by catalytic or autocatalytic cleavage of the propeptide from the proenzyme to produce the mature active enzyme.

The recombinant construct may also comprise one or more nucleotide sequences which encode one or more factors that are advantageous for directing the expression of an aspartic protease, e.g., a transcriptional activator such as a transacting factor, a chaperone, and a processing protease. Any factor that is functional in the production host of choice may be used. The nucleotide sequence encoding one or more of these factors are not necessarily in tandem with the nucleotide sequence encoding the aspartic protease.

It may also be desirable to add regulatory sequences which allow the regulation of the expression of an aspartic protease relative to the growth of the production host. Examples of regulatory systems are those which cause the expression of the gene to be turned off or on in response to a chemical or physical stimulus, including the presence of a regulatory compound.

For example, with respect to a *Trichoderma* strain, a compound such as sophorose, a sophorose analogue, xylose, and lactose can be used to induce expression.

Also of interest are recombinant expression vectors comprising the nucleotide sequence described herein, encoding:
(a) an aspartic protease having at least 90% sequence identity to SEQ ID NO:2 or SEQ ID NO:7; or
(b) an aspartic protease having at least 90% sequence identity to SEQ ID NO:6, or SEQ ID NO:9;
along with a promoter and transcriptional and translational stop signals.

Various nucleic acid and control sequences are well known to those skilled in the art. Such sequences may be joined together to produce a recombinant expression vector which may include one or more convenient restriction sites to allow for insertion or substitution of the nucleotide sequences encoding an aspartic protease as such sites. Alternatively, the nucleotide sequences encoding an aspartic protease described herein may be expressed by inserting such nucleotide sequences or a recombinant construct comprising the sequence into an appropriate vector for expression. In creating the expression vector, the coding sequence is located in the vector so that the coding sequence is operably linked with the appropriate control sequences for expression.

The recombinant expression vector may be any vector such as a plasmid or virus which can conveniently be subjected to recombinant DNA procedures and lead to expression of the nucleotide sequence. The vector choice will typically depend on the compatibility of the vector with the production host into which the vector is to be introduced. The vectors may be linear or closed circular plasmids. The vector may be an autonomously replicating vector, i.e., a vector, which exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one which, when introduced into the production host, is integrated into the genome and replicated together with the chromosome(s) into which it has been integrated. Some non-limiting examples of such vectors is provided in the Fungal Genetics Stock Center Catalogue of Strains (FGSC, < www.fgsc.net»). Additional examples of suitable expression and/or integration vectors are provided in Sambrook et al., (1989) *supra,* Ausubel (1987) *supra,* van den Hondel et al. (1991) in Bennett and Lasure (Eds.) MORE GENE MANIPULATIONS IN FUNGI, Academic Press. 396-428 and U.S. Patent No. 5,874,276. Particularly useful vectors include pTREX, pFB6, pBR322, PUCI8, pUCI00 and pENTR/D. Suitable plasmids for use in bacterial cells include pBR322 and pUC19 permitting replication in *E.coli* and pE194 for example permitting replication in *Bacillus.*

Briefly with respect to production of an aspartic protease in fungal production host cells reference can be made to Sambrook et al., (1989) *supra,* Ausubel (1987) *supra,* van den Hondel et al. (1991) in Bennett and Lasure (Eds.) MORE GENE MANIPULATIONS IN FUNGI, Academic Press (1991) pp. 70 -76 and 396-428; Nunberg et al., (1984) Mol. Cell Biol. 4:2306-2315; Boel et al., (1984) 30 EMBO J. 3:1581-1585; Finkelstein in BIOTECHNOLOGY OF FILAMENTOUS FUNGI, Finkelstein et al. Eds. Butterworth-Heinemann, Boston, MA (1992), Chap. 6; Kinghorn et al. (1992) APPLIED MOLECULAR GENETICS OF FILAMENTOUS FUNGI, Blackie Academic and Professional, Chapman and Hall, London; Kelley et al., (1985) EMBO J. 4:475 - 479; Penttila et al., (1987) Gene 61: 155-164; and U.S. Patent No. 5,874,276. A list of suitable vectors may be found in the Fungal Genetics Stock Center Catalogue of Strains (FGSC, www at fgsc.net). Suitable vectors include those obtained from for example Invitrogen Life Technologies and Promega. Specific vectors suitable for use in fungal host cells include vectors such as pFB6, pBR322, pUC 18, pUC100, pDON™201, pDONR™221, pENTR™, pGEM®3Z and pGEM®4Z.

The vector system may be a single vector or plasmid or two or more vectors or plasmids which together contain the total DNA to be introduced into the genome of the host cell, or a transposon.

The vector may also contain one or more selectable markers to permit easy selection of the transformed cells. A selectable marker is a gene, the product of which provides for biocide or viral resistance and the like. Examples of selectable markers include ones which confer antimicrobial resistance. Nutritional markers also find use in the present invention including those markers known in the art as *amdS, argB* and *pyr4.* Markers useful for the transformation of *Trichoderma* are known in the art (see, e.g., Finkelstein, chapter 6, in Biotechnology of Filamentous Fungi, Finkelstein et al., EDS Butterworth-Heinemann, Boston MA (1992) and Kinghorn et al., (1992) Applied Molecular Genetics of Filamentous Fungi, Blackie Academic and Professional, Chapman and Hall, London). In some embodiments, the expression vectors will also include a replicon, a gene encoding antibiotic resistance to permit selection of bacteria that harbor recombinant plasmids, and unique restriction sites in nonessential regions of the plasmid to allow insertion of heterologous sequences. The particular antibiotic resistance gene chosen is not critical; any of the many resistance genes known in the art are suitable. The prokaryotic sequences are preferably chosen such that they do not interfere with the replication or integration of the DNA in *Trichoderma reesei.*

The vector may also contain an element(s) permitting stable integration of the vector into the product host genome or autonomous replication of the vector in the production host independent of the genome of the cell. For integration into the host cell genome, the vector may rely on the nucleotide sequence encoding the aspartic protease or any other element of the vector for stable integration of the vector into the genome by homologous or nonhomologous recombination.

For autonomous replication, the vector may further comprise an origin of replication enabling the vector to replicate autonomously in the production host.

More than one copy of the nucleotide sequence encoding an aspartic protease may be inserted into the production host to increase production of the aspartic protease. An increase in the copy number of the nucleotide sequence can be obtained by integrating at least one additional copy of the sequence into the genome of the production host or by including an amplifiable selectable marker gene, and thereby additional copies of the nucleotide sequence can be selected for by culturing the production host cells in the presence of an appropriate selectable agent.

A vector comprising the nucleotide sequence encoding an aspartic protease is introduced into the production host so that the vector is maintained as a chromosomal integrant or as a self-replicating extra-chromosomal vector. Integration is generally considered to be an advantage as the nucleotide sequence is more likely to be stably maintained the production host. Integration of the vector into the production host chromosome may occur by homologous or non-homologous recombination as was discussed above.

Exemplary vectors include, but are not limited to pGXT (the same as the pTTTpyr2 vector as described in published PCT application WO2015/017256). There can also be mentioned standard bacterial expression vectors include bacteriophages λ and M13, as well as plasmids such as pBR322 based plasmids, pSKF, pET23D, and fusion expression systems such as MBP, GST, and LacZ. Epitope tags can also be added to recombinant proteins to provide convenient methods of isolation, e.g., c-myc.

Examples of suitable expression and/or integration vectors are provided in Sambrook et al., (1989) *supra,* Bennett and Lasure (Eds.) More Gene Manipulations in Fungi, (1991) Academic Press pp. 70 - 76 and pp. 396 - 428 and articles cited therein; USP 5,874,276 and Fungal Genetic Stock Center Catalogue of Strains, (FGSC, www.fgsc.net.). Useful vectors may be obtained from Promega and Invitrogen. Some specific useful vectors include pBR322, pUC18, pUC100, pDON™201, pENTR™, pGEN®3Z and pGEN®4Z. However, other forms of expression vectors which serve equivalent functions and which are, or become, known in the art can also be used. Thus, a wide variety of host/expression vector combinations may be employed in expressing the DNA sequences disclosed herein. Useful expression vectors, for example, may consist of segments of chromosomal, non-chromosomal and synthetic DNA sequences such as various known derivatives of SV40 and known bacterial plasmids, e.g., plasmids from E. coli including col E1, pCR1, pBR322, pMb9, pUC 19 and their derivatives, wider host range plasmids, e.g., RP4, phage DNAs e.g., the numerous derivatives of phage .lambda., e.g., NM989, and other DNA phages, e.g., M13 and filamentous single stranded DNA phages, yeast plasmids such as the 2.mu plasmid or derivatives thereof.

The choice of a production host can be any suitable microorganism such as bacteria, fungi, yeast and algae.

Typically, the choice will depend upon the gene encoding the aspartic protease and its source, for example, if derived from a bacterium or a fungus, such as a filamentous fungus.

The term "filamentous fungi" refers to all filamentous forms of the subdivision Eumycotina (See, Alexopoulos, C. J. (1962), INTRODUCTORY MYCOLOGY, Wiley, New York and AINSWORTH AND BISBY DICTIONARY OF THE FUNGI, 9th Ed. (2001) Kirk et al., Eds., CAB International University Press, Cambridge UK). These fungi are characterized by a vegetative mycelium with a cell wall composed of chitin, cellulose, and other complex polysaccharides. The filamentous fungi of the present invention are morphologically, physiologically, and genetically distinct from yeasts. Vegetative growth by filamentous fungi is by hyphal elongation and carbon catabolism is obligatory aerobic.

Non-limiting examples of filamentous fungal host cells include *Trichoderma* spp. (*e.g. T. viride* and *T. reesei,* the asexual morph of *Hypocrea jecorina,* previously classified as *T. longibrachiatum*)*, Penicillium* spp., *Humicola* spp. (*e.g. H. insolens* and *H. grisea*)*, Aspergillus* spp. (*e.g., A. niger, A. nidulans, A. orzyae,* and *A. awamori*)*, Fusarium* spp. (*F. graminum*)*, Neurospora* spp., *Hypocrea* spp. and *Mucor* spp.

There can also be mentioned Strain C1 that was initially classified as a *Chrysosporium lucknowense* based on morphological and growth characteristics of the microorganism, as discussed in detail in U.S. Patent No. 6,015,707, U.S. Patent No. 6,573,086 and patent PCT/NL2010/000045. The C1 strain was subsequently reclassified as *Myceliophthora thermophila* based on genetic tests. *C. lucknowense* has also appeared in the literature as *Sporotrichum thermophile.*

As used herein, the term *"Trichoderma"* or *"Trichoderma sp."* refer to any fungal genus previously or currently classified as *Trichoderma.*

In certain embodiments, the fungal host cell can be a yeast cell. A suitable yeast host organism can be selected from the biotechnologically relevant yeasts species such as but not limited to yeast species such as *Pichia* sp., *Hansenula* sp., or *Kluyveromyces, Yarrowinia, Schizosaccharomyces* species or a species of *Saccharomyces,* including *Saccharomyces cerevisiae* or a species belonging to *Schizosaccharomyces* such as, for example, S. *pombe* species. A strain of the methylotrophic yeast species, *Pichia pastoris,* can be used as the host organism. Alternatively, the host organism can be a *Hansenula* species, *Candida* species or a *Yarrowia* species. In certain embodiments, bacterial host strains include *Escherichia, Bacillus, Kluyveromyces,* and *Pseudomonas.* In some embodiments, the bacterial host cell is *Bacillus subtilis* or *Escherichia coli.* Further host cells may include *Bacillus* spp (*e.g. B. subtilis, B. licheniformis, B. lentus, B. stearothremophilus* and *B. brevis*) and *Streptomyces* spp. (*e.g., S coelicolor* and *S*. *lividans* (TK23 and TK21)).

Examples of algal hosts include, but are not limited to, green
alga *Chlamydomonas reinhardtii* and the blue-green alga *Synechococcus elongatus,* the red alga *Cyanidioschyzon merolae,* and the brown alga *Ectocarpus siliculosus.* Also of interest, is a method for producing an aspartic protease comprising:
(a) transforming a production host with the recombinant construct as described herein; and
(b) culturing the production host of step (a) under conditions whereby the aspartic protease is produced.

Introduction of a DNA construct or vector into a host cell includes techniques such as transformation; electroporation; nuclear microinjection; transduction; transfection, (e.g., lipofection mediated and DEAE-Dextrin mediated transfection); incubation with calcium phosphate DNA precipitate; high velocity bombardment with DNA-coated microprojectiles; and protoplast fusion.

Basic texts disclosing the general methods that can be used include Sambrook et al., Molecular Cloning, A Laboratory Manual (2nd ed. 1989); Kriegler, Gene Transfer and Expression: A Laboratory Manual (1990); and Ausubel et al., eds., Current Protocols in Molecular Biology (1994)). The methods of transformation of the present invention may result in the stable integration of all or part of the transformation vector into the genome of a host cell, such as a filamentous fungal host cell. However, transformation resulting in the maintenance of a self-replicating extra-chromosomal transformation vector is also contemplated.

Many standard transfection methods can be used to produce bacterial and filamentous fungal (e.g. *Aspergillus* or *Trichoderma*) cell lines that express large quantities of the protease. Some of the published methods for the introduction of DNA constructs into cellulase-producing strains of *Trichoderma* include Lorito, Hayes, DiPietro and Harman, (1993) Curr. Genet. 24: 349-356; Goldman, VanMontagu and Herrera-Estrella, (1990) Curr. Genet. 17:169-174; and Penttila, Nevalainen, Ratto, Salminen and Knowles, (1987) Gene 6: 155-164, also see USP 6.022,725; USP 6,268,328 and Nevalainen et al., "The Molecular Biology of Trichoderma and its Application to the Expression of Both Homologous and Heterologous Genes" in Molecular Industrial Mycology, Eds, Leong and Berka, Marcel Dekker Inc., NY (1992) pp 129 - 148; for *Aspergillus* include Yelton, Hamer and Timberlake, (1984) Proc. Natl. Acad. Sci. USA 81: 1470-1474, for *Fusarium* include Bajar, Podila and Kolattukudy, (1991) Proc. Natl. Acad. Sci. USA 88: 8202-8212, for *Streptomyces* include Hopwood et al., 1985, Genetic Manipulation of Streptomyces: Laboratory Manual, The John Innes Foundation, Norwich, UK and Fernandez-Abalos et al., Microbiol 149:1623 - 1632 (2003) and for *Bacillus* include Brigidi, DeRossi, Bertarini, Riccardi and Matteuzzi, (1990) FEMS Microbiol. Lett. 55: 135-138).

However, any of the well-known procedures for introducing foreign nucleotide sequences into host cells may be used. These include the use of calcium phosphate transfection, polybrene, protoplast fusion, electroporation, biolistics, liposomes, microinjection, plasma vectors, viral vectors and any of the other well-known methods for introducing cloned genomic DNA, cDNA, synthetic DNA or other foreign genetic material into a host cell (see, e.g., Sambrook *et al., supra*)*.* Also of use is the Agrobacterium-mediated transfection method described in U.S. Patent No. 6,255,115. It is only necessary that the particular genetic engineering procedure used be capable of successfully introducing at least one gene into the host cell capable of expressing the gene.

Transformation methods for *Aspergillus* and *Trichoderma* are described in, for example, Yelton et al. (1984) Proc. Natl. Acad. Sci. USA 81: 1470 - 1474; Berka et al., (1991) in Applications of Enzyme Biotechnology, Eds. Kelly and Baldwin, Plenum Press (NY); Cao et al., (2000) Sci. 9:991 - 1001; Campbell et al., (1989) Curro Genet. 16:53-56; Pentilla et al., (1987) Gene 61:155 - 164); de Groot et al., (1998) Nat. Biotechnol. 16:839 - 842; USP 6,022,725; USP 6,268,328 and EP 238 023. The expression of heterologous protein in *Trichoderma* is described in USP 6,022,725; USP 6,268,328; Harkki et ale (1991); Enzyme Microb. Technol. 13:227-233; Harkki et al., (1989) Bio Technol. 7:596-603; EP 244,234; EP 215,594; and Nevalainen et al., "The Molecular Biology of Trichoderma and its Application to the Expression of Both Homologous and Heterologous Genes", in MOLECULAR INDUSTRIAL MYCOLOGY, Eds. Leong and Berka, Marcel Dekker Inc., NY (1992) pp. 129 - 148). Reference is also made to WO96100787 *and* Bajar et al., (1991) Proc. Natl. Acad. Sci. USA 88:8202 - 28212 for transformation of *Fusarium* strains.

After the expression vector is introduced into the cells, the transfected or transformed cells are cultured under conditions favoring expression of genes under control of the promoter sequences. In some instances, the promoter sequence is the *cbh1* promoter. Large batches of transformed cells can be cultured as described in Ilmen et al 1997 ("Regulation of cellulase gene expression in the filamentous fungus Trichoderma reesei." Appl. Envir. Microbiol. 63:1298-1306).

Uptake of DNA into the host *Trichoderma* sp. strain depends upon the calcium ion concentration. Generally, between about 10-50 mM CaCl₂ is used in an uptake solution. Additional suitable compounds include a buffering system, such as TE buffer (10 mM Tris, pH 7.4; 1 mM EDTA) or 10 mM MOPS, pH 6.0 and polyethylene glycol. The polyethyleneglycol is believed to fuse the cell membranes, thus permitting the contents of the medium to be delivered into the cytoplasm of the *Trichoderma* sp. strain. This fusion frequently leaves multiple copies the host chromosome.

Usually transformation of *Trichoderma* sp. uses protoplasts or cells that have been subjected to a permeability treatment, typically at a density of 10⁵ to 107/mL, particularly 2x10⁶/mL. A volume of the plasmid DNA integrated into of 100 µL of these protoplasts or cells in an appropriate solution (*e.g.,* 1.2 M sorbitol and 50 mM CaCl₂) may be mixed with the desired DNA. Generally, a high concentration of PEG is added to the uptake solution. From 0.1 to 1 volume of 25% PEG 4000 can be added to the protoplast suspension; however, it is useful to add about 0.25 volumes to the protoplast suspension. Additives, such as dimethyl sulfoxide, heparin, spermidine, potassium chloride and the like, may also be added to the uptake solution to facilitate transformation. Similar procedures are available for other fungal host cells. *See, e.g.,* U.S. Patent No. 6,022,725.

The medium used to cultivate the cells may be any conventional medium suitable for growing the host cell and obtaining expression of an aspartic protease polypeptide. Suitable media and media components are available from commercial suppliers or may be prepared according to published recipes (e.g., as described in catalogues of the American Type Culture Collection).

An aspartic acid polypeptide secreted from the host cells can be used, with minimal post-production processing, as a whole broth preparation.

Depending upon the host cell used post-transcriptional and/or post-translational modifications may be made. One non-limiting example of a post-transcriptional and/or post-translational modification is "clipping" or "truncation" of a polypeptide. For example, this may result in taking an aspartic protease from an inactive or substantially inactive state to an active state as in the case of a pro-peptide undergoing further post-translational processing to a mature peptide having the enzymatic activity. In another instance, this clipping may result in taking a mature aspartic protease polypeptide and further removing N or C-terminal amino acids to generate truncated forms of the aspartic protease that retain enzymatic activity.

Other examples of post-transcriptional or post-translational modifications include, but are not limited to, myristoylation, glycosylation, truncation, lipidation and tyrosine, serine or threonine phosphorylation. The skilled person will appreciate that the type of post-transcriptional or post-translational modifications that a protein may undergo may depend on the host organism in which the protein is expressed.

In some embodiments, the preparation of a spent whole fermentation broth of a recombinant microorganism can be achieved using any cultivation method known in the art resulting in the expression of a polypeptide of interest. Fermentation may, therefore, be understood as comprising shake flask cultivation, small- or large-scale fermentation (including continuous, batch, fed-batch, or solid- state fermentations) in laboratory or industrial fermenters performed in a suitable medium and under conditions allowing an aspartic protease polypeptide, e.g., an aspartic protease) to be expressed or isolated. The term "spent whole fermentation broth" is defined herein as unfractionated contents of fermentation material that includes culture medium, extracellular proteins (e.g., enzymes), and cellular biomass. It is understood that the term "spent whole fermentation broth" also encompasses cellular biomass that has been lysed or permeabilized using methods well known in the art.

Host cells may be cultured under suitable conditions that allow expression of an aspartic protease. Expression of the enzymes may be constitutive such that they are continually produced, or inducible, requiring a stimulus to initiate expression. In the case of inducible expression, protein production can be initiated when required by, for example, addition of an inducer substance to the culture medium, for example dexamethasone or IPTG or sophorose.

Polypeptides can also be produced recombinantly in an *in vitro* cell-free system, such as the TNT™ (Promega) rabbit reticulocyte system. An expression host also can be cultured in the appropriate medium for the host, under aerobic conditions. Shaking or a combination of agitation and aeration can be provided, with production occurring at the appropriate temperature for that host, *e.g.,* from about 25°C to about 75°C (e.g., 30°C to 45°C), depending on the needs of the host and production of the desired aspartic protease. Culturing can occur from about 12 to about 100 hours or greater (and any hour value there between, *e.g.,* from 24 to 72 hours). Typically, the culture broth is at a pH of about 4.0 to about 8.0, again depending on the culture conditions needed for the host relative to production of an aspartic protease. Since production hosts and transformed cells can be cultured in conventional nutrient media. The culture media for transformed host cells may be modified as appropriate for activating promoters and selecting transformed cells. The specific culture conditions, such as temperature, pH and the like, may be those that are used for the host cell selected for expression, and will be apparent to those skilled in the art. In addition, preferred culture conditions may be found in the scientific literature such as Sambrook, (1982) *supra;* Kieser, T, MJ. Bibb, MJ. Buttner, KF Chater, and D.A. Hopwood (2000) PRACTICAL STREPTOMYCES GENETICS. John Innes Foundation, Norwich UK; Harwood, et al., (1990) MOLECULAR BIOLOGICAL METHODS FOR BACILLUS, John Wiley and/or from the American Type Culture Collection (ATCC; www.atcc.org).

Any of the fermentation methods well known in the art can suitably be used to ferment the transformed or the derivative fungal strain as described above. In some embodiments, fungal cells are grown under batch or continuous fermentation conditions.

A classical batch fermentation is a closed system, where the composition of the medium is set at the beginning of the fermentation, and the composition is not altered during the fermentation. At the beginning of the fermentation, the medium is inoculated with the desired organism(s). In other words, the entire fermentation process takes place without addition of any components to the fermentation system throughout.

Alternatively, a batch fermentation qualifies as a "batch" with respect to the addition of the carbon source. Moreover, attempts are often made to control factors such as pH and oxygen concentration throughout the fermentation process. Typically the metabolite and biomass compositions of the batch system change constantly up to the time the fermentation is stopped. Within batch cultures, cells progress through a static lag phase to a high growth log phase and finally to a stationary phase, where growth rate is diminished or halted. Left untreated, cells in the stationary phase would eventually die. In general, cells in log phase are responsible for the bulk of production of product. A suitable variation on the standard batch system is the "fed-batch fermentation" system. In this variation of a typical batch system, the substrate is added in increments as the fermentation progresses. Fed-batch systems are useful when it is known that catabolite repression would inhibit the metabolism of the cells, and/or where it is desirable to have limited amounts of substrates in the fermentation medium. Measurement of the actual substrate concentration in fed-batch systems is difficult and is therefore estimated based on the changes of measurable factors, such as pH, dissolved oxygen and the partial pressure of waste gases, such as CO₂. Batch and fed-batch fermentations are well known in the art.

Continuous fermentation is another known method of fermentation. It is an open system where a defined fermentation medium is added continuously to a bioreactor, and an equal amount of conditioned medium is removed simultaneously for processing. Continuous fermentation generally maintains the cultures at a constant density, where cells are maintained primarily in log phase growth. Continuous fermentation allows for the modulation of one or more factors that affect cell growth and/or product concentration. For example, a limiting nutrient, such as the carbon source or nitrogen source, can be maintained at a fixed rate and all other parameters are allowed to moderate. In other systems, a number of factors affecting growth can be altered continuously while the cell concentration, measured by media turbidity, is kept constant. Continuous systems strive to maintain steady state growth conditions. Thus, cell loss due to medium being drawn off should be balanced against the cell growth rate in the fermentation. Methods of modulating nutrients and growth factors for continuous fermentation processes, as well as techniques for maximizing the rate of product formation, are well known in the art of industrial microbiology.

Separation and concentration techniques are known in the art and conventional methods can be used to prepare a concentrated solution or broth comprising an aspartic protease of the invention.

After fermentation, a fermentation broth is obtained, the microbial cells and various suspended solids, including residual raw fermentation materials, are removed by conventional separation techniques to obtain an aspartic protease solution. Filtration, centrifugation, microfiltration, rotary vacuum drum filtration, ultrafiltration, centrifugation followed by ultra- filtration, extraction, or chromatography, or the like, are generally used.

It may at times be desirable to concentrate a solution or broth comprising an alpha- glucosidase polypeptide to optimize recovery. Use of unconcentrated solutions or broth would typically increase incubation time in order to collect the enriched or purified enzyme precipitate.

The enzyme-containing solution can be concentrated using conventional concentration techniques until the desired enzyme level is obtained. Concentration of the enzyme containing solution may be achieved by any of the techniques discussed herein. Examples of methods of enrichment and purification include but are not limited to rotary vacuum filtration and/or ultrafiltration.

The aspartic protease-containing solution or broth may be concentrated until such time the enzyme activity of the concentrated aspartic protease polypeptide-containing solution or broth is at a desired level.

Concentration may be performed using, *e.g.,* a precipitation agent, such as a metal halide precipitation agent. Metal halide precipitation agents include but are not limited to alkali metal chlorides, alkali metal bromides and blends of two or more of these metal halides.

Exemplary metal halides include sodium chloride, potassium chloride, sodium bromide, potassium bromide and blends of two or more of these metal halides. The metal halide precipitation agent, sodium chloride, can also be used as a preservative. For production scale recovery, aspartic protease polypeptides can be enriched or partially purified as generally described above by removing cells via flocculation with polymers. Alternatively, the enzyme can be enriched or purified by microfiltration followed by concentration by ultrafiltration using available membranes and equipment. However, for some applications, the enzyme does not need to be enriched or purified, and whole broth culture can be lysed and used without further treatment. The enzyme can then be processed, for example, into granules.

Aspartic proteases may be isolated or purified in a variety of ways known to those skilled in the art depending on what other components are present in the sample. Standard purification methods include, but are not limited to, chromatography (e.g., ion exchange, affinity, hydrophobic, chromoatofocusing, immunological and size exclusion), electrophoretic procedures (e.g., preparative isoelectric focusing), differential solubility (e.g., ammonium sulfate precipitation), extraction microfiltration, two phase separations. For example, the protein of interest may be purified using a standard anti-protein of interest antibody column. Ultrafiltration and diafiltration techniques, in conjunction with protein concentration, are also useful. For general guidance in suitable purification techniques, see Scopes, Protein Purification (1982). The degree of purification necessary will vary depending on the use of the protein of interest. In some instances, no purification will be necessary.

Assays for detecting and measuring the enzymatic activity of an enzyme, such as a fungal aspartic protease polypeptide of the invention, are well known. Various assays for detecting and measuring activity of proteases (e.g., fungal aspartic protease polypeptides of the invention), are also known to those of ordinary skill in the art. In particular, assays are available for measuring protease activity that are based on the release of acid-soluble peptides from casein or hemoglobin, measured as absorbance at 280 nm or colorimetrically using the Folin method, and hydrolysis of the dye-labeled azocasein, measured as absorbance at 440-450 nm

Other exemplary assays involve the solubilization of chromogenic substrates (See e.g., Ward, "Proteinases," in Fogarty (ed.)., Microbial Enzymes and Biotechnology, Applied Science, London, [1983], pp. 251-317). A protease detection assay method using highly labeled fluorescein isothiocyanate (FITC) casein as the substrate, a modified version of the procedure described by Twining [Twining, S.S., (1984) "Fluorescein Isothiocyanate-Labeled Casein Assay for Proteolytic Enzymes" Anal. Biochem. 143:30-34] may also be used.

Other exemplary assays include, but are not limited to: cleavage of casein into trichloroacetic acid-soluble peptides containing tyrosine and tryptophan residues, followed by reaction with Folin-Ciocalteu reagent and colorimetric detection of products at 660 nm, cleavage of internally quenched FRET (Fluorescence Resonance Energy Transfer) peptide substrates followed by detection of product using a fluorometer. Fluorescence Resonance Energy Transfer (FRET) is the non-radiative transfer of energy from an excited fluorophore (or donor) to a suitable quencher (or acceptor) molecule. FRET is used in a variety of applications including the measurement of protease activity with substrates, in which the fluorophore is separated from the quencher by a short peptide sequence containing the enzyme cleavage site. Proteolysis of the peptide results in fluorescence as the fluorophore and quencher are separated.

Numerous additional references known to those in the art provide suitable methods (See e.g., Wells et al., Nucleic Acids Res. 11:7911-7925 [1983]; Christianson et al., Anal. Biochem. 223:119 -129 [1994]; and Hsia et al., Anal Biochem. 242:221-227 [1999]).

In still another aspect, the aspartic proteases described herein can be used in any of a feed, feedstuff, a feed additive composition or a premix wherein the aspartic protease is selected from the group consisting of
(a) an aspartic protease having at least 90% sequence identity to SEQ ID NO:2 or SEQ ID NO:7; and
(b) an aspartic protease having at least 90% sequence identity to SEQ ID NO:6, or SEQ ID NO:9;
wherein the aspartic protease may be used (i) alone or (ii) in combination with a direct fed microbial comprising at least one bacterial strain or (iii) with at least one other enzyme or (iv) in combination with a direct fed microbial comprising at least one bacterial strain and at least one other enzyme.

At least one DFM may comprise at least one viable microorganism such as a viable bacterial strain or a viable yeast or a viable fungus. Preferably, the DFM comprises at least one viable bacteria.

It is possible that the DFM may be a spore forming bacterial strain and hence the term DFM may be comprised of or contain spores, e.g. bacterial spores. Thus, the term "viable microorganism" as used herein may include microbial spores, such as endospores or conidia. Alternatively, the DFM in the feed additive composition described herein may not comprise of or may not contain microbial spores, e.g. endospores or conidia.

The microorganism may be a naturally-occurring microorganism or it may be a transformed microorganism.

A DFM as described herein may comprise microorganism from one or more of the following genera: *Lactobacillus, Lactococcus, Streptococcus, Bacillus, Pediococcus, Enterococcus, Leuconostoc, Carnobacterium, Propionibacterium, Bifidobacterium, Clostridium* and *Megasphaera* and combinations thereof.

Preferably, the DFM comprises one or more bacterial strains selected from the following *Bacillus* spp: *Bacillus subtilis, Bacillus cereus, Bacillus licheniformis, Bacillus pumilis* and *Bacillus amyloliquefaciens.*

The genus "Bacillus", as used herein, includes all species within the genus "Bacillus," as known to those of skill in the art, including but not limited to *B*. *subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. gibsonii, B. pumilis* and *B*. *thuringiensis.* It is recognized that the genus Bacillus continues to undergo taxonomical reorganization. Thus, it is intended that the genus include species that have been reclassified, including but not limited to such organisms as *Bacillus stearothermophilus,* which is now named *"Geobacillus stearothermophilus",* or *Bacillus polymyxa,* which is now *"Paenibacillus polymyxa"* The production of resistant endospores under stressful environmental conditions is considered the defining feature of the genus Bacillus, although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*
In another aspect, the DFM may be further combined with the following *Lactococcus* spp: *Lactococcus cremoris* and *Lactococcus lactis* and combinations thereof.

The DFM may be further combined with the following *Lactobacillus* spp: *Lactobacillus buchneri, Lactobacillus acidophilus, Lactobacillus casei, Lactobacillus kefiri, Lactobacillus bifidus, Lactobacillus brevis, Lactobacillus helveticus, Lactobacillus paracasei, Lactobacillus rhamnosus, Lactobacillus salivarius, Lactobacillus curvatus, Lactobacillus bulgaricus, Lactobacillus sakei, Lactobacillus reuteri, Lactobacillus fermentum, Lactobacillus farciminis, Lactobacillus lactis, Lactobacillus delbreuckii, Lactobacillus plantarum, Lactobacillus paraplantarum, Lactobacillus farciminis, Lactobacillus rhamnosus, Lactobacillus crispatus, Lactobacillus gasseri, Lactobacillus johnsonii* and *Lactobacillus jensenii,* and combinations of any thereof.

In still another aspect, the DFM may be further combined with the following *Bifidobacteria* spp: *Bifidobacterium lactis, Bifidobacterium bifidium, Bifidobacterium longum, Bifidobacterium animalis, Bifidobacterium breve, Bifidobacterium infantis, Bifidobacterium catenulatum, Bifidobacterium pseudocatenulatum, Bifidobacterium adolescentis,* and *Bifidobacterium angulatum,* and combinations of any thereof.

There can be mentioned bacteria of the following species: *Bacillus subtilis, Bacillus licheniformis, Bacillus amyloliquefaciens, Bacillus pumilis, Enterococcus, Enterococcus* spp, and *Pediococcus* spp, *Lactobacillus* spp, *Bifidobacterium* spp, *Lactobacillus acidophilus, Pediococsus acidilactici, Lactococcus lactis, Bifidobacterium bifidum, Bacillus subtilis, Propionibacterium thoenii, Lactobacillus farciminis, Lactobacillus rhamnosus, Megasphaera elsdenii, Clostridium butyricum, Bifidobacterium animalis* ssp. *animalis, Lactobacillus reuteri, Bacillus cereus, Lactobacillus salivarius* ssp. *Salivarius, Propionibacteria sp* and combinations thereof.

A direct-fed microbial described herein comprising one or more bacterial strains may be of the same type (genus, species and strain) or may comprise a mixture of genera, species and/or strains.

Alternatively, a DFM may be combined with one or more of the products or the microorganisms contained in those products disclosed in WO2012110778, and summarized as follows:
*Bacillus subtilis* strain 2084 Accession No. NRRI B-50013, *Bacillus subtilis* strain LSSAO1 Accession No. NRRL B-50104, and *Bacillus subtilis* strain 15A-P4 ATCC Accession No. PTA-6507 (from Enviva Pro®. (formerly known as Avicorr®); *Bacillus subtilis* Strain C3102 (from Calsporin®); *Bacillus subtilis* Strain PB6 (from Clostat®); *Bacillus pumilis* (8G-134); *Enterococcus* NCIMB 10415 (SF68) (from Cylactin®); *Bacillus subtilis* Strain C3102 (from Gallipro® & GalliproMax®); *Bacillus licheniformis* (from Gallipro®Tect®); *Enterococcus* and *Pediococcus* (from Poultry star®); *Lactobacillus, Bifidobacterium* and/or *Enterococcus* from Protexin®); *Bacillus subtilis* strain QST 713 (from Proflora®); *Bacillus amyloliquefaciens* CECT-5940 (from Ecobiol® & Ecobiol® Plus); *Enterococcus faecium* SF68 (from Fortiflora®); *Bacillus subtilis* and *Bacillus licheniformis* (from BioPlus2B®); Lactic acid bacteria 7 *Enterococcus faecium* (from Lactiferm®); *Bacillus* strain (from CSI®); *Saccharomyces cerevisiae* (from Yea-Sacc®); *Enterococcus* (from Biomin IMB52®); *Pediococcus acidilactici, Enterococcus, Bifidobacterium animalis* ssp. *animalis, Lactobacillus reuteri, Lactobacillus salivarius* ssp. *salivarius* (from Biomin C5®); *Lactobacillus farciminis* (from Biacton®); *Enterococcus* (from Oralin E1707®); *Enterococcus* (2 strains), *Lactococcus lactis* DSM 1103(from Probios-pioneer PDFM®); *Lactobacillus rhamnosus and Lactobacillus farciminis* (from Sorbiflore®); *Bacillus subtilis* (from Animavit®); *Enterococcus* (from Bonvital®); *Saccharomyces cerevisiae* (from Levucell SB 20®); *Saccharomyces cerevisiae* (from Levucell SC 0 & SC10® ME); *Pediococcus acidilacti* (from Bactocell); *Saccharomyces cerevisiae* (from ActiSaf® (formerly BioSaf®)); *Saccharomyces cerevisiae* NCYC Sc47 (from Actisaf® SC47); *Clostridium butyricum* (from Miya-Gold®); *Enterococcus* (from Fecinor and Fecinor Plus®); *Saccharomyces cerevisiae* NCYC R-625 (from InteSwine®); *Saccharomyces cerevisia* (from BioSprint®); *Enterococcus* and *Lactobacillus rhamnosus* (from Provita®); *Bacillus subtilis* and *Aspergillus oryzae* (from PepSoyGen-C®); *Bacillus cereus* (from Toyocerin®); *Bacillus cereus* var. *toyoi* NCIMB 40112/CNCM 1-1012 (from TOYOCERIN®), or other DFMs such as *Bacillus licheniformis* and *Bacillus subtilis* (from BioPlus® YC) and *Bacillus subtilis* (from GalliPro®).

The DFM may be combined with Enviva® PRO which is commercially available from Danisco A/S. Enviva Pro® is a combination of *Bacillus* strain 2084 Accession No. NRRI B-50013, *Bacillus* strain LSSAO1 Accession No. NRRL B-50104 and *Bacillus* strain 15A-P4 ATCC Accession No. PTA-6507 (as taught in US 7,754,469 B).

It is also possible to combine the DFM described herein with a yeast from the genera: *Saccharomyces spp.*

Preferably, the DFM described herein comprises microorganisms which are generally recognized as safe (GRAS) and, preferably are GRAS-approved.

A person of ordinary skill in the art will readily be aware of specific species and/or strains of microorganisms from within the genera described herein which are used in the food and/or agricultural industries and which are generally considered suitable for animal consumption.

In some embodiments, it is important that the DFM be heat tolerant, i.e. is thermotolerant. This is particularly the case when the feed is pelleted. Therefore, in another embodiment, the DFM may be a thermotolerant microorganism, such as a thermotolerant bacteria,_including for example *Bacillus* spp.

In other aspects, it may be desirable that the DFM comprises a spore producing bacteria, such as *Bacilli,* e.g. *Bacillus* spp. Bacilli can form stable endospores when conditions for growth are unfavorable and are very resistant to heat, pH, moisture and disinfectants.

The DFM described herein may decrease or prevent intestinal establishment of pathogenic microorganism (such as *Clostridium perfringens* and/or *E*. *coli* and/or *Salmonella* spp and/or *Campylobacter* spp.). In other words, the DFM may be antipathogenic. The term "antipathogenic" as used herein means the DFM counters an effect (negative effect) of a pathogen.

As described above, the DFM may be any suitable DFM. For example, the following assay "DFM ASSAY" may be used to determine the suitability of a microorganism to be a DFM. The DFM assay as used herein is explained in more detail in US2009/0280090. For avoidance of doubt, the DFM selected as an inhibitory strain (or an antipathogenic DFM) in accordance with the "DFM ASSAY" taught herein is a suitable DFM for use in accordance with the present disclosure, i.e. in the feed additive composition according to the present disclosure.

Tubes were seeded each with a representative pathogen (e.g., bacteria) from a representative cluster.

Supernatant from a potential DFM, grown aerobically or anaerobically, is added to the seeded tubes (except for the control to which no supernatant is added) and incubated. After incubation, the optical density (OD) of the control and supernatant treated tubes was measured for each pathogen.

Colonies of (potential DFM) strains that produced a lowered OD compared with the control (which did not contain any supernatant) can then be classified as an inhibitory strain (or an antipathogenic DFM). Thus, The DFM assay as used herein is explained in more detail in US2009/0280090.

Preferably, a representative pathogen used in this DFM assay can be one (or more) of the following: Clostridium, such as *Clostridium perfringens* and/or *Clostridium difficile,* and/or *E. coli* and/or *Salmonella* spp and/or *Campylobacter* spp. In one preferred embodiment, the assay is conducted with one or more of *Clostridium perfringens* and/or *Clostridium difficile* and/or *E*. *coli,* preferably *Clostridium perfringens* and/or *Clostridium difficile,* more preferably *Clostridium perfringens.*

Antipathogenic DFMs include one or more of the following bacteria and are described in WO2013029013.:
*Bacillus subtilis* strain 3BP5 Accession No. NRRL B-50510,
*Bacillus subtilis* strain 918 ATCC Accession No. NRRL B-50508, and
*Bacillus subtilis* strain 1013 ATCC Accession No. NRRL B-50509.

DFMs may be prepared as culture(s) and carrier(s) (where used) and can be added to a ribbon or paddle mixer and mixed for about 15 minutes, although the timing can be increased or decreased. The components are blended such that a uniform mixture of the cultures and carriers result. The final product is preferably a dry, flowable powder. The DFM(s) comprising one or more bacterial strains can then be added to animal feed or a feed premix, added to an animal's water, or administered in other ways known in the art (preferably simultaneously with the enzymes described herein. Inclusion of the individual strains in the DFM mixture can be in proportions varying from 1% to 99% and, preferably, from 25% to 75%

Suitable dosages of the DFM in animal feed may range from about 1x10³ CFU/g feed to about 1x10¹⁰ CFU/g feed, suitably between about 1x10⁴ CFU/g feed to about 1x10⁸ CFU/g feed, suitably between about 7.5x10⁴ CFU/g feed to about 1x10⁷ CFU/g feed.

In another aspect, the DFM may be dosed in feedstuff at more than about 1x10³ CFU/g feed, suitably more than about 1x10⁴ CFU/g feed, suitably more than about 5x10⁴ CFU/g feed, or suitably more than about 1x10⁵ CFU/g feed.

The DFM may be dosed in a feed additive composition from about 1x10³ CFU/g composition to about 1x10¹³ CFU/g composition, preferably 1x10⁵ CFU/g composition to about 1x10¹³ CFU/g composition, more preferably between about 1x10⁶ CFU/g composition to about 1x10¹² CFU/g composition, and most preferably between about 3.75x10⁷ CFU/g composition to about 1x10¹¹ CFU/g composition. In another aspect, the DFM may be dosed in a feed additive composition at more than about 1x10⁵ CFU/g composition, preferably more than about 1x10⁶ CFU/g composition, and most preferably more than about 3.75x10⁷ CFU/g composition. In one embodiment, the DFM is dosed in the feed additive composition at more than about 2x10⁵ CFU/g composition, suitably more than about 2x10⁶ CFU/g composition, suitably more than about 3.75x10⁷ CFU/g composition.

The at least one enzyme can be selected from, but is not limited to, enzymes such as, e.g., alpha-amylase, amyloglucosidase, phytase, pullulanase, beta-glucanase, cellulase, xylanase, etc.

Any of these enzymes can be used in an amount ranging from 0.5 to 500 micrograms/g feed or feedstock.

Alpha-amylases (alpha-1,4-glucan-4-glucanohydrolase, EC 3.2.1.1.) hydrolyze internal alpha-1,4-glucosidic linkages in starch, largely at random to produce smaller molecular weight dextrans. These polypeptides are used, *inter alia,* in starch processing and in alcohol production. Any alpha-amylases can be used, e.g., those described in U.S. Patent Nos. 8,927,250 and 7,354,752.

Amyloglucosidase catalyzes the hydrolysis of terminal 1,4-linked alpha-D-glucose residues successively from the non-reducing ends of maltooligo- and polysaccharides with release of beta-D-glucose. Any amyloglucosidase can be used.

Phytase refers to a protein or polypeptide which is capable of catalyzing the hydrolysis of phytate to (1) myo-inositol and/or (2) mono-, di-, tri-, tetra-, and/or penta-phosphates thereof and (3) inorganic phosphate. For example, enzymes having catalytic activity as defined in Enzyme Commission EC number 3.1.3.8 or EC number 3.1.3.26. Any phytase can be used such as described in U.S. Patent Nos. 8,144,046, 8,673,609, and 8,053,221.

Pullulanase (EC 3.2.1.41) is a specific kind of glucanase, an amylolytic exoenzyme that degrades pullan (a polysaccharide polymer consisting of maltotriose units, also known as alpha-1,4-; alpha-1,6-glucan. Thus, it is an example of a debranching enzyme. Pullulanase is also known as pullulan-6-glucanohydrolase. Pullulanases are generally secreted by a *Bacillus* species. For example, *Bacillus deramificans* (US Patent No. 5,817,498; 1998), *Bacillus acidopullulyticus* (European Patent No. 0 063 909) and *Bacillus naganoensis* (US Patent No. 5,055,403). Enzymes having pullulanase activity used commercially are produced, for example, from *Bacillus* species (trade name OPITMAX® 1-100 from DuPont-Genencor and Promozyme® D2 from Novozymes). Other examples of debranching enzymes include, but are not limited to, iso-amylase from *Sulfolobus solfataricus, Pseudomonas* sp. and thermostable pullulanase from *Fervidobacterium nodosum* (e.f., WO2010/76113). The iso-amylase from *Pseudomonas* sp. is available as purified enzyme from Megazyme International. Any pullulanase can be used.

Glucanases are enzymes that break down a glucan, a polysaccharide made several glucose sub-units. As they perform hydrolysis of the glucosidic bond, they are hydrolases.

Beta-glucanase enzymes (EC 3.2.1.4) digests fiber. It helps in the breakdown of plant walls (cellulose).

Cellulases are any of several enzymes produced by fungi, bacteria and protozoans that catalyze cellulolysis, the decomposition of cellulose and of some related polysaccharides. The name is also used for any naturally-occurring mixture or complex of various such enzymes, that act serially or synergistically to decompose cellulosic material. Any cellulases can be used.

Xylanase (EC 3.2.1.8) is the name given to a class of enzymes which degrade the linear polysaccharide beta-1,4-xylan into xylose, those breaking down hemicellulose, one of the major components of plant cell walls. Any xylanases can be used.

Animal feeds may include plant material such as corn, wheat, sorghum, soybean, canola, sunflower or mixtures of any of these plant materials or plant protein sources for poultry, pigs, ruminants, aquaculture and pets. It is contemplated that animal performance parameters, such as growth, feed intake and feed efficiency, but also improved uniformity, reduced ammonia concentration in the animal house and consequently improved welfare and health status of the animals will be improved. More specifically, as used herein, "animal performance" may be determined by the feed efficiency and/or weight gain of the animal and/or by the feed conversion ratio and/or by the digestibility of a nutrient in a feed (e.g. amino acid digestibility) and/or digestible energy or metabolizable energy in a feed and/or by nitrogen retention and/or by animal's ability to avoid the negative effects of necrotic enteritis and/or by the immune response of the subject.

Preferably "animal performance" is determined by feed efficiency and/or weight gain of the animal and/or by the feed conversion ratio.

By "improved animal performance" it is meant that there is increased feed efficiency, and/or increased weight gain and/or reduced feed conversion ratio and/or improved digestibility of nutrients or energy in a feed and/or by improved nitrogen retention and/or by improved ability to avoid the negative effects of necrotic enteritis and/or by an improved immune response in the subject resulting from the use of feed additive composition of the present invention in feed in comparison to feed which does not comprise said feed additive composition.

Preferably, by "improved animal performance" it is meant that there is increased feed efficiency and/or increased weight gain and/or reduced feed conversion ratio. As used herein, the term "feed efficiency" refers to the amount of weight gain in an animal that occurs when the animal is fed ad-libitum or a specified amount of food during a period of time.

By "increased feed efficiency" it is meant that the use of a feed additive composition according the present invention in feed results in an increased weight gain per unit of feed intake compared with an animal fed without said feed additive composition being present.

As used herein, the term "feed conversion ratio" refers to the amount of feed fed to an animal to increase the weight of the animal by a specified amount.

An improved feed conversion ratio means a lower feed conversion ratio.

By "lower feed conversion ratio" or "improved feed conversion ratio" it is meant that the use of a feed additive composition in feed results in a lower amount of feed being required to be fed to an animal to increase the weight of the animal by a specified amount compared to the amount of feed required to increase the weight of the animal by the same amount when the feed does not comprise said feed additive composition.

Nutrient digestibility as used herein means the fraction of a nutrient that disappears from the gastro-intestinal tract or a specified segment of the gastro-intestinal tract, e.g. the small intestine. Nutrient digestibility may be measured as the difference between what is administered to the subject and what comes out in the faeces of the subject, or between what is administered to the subject and what remains in the digesta on a specified segment of the gastro intestinal tract, e.g. the ileum.

Nutrient digestibility as used herein may be measured by the difference between the intake of a nutrient and the excreted nutrient by means of the total collection of excreta during a period of time; or with the use of an inert marker that is not absorbed by the animal, and allows the researcher calculating the amount of nutrient that disappeared in the entire gastro-intestinal tract or a segment of the gastro-intestinal tract. Such an inert marker may be titanium dioxide, chromic oxide or acid insoluble ash. Digestibility may be expressed as a percentage of the nutrient in the feed, or as mass units of digestible nutrient per mass units of nutrient in the feed.

Nutrient digestibility as used herein encompasses starch digestibility, fat digestibility, protein digestibility, and amino acid digestibility.

Energy digestibility as used herein means the gross energy of the feed consumed minus the gross energy of the faeces or the gross energy of the feed consumed minus the gross energy of the remaining digesta on a specified segment of the gastro-intestinal tract of the animal, e.g. the ileum. Metabolizable energy as used herein refers to apparent metabolizable energy and means the gross energy of the feed consumed minus the gross energy contained in the faeces, urine, and gaseous products of digestion. Energy digestibility and metabolizable energy may be measured as the difference between the intake of gross energy and the gross energy excreted in the faeces or the digesta present in specified segment of the gastro-intestinal tract using the same methods to measure the digestibility of nutrients, with appropriate corrections for nitrogen excretion to calculate metabolizable energy of feed.

In some embodiments, the compositions described herein can improve the digestibility or utilization of dietary hemicellulose or fibre in a subject. In some embodiments, the subject is a pig.

Nitrogen retention as used herein means as subject's ability to retain nitrogen from the diet as body mass. A negative nitrogen balance occurs when the excretion of nitrogen exceeds the daily intake and is often seen when the muscle is being lost. A positive nitrogen balance is often associated with muscle growth, particularly in growing animals.

Nitrogen retention may be measured as the difference between the intake of nitrogen and the excreted nitrogen by means of the total collection of excreta and urine during a period of time. It is understood that excreted nitrogen includes undigested protein from the feed, endogenous proteinaceous secretions, microbial protein, and urinary nitrogen.

The term survival as used herein means the number of subject remaining alive. The term "improved survival" may be another way of saying "reduced mortality".

The term carcass yield as used herein means the amount of carcass as a proportion of the live body weight, after a commercial or experimental process of slaughter. The term carcass means the body of an animal that has been slaughtered for food, with the head, entrails, part of the limbs, and feathers or skin removed. The term meat yield as used herein means the amount of edible meat as a proportion of the live body weight, or the amount of a specified meat cut as a proportion of the live body weight.

An "increased weight gain" refers to an animal having increased body weight on being fed feed comprising a feed additive composition compared with an animal being fed a feed without said feed additive composition being present.

The term "animal" as used herein includes all non-ruminant and ruminant animals. In a particular embodiment, the animal is a non-ruminant animal, such as a horse and a mono-gastric animal. Examples of mono-gastric animals include, but are not limited to, pigs and swine, such as piglets, growing pigs, sows; poultry such as turkeys, ducks, chicken, broiler chicks, layers; fish such as salmon, trout, tilapia, catfish and carps; and crustaceans such as shrimps and prawns. In a further embodiment the animal is a ruminant animal including, but not limited to, cattle, young calves, goats, sheep, giraffes, bison, moose, elk, yaks, water buffalo, deer, camels, alpacas, llamas, antelope, pronghorn and nilgai.

In the present context, it is intended that the term "pet food" is understood to mean a food for a household animal such as, but not limited to, dogs, cats, gerbils, hamsters, chinchillas, fancy rats, guinea pigs; avian pets, such as canaries, parakeets, and parrots; reptile pets, such as turtles, lizards and snakes; and aquatic pets, such as tropical fish and frogs.

The terms "animal feed composition," "feed", "feedstuff" and "fodder" are used interchangeably and can comprise one or more feed materials selected from the group comprising a) cereals, such as small grains (e.g., wheat, barley, rye, oats and combinations thereof) and/or large grains such as maize or sorghum; b) by products from cereals, such as corn gluten meal, Distillers Dried Grains with Solubles (DDGS) (particularly corn based Distillers Dried Grains with Solubles (cDDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; and/or e) minerals and vitamins.

Aspartic proteases described herein or a feed additive composition may be used as, or in the preparation of, a feed. The terms "feed additive composition" and "enzyme composition" are used interchangeably herein.

The feed may be in the form of a solution or as a solid or as a semi-solid depending on the use and/or the mode of application and/or the mode of administration.

When used as, or in the preparation of, a feed, such as functional feed, the enzyme or feed additive composition of the present invention may be used in conjunction with one or more of: a nutritionally acceptable carrier, a nutritionally acceptable diluent, a nutritionally acceptable excipient, a nutritionally acceptable adjuvant, a nutritionally active ingredient. For example, there be mentioned at least one component selected from the group consisting of a protein, a peptide, sucrose, lactose, sorbitol, glycerol, propylene glycol, sodium chloride, sodium sulfate, sodium acetate, sodium citrate, sodium formate, sodium sorbate, potassium chloride, potassium sulfate, potassium acetate, potassium citrate, potassium formate, potassium acetate, potassium sorbate, magnesium chloride, magnesium sulfate, magnesium acetate, magnesium citrate, magnesium formate, magnesium sorbate, sodium metabisulfite, methyl paraben and propyl paraben.

In a preferred embodiment the enzyme or feed additive composition of the present invention is admixed with a feed component to form a feedstuff. The term "feed component" as used herein means all or part of the feedstuff. Part of the feedstuff may mean one constituent of the feedstuff or more than one constituent of the feedstuff, e.g. 2 or 3 or 4 or more. In one embodiment the term "feed component" encompasses a premix or premix constituents. Preferably, the feed may be a fodder, or a premix thereof, a compound feed, or a premix thereof. A feed additive composition according to the present invention may be admixed with a compound feed, a compound feed component or to a premix of a compound feed or to a fodder, a fodder component, or a premix of a fodder.

Any feedstuff described herein may comprise one or more feed materials selected from the group comprising a) cereals, such as small grains (e.g., wheat, barley, rye, oats, triticale and combinations thereof) and/or large grains such as maize or sorghum; b) by products from cereals, such as corn gluten meal, wet-cake (particularly corn based wet- cake), Distillers Dried Grains (DDG) (particularly corn based Distillers Dried Grains (cDDG)), Distillers Dried Grains with Solubles (DDGS) (particularly corn based Distillers Dried Grains with Solubles (cDDGS)), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

The term "fodder" as used herein means any food which is provided to an animal (rather than the animal having to forage for it themselves). Fodder encompasses plants that have been cut. Furthermore, fodder includes silage, compressed and pelleted feeds, oils and mixed rations, and also sprouted grains and legumes.

Fodder may be obtained from one or more of the plants selected from: corn (maize), alfalfa (Lucerne), barley, birdsfoot trefoil, brassicas, Chau moellier, kale, rapeseed (canola), rutabaga (swede), turnip, clover, alsike clover, red clover, subterranean clover, white clover, fescue, brome, millet, oats, sorghum, soybeans, trees (pollard tree shoots for tree-hay), wheat, and legumes.

The term "compound feed" means a commercial feed in the form of a meal, a pellet, nuts, cake or a crumble. Compound feeds may be blended from various raw materials and additives. These blends are formulated according to the specific requirements of the target animal.

Compound feeds can be complete feeds that provide all the daily required nutrients, concentrates that provide a part of the ration (protein, energy) or supplements that only provide additional micronutrients, such as minerals and vitamins.

The main ingredients used in compound feed are the feed grains, which include corn, wheat, canola meal, rapeseed meal, lupin, soybeans, sorghum, oats, and barley.

Suitably a premix as referred to herein may be a composition composed of microingredients such as vitamins, minerals, chemical preservatives, antibiotics, fermentation products, and other essential ingredients. Premixes are usually compositions suitable for blending into commercial rations.

In one embodiment the feedstuff comprises or consists of corn, DDGS (such as cDDGS), wheat, wheat bran or any combination thereof.

In one embodiment the feed component may be corn, DDGS (e.g. cDDGS), wheat, wheat bran or a combination thereof. In one embodiment the feedstuff comprises or consists of corn, DDGS (such as cDDGS) or a combination thereof.

A feedstuff described herein may contain at least 30%, at least 40%, at least 50% or at least 60% by weight corn and soybean meal or corn and full fat soy, or wheat meal or sunflower meal.

For example, a feedstuff may contain between about 5 to about 40% corn DDGS. For poultry, the feedstuff on average may contain between about 7 to 15% corn DDGS. For swine (pigs), the feedstuff may contain on average 5 to 40% corn DDGS. It may also contain corn as a single grain, in which case the feedstuff may comprise between about 35% to about 80% corn.

In feedstuffs comprising mixed grains, e.g. comprising corn and wheat for example, the feedstuff may comprise at least 10% corn.

In addition or in the alternative, a feedstuff also may comprise at least one high fibre feed material and/or at least one by-product of the at least one high fibre feed material to provide a high fibre feedstuff. Examples of high fibre feed materials include: wheat, barley, rye, oats, by products from cereals, such as corn gluten meal, corn gluten feed, wet-cake, Distillers Dried Grains (DDG), Distillers Dried Grains with Solubles (DDGS), wheat bran, wheat middlings, wheat shorts, rice bran, rice hulls, oat hulls, palm kernel, and citrus pulp. Some protein sources may also be regarded as high fibre: protein obtained from sources such as sunflower, lupin, fava beans and cotton. In one aspect, the feedstuff as described herein comprises at least one high fibre material and/or at least one by-product of the at least one high fibre feed material selected from the group consisting of Distillers Dried Grains with Solubles (DDGS), particularly cDDGS, wet-cake, Distillers Dried Grains (DDG), particularly cDDG, wheat bran, and wheat for example. In one embodiment the feedstuff of the present invention comprises at least one high fibre material and/or at least one by-product of the at least one high fibre feed material selected from the group consisting of Distillers Dried Grains with Solubles (DDGS), particularly cDDGS, wheat bran, and wheat for example.

The feed may be one or more of the following: a compound feed and premix, including pellets, nuts or (cattle) cake; a crop or crop residue: corn, soybeans, sorghum, oats, barley copra, straw, chaff, sugar beet waste; fish meal; meat and bone meal; molasses; oil cake and press cake; oligosaccharides; conserved forage plants: silage; seaweed; seeds and grains, either whole or prepared by crushing, milling etc.; sprouted grains and legumes; yeast extract.

The term "feed" as used herein encompasses in some embodiments pet food. A pet food is plant or animal material intended for consumption by pets, such as dog food or cat food. Pet food, such as dog and cat food, may be either in a dry form, such as kibble for dogs, or wet canned form. Cat food may contain the amino acid taurine.

Animal feed can also include a fish food. A fish food normally contains macro nutrients, trace elements and vitamins necessary to keep captive fish in good health. Fish food may be in the form of a flake, pellet or tablet. Pelleted forms, some of which sink rapidly, are often used for larger fish or bottom feeding species. Some fish foods also contain additives, such as beta carotene or sex hormones, to artificially enhance the color of ornamental fish.

In still another aspect, animal feed encompasses bird food. Bird food includes food that is used both in birdfeeders and to feed pet birds. Typically bird food comprises of a variety of seeds, but may also encompass suet (beef or mutton fat).

As used herein the term "contacted" refers to the indirect or direct application of an aspartic protease enzyme (or composition comprising the aspartic protease) to a product (e.g. the feed). Examples of application methods which may be used, include, but are not limited to, treating the product in a material comprising the feed additive composition, direct application by mixing the feed additive composition with the product, spraying the feed additive composition onto the product surface or dipping the product into a preparation of the feed additive composition. In one embodiment the feed additive composition of the present invention is preferably admixed with the product (e.g. feedstuff). Alternatively, the feed additive composition may be included in the emulsion or raw ingredients of a feedstuff. For some applications, it is important that the composition is made available on or to the surface of a product to be affected/treated. This allows the composition to impart a performance benefit.

In some aspects, the aspartic proteases described are used for the pre-treatment of food or feed. For example, the feed having 10-300% moisture is mixed and incubated with the proteases at 5-80°C, preferably at 25-50°C, more preferably between 30-45 °C for 1 min to 72 hours under aerobic conditions or 1 day to 2 months under anaerobic conditions. The pre-treated material can be fed directly to the animals (so called liquid feeding). The pre-treated material can also be steam pelleted at elevated temperatures of 60-120°C. The proteases can be impregnated to feed or food material by a vacuum coater.

Aspartic proteases (or composition comprising the aspartic proteases) may be applied to intersperse, coat and/or impregnate a product (e.g. feedstuff or raw ingredients of a feedstuff) with a controlled amount of said enzyme.

Preferably, the aspartic proteases (or composition comprising the aspartic proteases) will be thermally stable to heat treatment up to about 70°C; up to about 85°C; or up to about 95°C. The heat treatment may be performed for up to about 1 minute; up to about 5 minutes; up to about 10 minutes; up to about 30 minutes; up to about 60 minutes.

The term thermally stable means that at least about 50%, 55%, 60%, 65%, 70%, 75%, 80%, 85%, 90%, 95%, 96%, 97% or 98% of the enzyme that was present/active in the additive before heating to the specified temperature is still present/active after it cools to room temperature. Preferably, at least about 80% of the enzyme that is present and active in the additive before heating to the specified temperature is still present and active after it cools to room temperature.

It is also possible that aspartic proteases (or composition comprising the aspartic proteases) described herein can be homogenized to produce a powder.

In an alternative preferred embodiment, the aspartic proteases (or composition comprising the aspartic proteases) can be formulated to granules as described in WO2007/044968 (referred to as TPT granules) or WO1997/016076 or WO1992/012645. "TPT" means Thermo Protection Technology.

In another aspect, when the feed additive composition is formulated into granules the granules comprise a hydrated barrier salt coated over the protein core. The advantage of such salt coating is improved thermo-tolerance, improved storage stability and protection against other feed additives otherwise having adverse effect on the enzyme. Preferably, the salt used for the salt coating has a water activity greater than 0.25 or constant humidity greater than 60 % at 20°C. In some embodiments, the salt coating comprises Na2S04.

A method of preparing aspartic proteases (or composition comprising the aspartic proteases) may also comprise the further step of pelleting the powder. The powder may be mixed with other components known in the art. The powder, or mixture comprising the powder, may be forced through a die and the resulting strands are cut into suitable pellets of variable length.

Optionally, the pelleting step may include a steam treatment, or conditioning stage, prior to formation of the pellets. The mixture comprising the powder may be placed in a conditioner, e.g. a mixer with steam injection. The mixture is heated in the conditioner up to a specified temperature, such as from 60-100°C, typical temperatures would be 70°C, 80°C, 85°C, 90°C or 95°C. The residence time can be variable from seconds to minutes and even hours. Such as 5 seconds, 10 seconds, 15 seconds, 30 seconds, 1 minutes 2 minutes., 5 minutes, 10 minutes, 15 minutes, 30 minutes and 1 hour. It will be understood that the aspartic proteases (or composition comprising the aspartic proteases) described herein are suitable for addition to any appropriate feed material.

It will be understood by the skilled person that different animals require different feedstuffs, and even the same animal may require different feedstuffs, depending upon the purpose for which the animal is reared.

Optionally, the feedstuff may also contain additional minerals such as, for example, calcium and/or additional vitamins. In some embodiments, the feedstuff is a corn soybean meal mix.

Feedstuff is typically produced in feed mills in which raw materials are first ground to a suitable particle size and then mixed with appropriate additives. The feedstuff may then be produced as a mash or pellets; the later typically involves a method by which the temperature is raised to a target level and then the feed is passed through a die to produce pellets of a particular size. The pellets are allowed to cool. Subsequently liquid additives such as fat and enzyme may be added. Production of feedstuff may also involve an additional step that includes extrusion or expansion prior to pelleting, in particular by suitable techniques that may include at least the use of steam.

The feedstuff may be a feedstuff for a monogastric animal, such as poultry (for example, broiler, layer, broiler breeders, turkey, duck, geese, water fowl), and swine (all age categories), a ruminant such as cattle (e.g. cows or bulls (including calves)), horses, sheep, a pet (for example dogs, cats) or fish (for example agastric fish, gastric fish, freshwater fish such as salmon, cod, trout and carp, e.g. koi carp, marine fish such as sea bass, and crustaceans such as shrimps, mussels and scallops). Preferably the feedstuff is for poultry.

The feed additive composition and/or the feedstuff comprising same may be used in any suitable form. The feed additive composition may be used in the form of solid or liquid preparations or alternatives thereof. Examples of solid preparations include powders, pastes, boluses, capsules, pellets, tablets, dusts, and granules which may be wettable, spray-dried or freeze-dried. Examples of liquid preparations include, but are not limited to, aqueous, organic or aqueous-organic solutions, suspensions and emulsions.

In some applications, the feed additive compositions may be mixed with feed or administered in the drinking water.

A feed additive composition, comprising admixing a protease as taught herein with a feed acceptable carrier, diluent or excipient, and (optionally) packaging.

The feedstuff and/or feed additive composition may be combined with at least one mineral and/or at least one vitamin. The compositions thus derived may be referred to herein as a premix.

In some embodiments, aspartic protease can be present in the feedstuff in the range of 1 ppb (parts per billion) to 10 % (w/w) based on pure enzyme protein. In some embodiments, the protease is present in the feedstuff is in the range of 1-100 ppm (parts per million). A preferred dose can be 1-20 g of aspartic protease per ton of feed product or feed composition or a final dose of 1 - 20 ppm aspartic protease in final product.

In some instances, it will be understood that one protease unit (PU) is the amount of enzyme that liberates from the substrate (0.6% casein solution) one microgram of phenolic compound (expressed as tyrosine equivalents) in one minute at pH 7.5 (40mM Na₂PO₄ / lactic acid buffer) and 40°C. This may be referred to as the assay for determining 1PU.

In one embodiment, suitably the enzyme is classified using the E.C. classification above, and the E.C. classification designates an enzyme having that activity when tested in the assay taught herein for determining 1 PU.

Preferably, the aspartic protease is present in the feedstuff should be at least about 200PU/kg or at least about 300 PU/kg feed or at least about 400 PU/kg feed or at least about 500 PU/kg feed or at least about 600 PU/kg feed, at least about 700 PU/kg feed, at least about 800 PU/kg feed, at least about 900 PU/kg feed or aat least about 1000 PU/ kg feed, or at least about 1500PU/kg feed, or at least about 2000PU/kg feed or at least about 2500 PU/kg feed, or at least about 3000 PU/kg feed, or at least about 3500 PU/kg feed, or at least about 4000 PU/kg feed, or at least about 4500 PU/kg feed, or at least about 5000 PU/kg feed.

In another aspect, aspartic protease can be present in the feedstuff at less than about 60,000PU/kg feed, or at less than about 70,000PU/kg feed, or at less than about 80,000PU/kg feed, or at less than about 90,000PU/kg feed, or at less than about 100,000PU/kg feed, or at less than about 200,000PU/kg feed, or at less than about 60000PU/kg feed, or at less than about 70000 PU/kg feed.

Ranges can include, but are not limited to, any combination of the lower and upper ranges discussed above.

Formulations comprising any of the aspartic proteases and compositions described herein may be made in any suitable way to ensure that the formulation comprises active enzymes. Such formulations may be as a liquid, a dry powder or a granule. Preferably, the feed additive composition is in a liquid form suitable for spray-drying on a feed pellet.

Dry powder or granules may be prepared by means known to those skilled in the art, such as, high shear granulation, drum granulation, extrusion, spheronization, fluidized bed agglomeration, fluidized bed spray

Aspartic proteases and compositions described herein may be coated, for example encapsulated. In one embodiment, the coating protects the enzymes from heat and may be considered a thermoresistant.

Feed additive composition described herein can be formulated to a dry powder or granules as described in WO20071044968 (referred to as TPT granules) or WO1997/016076 or WO1992/012645.

In one embodiment the feed additive composition may be formulated to a granule for feed compositions comprising: a core; an active agent; and at least one coating, the active agent of the granule retaining at least 50% activity, at least 60% activity, at least 70% activity, at least 80% activity after conditions selected from one or more of a) a feed pelleting process, b) a steam-heated feed pretreatment process, c) storage, d) storage as an ingredient in an unpelleted mixture, and e) storage as an ingredient in a feed base mix or a feed premix comprising at least one compound selected from trace minerals, organic acids, reducing sugars, vitamins, choline chloride, and compounds which result in an acidic or a basic feed base mix or feed premix.

With regard to the granule at least one coating may comprise a moisture hydrating material that constitutes at least 55% w/w of the granule; and/or at least one coating may comprise two coatings. The two coatings may be a moisture hydrating coating and a moisture barrier coating. In some embodiments, the moisture hydrating coating may be between 25% and 60% w/w of the granule and the moisture barrier coating may be between 2% and 15% w/w of the granule. The moisture hydrating coating may be selected from inorganic salts, sucrose, starch, and maltodextrin and the moisture barrier coating may be selected from polymers, gums, whey and starch.

The granule may be produced using a feed pelleting process and the feed pretreatment process may be conducted between 70°C and 95°C for up to several minutes, such as between 85°C and 95°C.

The feed additive composition may be formulated to a granule for animal feed comprising: a core; an active agent, the active agent of the granule retaining at least 80% activity after storage and after a steam-heated pelleting process where the granule is an ingredient; a moisture barrier coating; and a moisture hydrating coating that is at least 25% w/w of the granule, the granule having a water activity of less than 0.5 prior to the steam-heated pelleting process.

The granule may have a moisture barrier coating selected from polymers and gums and the moisture hydrating material may be an inorganic salt. The moisture hydrating coating may be between 25% and 45% w/w of the granule and the moisture barrier coating may be between 2% and 10% w/w of the granule.

The granule may be produced using a steam-heated pelleting process which may be conducted between 85°C and 95°C for up to several minutes.

Alternatively, the composition is in a liquid formulation suitable for consumption preferably such liquid consumption contains one or more of the following: a buffer, salt, sorbitol and/or glycerol.

Also, the feed additive composition may be formulated by applying, e.g. spraying, the enzyme(s) onto a carrier substrate, such as ground wheat for example.

In one embodiment the feed additive composition may be formulated as a premix. By way of example only the premix may comprise one or more feed components, such as one or more minerals and/or one or more vitamins.

In one embodiment, a direct fed microbial ("DFM") and/or an aspartic protease are formulated with at least one physiologically acceptable carrier selected from at least one of maltodextrin, limestone (calcium carbonate), cyclodextrin, wheat or a wheat component, sucrose, starch, Na₂SO₄, Talc, PVA, sorbitol, benzoate, sorbate, glycerol, sucrose, propylene glycol, 1,3-propane diol, glucose, parabens, sodium chloride, citrate, acetate, phosphate, calcium, metabisulfite, formate and mixtures thereof.

### EXAMPLES

Unless defined otherwise herein, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 2D ED., John Wiley and Sons, New York (1994), and Hale & Marham, THE HARPER COLLINS DICTIONARY OF BIOLOGY, Harper Perennial, N.Y. (1991) provide one of skill with a general dictionary of many of the terms used with this disclosure.

The disclosure is further defined in the following Examples. It should be understood that the Examples, while indicating certain embodiments, is given by way of illustration only. From the above discussion and the Examples, one skilled in the art can ascertain essential characteristics of this disclosure, and without departing from the spirit and scope thereof, can make various changes and modifications to adapt to various uses and conditions.

The meaning of abbreviations is as follows: "sec" or "s" means second(s), "ms" mean milliseconds, "min" means minute(s), "h" or "hr" means hour(s), "µL" means microliter(s), "mL" means milliliter(s), "L" means liter(s); "mL/min" is milliliters per minute; "µg/mL" is microgram(s) per milliliter(s); "LB" is Luria broth; "µm" is micrometers, "nm" is nanometers; "OD" is optical density; "IPTG" is isopropyl-β-D-thio-galactoside; "*g*" is gravitational force; "mM" is millimolar; "SDS-PAGE" is sodium dodecyl sulfate polyacrylamide; "mg/mL" is milligrams per milliliters; "mT" is metric ton, "N" is normal; "w/v" is weight for volume; "DTT" is dithiothreitol; "BCA" is bicinchoninic acid; "DMAc" is N, N'- dimethyl acetamide; "LiCI" is Lithium chloride' "NMR" is nuclear magnetic resonance; "DMSO" is dimethylsulfoxide; "SEC" is size exclusion chromatography; "GI" or "gi" means Genlnfo Identifier, a system used by GENBANK® and other sequence databases to uniquely identify polynucleotide and/or polypeptide sequences within the respective databases; "DPx" means glucan degree of polymerization having "x" units in length; "ATCC" means American Type Culture Collection (Manassas, VA), "DSMZ" and "DSM" refer to Leibniz Institute DSMZ-German Collection of Microorganisms and Cell Cultures, (Braunschweig, Germany); "EELA" is the Finish Food Safety Authority (Helsinki, Finland;) "CCUG" refer to the Culture Collection, University of Goteborg, Sweden; "Suc." means sucrose; "Gluc." means glucose; "Fruc." means fructose; "Leuc." means leucrose; and "Rxn" means reaction.

### General Methods

Standard recombinant DNA and molecular cloning techniques used herein are well known in the art and are described by Sambrook, J. and Russell, D., Molecular Cloning: A Laboratory Manual, Third Edition, Cold Spring Harbor Laboratory Press, Cold Spring Harbor, NY (2001); and by Silhavy, T. J., Bennan, M. L. and Enquist, L. W., Experiments with Gene Fusions, Cold Spring Harbor Laboratory Cold Press Spring Harbor, NY (1984); and by Ausubel, F. M. et. al., Short Protocols in Molecular Biology, 5th Ed. Current Protocols and John Wiley and Sons, Inc., N.Y., 2002.

Materials and methods suitable for the maintenance and growth of bacterial cultures are also well known in the art. Techniques suitable for use in the following Examples may be found in Manual of Methods for General Bacteriology, Phillipp Gerhardt, R. G. E. Murray, Ralph N. Costilow, Eugene W. Nester, Willis A. Wood, Noel R. Krieg and G. Briggs Phillips, eds., (American Society for Microbiology Press, Washington, DC (1994)), Biotechnology: A Textbook of Industrial Microbiology by Wulf Crueger and Anneliese Crueger (authors), Second Edition, (Sinauer Associates, Inc., Sunderland, MA (1990)), and Manual of Industrial Microbiology and Biotechnology, Third Edition, Richard H. Baltz, Arnold L. Demain, and Julian E. Davis (Editors), (American Society of Microbiology Press, Washington, DC (2010).
All reagents, restriction enzymes and materials used for the growth and maintenance of bacterial cells were obtained from BD Diagnostic Systems (Sparks, MD), Invitrogen/Life Technologies Corp. (Carlsbad, CA), Life Technologies (Rockville, MD), QIAGEN (Valencia, CA), Sigma-Aldrich Chemical Company (St. Louis, MO) or Pierce Chemical Co. (A division of Thermo Fisher Scientific Inc., Rockford, IL) unless otherwise specified. IPTG, (cat#I6758) and triphenyltetrazolium chloride were obtained from the Sigma Co., (St. Louis, MO). Bellco spin flask was from the Bellco Co., (Vineland, NJ). LB medium was from Becton, Dickinson and Company (Franklin Lakes, New Jersey). BCA protein assay was from Sigma-Aldrich (St Louis, MO).

### EXAMPLE 1

### Cloning of fungal aspartic proteases

Three fungal strains *(Rasamsonia cylindrospora* CBS432.62, *Rasamsonia composticola* CBS549.92 and *Geosmithia cylindrospora* NRRL2673) were selected as potential sources of enzymes which may be useful in various industrial applications. *Rasamsonia cylindrospora* CBS432.62 and *Rasamsonia composticola* CBS549.92 were purchased from CBS-KNAW Fungal Biodiversity Centre (Uppsalalaan 8, 3584 CT Utrecht, The Netherlands). *Geosmithia cylindrospora* NRRL2673 were purchased from ARS (NRRL) Culture Collection, National Center for Agricultural Utilization Research (1815 N. University Street Peoria, IL 61604). Chromosomal DNA was isolated from the three strains and sequenced using Illumina's next generation sequencing technology. Genes encoding fungal aspartic proteases were identified after annotation in the three aforementioned fungal species; and their nucleotide or amino acid sequence identifiers (IDs) are listed in Table 1 above. The genes for all 3 proteins have an N-terminal signal peptide (Table 1) as predicted by SignalP software version 4.0 (Nordahl Petersen et al. (2011) Nature Methods, 8:785-786), suggesting that they are all secreted enzymes.

### EXAMPLE 2

### Expression of identified fungal aspartic proteases

The DNA sequences (SEQ ID NOs:1, 3 and 5) encoding each full length fungal aspartic protease (SEQ ID NOs: 2, 4 and 6) were chemically synthesized and inserted into a *Trichoderma reesei* expression vector pGXT (the same as the pTTTpyr2 vector described in published PCT Application WO2015/017256). In the pTTTpyr2 vector, the *A. nidulans pyrG* gene is replaced with the *H. jecorina pyr2* gene. The pTTT-pyr2 expression vector contained the *T. reesei* cbhl-derived promoter (*cbhl*) and *cbhl* terminator regions allowing for a strong inducible expression of the gene of interest. The *A. nidulans amdS* and *pyr2* selective markers confer growth of transformants on acetamide as a sole nitrogen source, and the *T. reesei* telomere regions allow for non-chromosomal plasmid maintenance in a fungal cell.

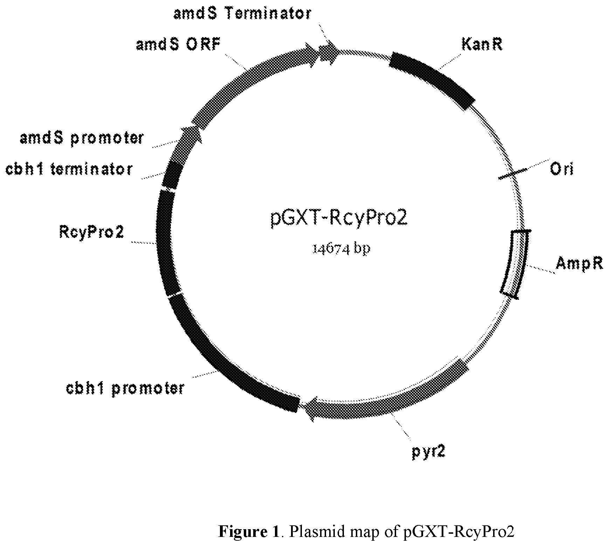

The resulting plasmids were labeled pGXT-RcyPro2, pGXT-RcoPro2 and pGXT-GcyPro1. The plasmid map of pGXT-RcyPro2 is provided in Figure 1 and the other two plasmids have similar composition with the exception of the inserted gene encoding each aspartic protease.

Each individual expression vector was then transformed into a suitable *Trichoderma reesei* strain (method described in published PCT application WO 05/001036) using protoplast transformation (Te'o et al. (2002) J. Microbiol. Methods 51:393-99). Transformants were selected on a medium containing acetamide as a sole source of nitrogen. After 5 days of growth on acetamide plates, transformants were collected and subjected to fermentation in 250 mL shake flasks in defined media containing a mixture of glucose and sophorose.

To purify the recombinant fungal aspartic proteases, each clarified culture supernatant was concentrated and ammonium sulfate was added to a final concentration of 1 M. The resulting solution was loaded onto a HiPrep™ Phenyl FF 16/10 column preequilibrated with 20 mM sodium acetate pH 5.0, and a gradient up to 1 M ammonium sulfate was run for protein separation. The resulting active protein fractions were then pooled and concentrated. Purified samples were adjusted to 40% glycerol and stored at -20°C until used.

### EXAMPLE 3

### Proteolytic activity of purified fungal aspartic proteases

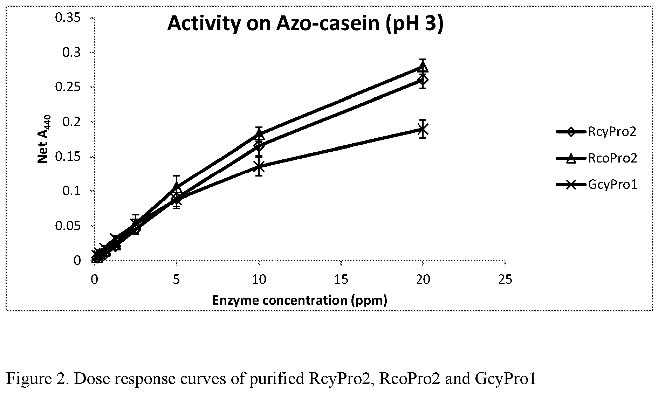

The proteolytic activity of purified aspartic proteases (RcyPro2 (SEQ ID NO: 7), RcoPro2 (SEQ ID NO: 8) and GcyPro1 (SEQ ID NO: 9)) was measured in 50 mM sodium acetate buffer (at pH 3), using azo-casein as substrate. Prior to the reaction, the enzyme samples were diluted with 20 mM sodium acetate buffer (at pH 5) to specified concentrations. The azo-casein was dissolved in 50 mM sodium acetate buffer (at pH 3) to a final concentration of 0.75% (w/v). To initiate the reaction, 90 µL of 0.75% azo-casein was added to the non-binding 96-MTP (Cat#3641, Corning Life Sciences) and incubated at 40°C for 5 min at 600 rpm in a thermomixer, followed by the addition of 10 µL of the diluted enzyme sample (or the dilution buffer alone as the blank control). After 10 min incubation in a thermomixer at 40°C and 600 rpm, the reaction was terminated by adding 100 µL of 10% (w/v) trichloroacetic acid (TCA). Following equilibration (5 min at the room temperature) and subsequent centrifugation (2000 g for 10 min at 4°C), 100 µL supernatant was transferred to a new 96-MTP and its absorbance at 440 nm (A₄₄₀) was measured using a spectrophotometer. Net A₄₄₀ was calculated by subtracting the A₄₄₀ of the blank control from that of each enzyme test sample, and then plotted versus the protein concentrations (from 0.16 ppm to 20 ppm). Each value was the mean of triplicate assays. The proteolytic activity is shown as Net A₄₄₀. The detection of proteolytic activity using azo-casein as the substrate (shown in Figure 2) indicates that all three purified fungal aspartyl proteases are active proteases.

### EXAMPLE 4

### pH profile of purified aspartic proteases

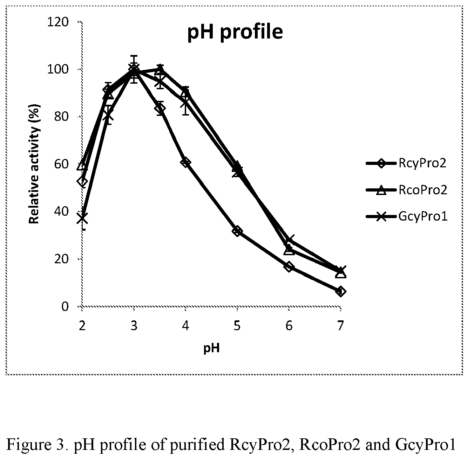

With azo-casein as the substrate, the pH profile of purified aspartic proteases (RcyPro2, RcoPro2 and GcyPro1) was studied in 25 mM glycine/sodium acetate/HEPES buffer with different pH values (ranging from pH 2 to 7). Prior to the assay, 45 µL of 50 mM glycine/sodium acetate/HEPES buffer with a specific pH value was first mixed with 45 µL of 1.5% azo-casein (dissolved in distilledH₂O) in a 96-MTP, and then 10 µL of diluted enzyme (1 mg/ml in 20 mM pH 5 sodium acetate buffer or dilution buffer alone as the blank control) was added. The reaction was performed and analyzed as described in Example 3. Enzyme activity at each pH was reported as the relative activity, where the activity at the optimal pH was set to be 100%. The pH values tested were 2, 2.5, 3, 3.5, 4, 5, 6 and 7. Each value was the mean of triplicate assays. As shown in Figure 3, the optimal pH of RcyPro2 is 3, with greater than 70% of maximal activity retained between pH 2.5 and 3.5; the optimal pH of RcoPro2 is 3.5, with greater than 70% of maximal activity retained between pH 2.5 and 4; the optimal pH of GcyPro1 is 3, with greater than 70% of maximal activity retained between pH 2.5 and 4.

### EXAMPLE 5

### Temperature profile of purified aspartic proteases

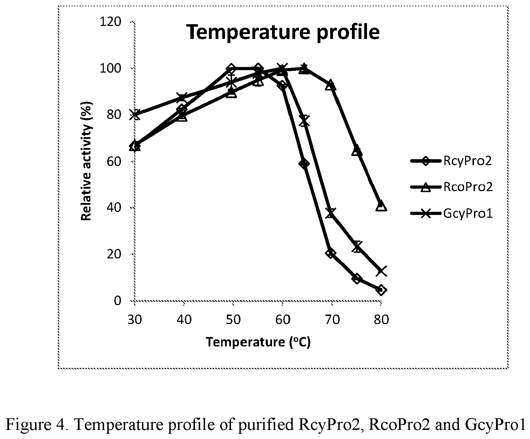

The temperature profile of purified aspartic proteases (RcyPro2, RcoPro2 and GcyPro1) was analyzed in 50 mM sodium acetate buffer pH 3 using azo-casein as substrate. Prior to the reaction, 90 µL of 0.75% azo-casein dissolved in 50 mM pH 3.0 sodium acetate buffer was added in a 200 µL PCR tube, which was subsequently incubated in a thermocycler at desired temperatures (i.e. 30 °C to 80°C) for 5 min. After the incubation, 10 µL of diluted enzyme (1 mg/ml, in 20 mM sodium acetate pH 5 buffer) or dilution buffer alone as the blank control was added to the substrate to initiate the reaction. Following 10 min incubation in a thermocycler at different temperatures, the reaction was quenched and analyzed as described in Example 3. The activity was reported as the relative activity, where the activity at the optimal temperature was set to be 100%. The tested temperatures are 30, 40, 50, 55, 60, 65, 70, 75 and 80°C. Each value was the mean of triplicate assays. As shown in Figure 4, the optimal temperature of RcyPro2 is 50°C, with greater than 70% of maximal activity retained between 40°C and 60°C; the optimal temperature of RcoPro2 is 65°C, with greater than 70% of maximal activity retained between 40°C and 70°C; the optimal temperature of GcyPro1 is 60°C, with greater than 70% of maximal activity retained between 30°C and 65°C.

### EXAMPLE 6

### Thermostability of purified aspartic proteases

The thermostability analysis of purified aspartic proteases (RcyPro2, RcoPro2 and GcyPro1) was performed using 50 mM sodium acetate pH 3.0 buffer as the incubation buffer, and azo-casein substrate for activity measurement. Samples of purified aspartic proteases were diluted in 0.2 mL incubation buffer to a final concentration of 1.0 mg/mL and subsequently incubated at 60°C for 0, 5, 10 or 30 min, respectively. At the end of each incubation period, a 50 µL aliquot of the enzyme-buffer mixture was transferred to a 96-MTP and placed on ice. The remaining activity for each sample was determined at pH 3.0. To initiate the reaction, 90 µL of 0.75% azo-casein substrate was mixed with 10 µL of each protease incubation solutions in a 96-MTP. Following a10 min incubation in a thermomixer at 40°C and 600 rpm, the reaction was quenched as described in Example 3; and enzyme activity was determined as described in Example 3. The activity was reported as the percent residual activity, where the activity at 0 min incubation time was set to be 100%.The results were summarized in Table 3.

**Table 3. Thermostability of purified aspartic proteases**

| **Enzyme Name** | **5 min** | **Residual activity (%) 10 min** | **30 min** |
|---|---|---|---|
| RcyPro2 | 80 | 71 | 45 |
| RcoPro2 | 96 | 94 | 88 |
| GcyPro1 | 93 | 86 | 73 |

### EXAMPLE 7

### Soycorn meal hydrolysis analyses of purified aspartic proteases

The extent of soycorn meal hydrolysis by the purified aspartic protease samples (RcyPro2, RcoPro2 and GcyPro1) was evaluated using the OPA (o-Phthalaldehyde) or the BCA (bicinchoninic acid) detection assays described below, to measure the amount of newly produced N-terminal amine groups or soluble peptides, respectively, released into the supernatant after the enzymatic reactions. To conduct the assays, 140 µL of the soycorn meal substrate (10% w/w in 50 mM sodium acetate pH 3 buffer) was mixed with 20 µL of a diluted enzyme sample (2 mg/mL) in a 96-MTP.After incubation for 2 hrs at 40°C in an incubator, the plates were centrifuged at 3700 rpm for 15 min at 4°C. The resulting supernatant was diluted 10 times in water to prepare for subsequent reaction product detection using the OPA and BCA assays. A sample of RONOZYME® ProAct protease was included as the commercial benchmark protease. Water was used as the (no enzyme) Blank control.

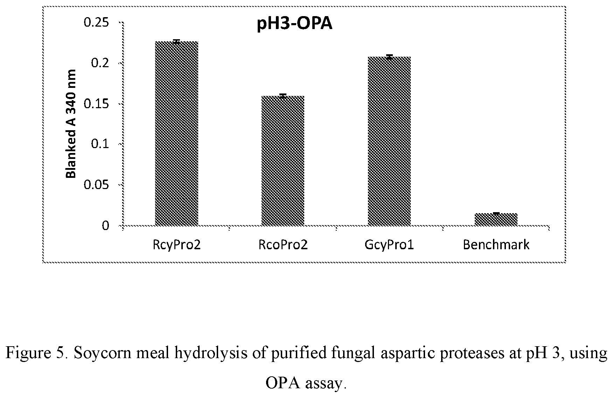

OPA detection: The OPA reagent was prepared by mixing 30 mL of 2% tri-sodium phosphate buffer (pH 11), 800 µL of 4% OPA (Cat# P1378, Sigma, dissolved in 96% ethanol), 1 mL of 3.52% Dithiothreitol (Cat# D0632, Sigma), and 8.2 mL of H₂O. The reaction was initiated by adding 10 µL of the 10X diluted protease reaction to 175 µL OPA reagent in a 96-MTP (Cat# 3635, Corning Life Sciences).After 2 min incubation, the absorbance of the resulting solution was measured at 340 nm (A₃₄₀) using a spectrophotometer. Net A₃₄₀ (Figure 5) was calculated by subtracting the A₃₄₀ of the blank (no enzyme) control from that of each protease reaction, to measure the extent of soycorn meal hydrolysis achieved by each protease sample.

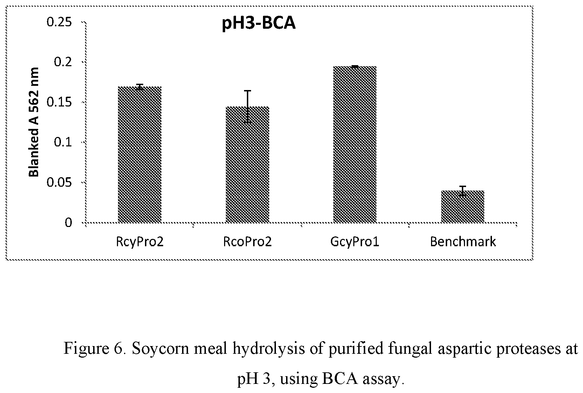

BCA detection: BCA color reaction is proportional to number of peptide bonds in polypeptides at least 3 residues long. Specifically, the BCA reaction was conducted by mixing 10 µL of the 10X diluted protease reaction with 200 µL BCA reagent. The mixture was incubated in a thermomixer at 37°C for 30 min. The absorbance was subsequently measured at 562 nm (A₅₆₂) using a spectrophotometer. Net A₅₆₂ (Figure 6) was calculated by subtracting the A₅₆₂ of the blank control (no enzyme) from that of each protease reaction, to determine the extent of soycorn meal hydrolysis observed for each protease sample.

As shown in Figure 5 and 6, purified RcyPro2, RcoPro2 and GcyPro1 proteases can effectively hydrolyze the soycorn meal substrate. Benchmark enzyme displayed very low levels of activity under these conditions.

### EXAMPLE 8

### Pepsin stability of RcyPro2 and GcyPro1

The pepsin stability of purified RcyPro2 and GcyPro1 was evaluated by measuring the residue activities of those enzymes after their incubation with high dose pepsin (Sigma, Cat. No. P7000); and Ac-Ala-Ala-Lys-NO₂-Phe-Ala-Ala-amide (AAK-NO₂-FAA, purchased from Invitrogen, Thermo) was used as the substrate. Prior to the reaction, the purified acidic protease (0.05 mg/mL, final concentration) was incubated with or without 25 mg/mL (final concentration) pepsin in 50 mM sodium acetate buffer (pH 3.0). After 30 min incubation at 37 °C, the resulting solution was diluted 10 times with water for downstream activity assay. The sodium acetate buffer in the presence or absence of 25 mg/mL pepsin was applied as the blank control. For remaining activity measurement of those acidic proteases, firstly 10 µL of 10 mM AAK-NO₂-FAA (dissolved in H₂O) was mixed with 80 µL of 50 mM sodium acetate buffer (pH 3.0) in a 96-MTP (Cat#3635, Corning Life Sciences), then 10 µL of the aforementioned diluted solution was added. The reaction was carried out at 40 °C for 30 min, and its absorbance at 296 nm (A₂₉₆) was measured using a spectrophotometer. Net A₂₉₆ was calculated by subtracting the A₂₉₆ of the enzyme from that of the corresponding blank control to represent the enzyme activity.

RONOZYME® ProAct protease was applied as the benchmark and its pepsin stability was evaluated using Suc-Ala-Ala-Pro-Phe-pNA (suc-AAPF-pNA; Cat# L-1400, BACHEM) as the substrate. All enzyme samples were diluted 100 times with water for downstream activity assays. To initiate the reaction, 10 µL of the diluted solution was mixed with 5 µL of 10 mM suc-AAPF-pNA and 85 µL of 50 mM HEPES buffer (pH 8.0) in a 96-MTP. After 10 min incubation in a thermomixer at 40 °C and 600 rpm, the absorbance of the reaction solution at 410 nm (A₄₁₀) was measured using a spectrophotometer. Net A₄₁₀ was calculated by subtracting the A₄₁₀ of the enzyme from that of the corresponding blank control to represent the benchmark activity.

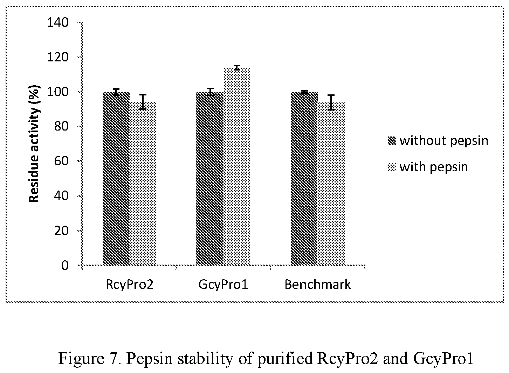

All the activities were reported as the residue activities, where the activities of the enzymes without pepsin treatment were set to be 100%. The data shown in Figure 7 indicated that both tested fungal acidic proteases (RcyPro2 and GcyPro1) are stable in the presence of exogenous pepsin.

### EXAMPLE 9

### Haze reduction performance of purified aspartic proteases

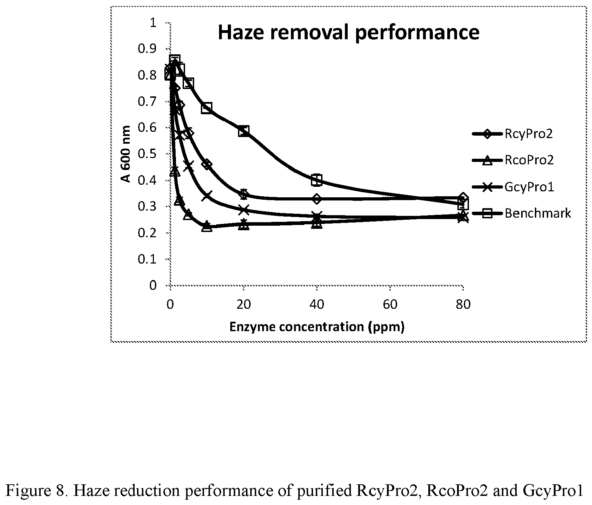

The haze reduction performance of purified fungal aspartic proteases (RcyPro2, RcoPro2 and GcyPro1) was evaluated using a gliadin-catechin assay (Michel Lopez and Luppo Edens (2005), J. Agric. Food Chem., 53, 7944-7949). Brewer Clarex® protease was applied as the benchmark. Prior to the reaction, the enzymes were diluted with 20 mM sodium acetate (pH 5) to specified concentrations. The wheat gliadin (Cat# G3375, Sigma) and the catechin (Cat# C1251, Sigma) proteins were dissolved in 20 mM acetate/phosphate buffer pH 4.5 supplemented with additional 0.2% ethanol to a final concentration of 1.6 mg/mL and 2 mg/mL, respectively. 100 µL of gliadin solution was mixed with 5 µL of diluted enzyme sample in a 96-MTP to initiate the assay; and after 90 min incubation at 45 °C in incubator, the 96-MTP was placed on ice for 5 min, followed by the addition of 100 µL catechin solution. Haze was developed at room temperature for 30 min. The absorbance of the developed haze was measured at 600 nm (A₆₀₀) on a spectrophotometer, and results were subsequently plotted versus the different enzyme concentrations (from 1.25 ppm to 80 ppm). These dose response curves for haze reduction are shown in Figure 8. These results suggest that the RcyPro2, RcoPro2 and GcyPro1 fungal aspartic proteases can effectively reduce haze.

### EXAMPLE 10

### Sequence comparison of RcyPro2, RcoPro2 and GcyPro1 to other fungal aspartic proteases

### A. Identification of Homologous Proteases

Protein homologs were identified by a BLASTP search (Altschul et al., Nucleic Acids Res, 25:3389-402, 1997) against the NCBI non-redundant protein database and the Genome Quest Patent database with search parameters set to default values. The full-length protein amino acid sequence for RcyPro2 (SEQ ID NO:2), RcoPro2 (SEQ ID NO:4) or GcyPro1 (SEQ ID NO:6), is used as the query sequence. Percent identity (PID) for both search sets is defined as the number of identical residues divided by the number of aligned residues in the pairwise alignment. Tables 4, 5 and 6 provide a list of sequences identified from NCBI non-redundant protein database, with the percent identity over 60% to RcyPro2, RcoPro2 and GcyPro1, respectively. Table 7 provides a list of sequences identified from Genome Quest Patent database, with the percent identity over 60% to RcyPro2, RcoPro2 and GcyPro1, respectively.

| **Table 4. List of RcyPro2 homologs identified from the NCBI non-redundant protein database** | | | |
|---|---|---|---|
| **Accession#** | **PID to query** | **Organism** | **AA length of subject sequence** |
| gb\|KKK27362.1\| | 71% | *Aspergillus rambellii* | 390 |
| ref\|XP_013330359.11 | 70% | *Rasamsonia emersonii* CBS 393.64 | 397 |
| ref\|XP_002567415.1\| | 70% | *Penicillium rubens* Wisconsin 54-1255 | 396 |
| emb\|CDM30996.1\| | 67% | *Penicillium roqueforti* FM164 | 396 |
| gb\|AAB35849.1\| | 68% | *Aspergillus oryzae,* M-9 | 390 |
| dbj\|GAA90749.1\| | 65% | *Aspergillus kawachii* IFO 4308 | 394 |
| dbj\|GAQ42734.1\| | 65% | *Aspergillus niger* | 394 |
| emb\|CRL26143.1\| | 67% | *Penicillium camemberti* | 396 |
| gb\|KGO67622.1\| | 67% | *Penicillium italicum* | 396 |
| gb\|EHA27889.1\| | 65% | *Aspergillus niger* ATCC 1015 | 394 |
| dbj\|BAA08123.1\| | 65% | *Aspergillus niger* | 394 |
| ref\|XP_001401093.1\| | 64% | *Aspergillus niger* CBS 513.88 | 394 |
| dbj\|BAA08640.1\| | 64% | *Aspergillus niger* | 394 |
| dbj\|BAC97797.1\| | 63% | *Monascus purpureus* | 396 |
| emb\|CEJ57934.1\| | 65% | *Penicillium brasilianum* | 391 |
| dbj\|GAO82919.1\| | 64% | *Neosartorya udagawae* | 395 |
| gb\|AAA78947.1\| | 64% | *Aspergillus awamori* | 394 |
| dbj\|GAQ09821.1\| | 65% | *Aspergillus lentulus* | 395 |
| emb\|CRG86996.1\| | 66% | *Talaromyces islandicus* | 390 |
| gb\|KGO40965.1\| | 68% | *Penicillium expansum* | 396 |
| gb\|KOS47505.1\| | 67% | *Penicillium nordicum* | 396 |
| ref\|XP_753324.1\| | 63% | *Aspergillus fumigatus* Af293 | 395 |
| ref\|XP_001259355.1\| | 63% | *Neosartorya fischeri* NRRL 181 | 395 |
| gb\|KJJ28210.1\| | 68% | *Penicillium solitum* | 396 |
| gb\|EPS32384.1\| | 64% | *Penicillium oxalicum* 114-2 | 395 |
| emb\|CEJ58766.1\| | 64% | *Penicillium brasilianum* | 394 |
| emb\|CAA59972.1\| | 64% | *Penicillium roqueforti* | 397 |
| ref\|XP_001274608.1\| | 63% | *Aspergillus clavatus* NRRL 1 | 394 |
| gb\|AAB63942.1\| | 62% | *Penicillium janthinellum* | 394 |
| dbj\|GAD93189.1\| | 64% | *Byssochlamys spectabilis* No. 5 | 398 |
| gb\|AAG34660.1\| | 62% | *Penicillium janthinellum* | 394 |
| ref\|XP_014532363.1\| | 66% | *Penicillium digitatum* Pd1 | 396 |
| gb\|KKK19892.1\| | 61% | *Aspergillus ochraceoroseus* | 396 |
| gb\|EPS32966.1\| | 63% | *Penicillium oxalicum* 114-2 | 390 |
| ref\|XP_002483414.1\| | 66% | *Talaromyces stipitatus* ATCC 10500 | 387 |
| gb\|KUL83076.1\| | 67% | *Talaromyces verruculosus* | 390 |
| emb\|CRG92082.1\| | 61% | *Talaromyces islandicus* | 393 |
| dbj\|GAM38640.1\| | 66% | *Talaromyces cellulolyticus* | 390 |
| gb\|AAB07619.1\| | 60% | *Aspergillus fumigatus* | 393 |
| emb\|CRG91238.1\| | 63% | *Talaromyces islandicus* | 681 |
| gb\|KIWO3891.1\| | 60% | *Verruconis gallopava* | 422 |
| ref\|XP_007922458.1\| | 61% | *Pseudocercospora fijiensis* | 354 |
| | | CIRAD86 | |

| **Table 5. List of RcoPro2 homologs identified from the NCBI non-redundant protein database** | | | |
|---|---|---|---|
| **Accession#** | **PID to query** | **Organism** | **AA length of subject sequence** |
| ref\|XP_013330359.1\| | 94% | *Rasamsonia emersonii* CBS 393.64 | 397 |
| gb\|KKK15635.1\| | 66% | *Aspergillus ochraceoroseus* | 390 |
| dbj\|GAO82919.1\| | 65% | *Neosartorya udagawae* | 395 |
| dbj\|GAQ09821.1\| | 65% | *Aspergillus lentulus* | 395 |
| ref\|XP_753324.1\| | 64% | *Aspergillus fumigatus* Af293 | 395 |
| ref\|XP_001259355.1\| | 64% | *Neosartorya fischeri* NRRL 181 | 395 |
| ref\|XP_002567415.1\| | 66% | *Penicillium rubens* Wisconsin 54-1255 | 396 |
| emb\|CDM30996.1\| | 64% | *Penicillium roqueforti* FM164 | 396 |
| gb\|EPS32384.1\| | 66% | *Penicillium oxalicum* 114-2 | 395 |
| emb\|CEJ57934.1\| | 64% | *Penicillium brasilianum* | 391 |
| dbj\|BAA02994.1\| | 60% | *Aspergillus oryzae* | 404 |
| gb\|KJK68602.1\| | 60% | *Aspergillus parasiticus* SU-1 | 404 |
| ref\|XP_001824175.11 | 60% | *Aspergillus oryzae* RIB40 | 404 |
| dbj\|BAC97797.1\| | 63% | *Monascus purpureus* | 396 |
| dbj\|GAA90749.1\| | 64% | *Aspergillus kawachii* IFO 4308 | 394 |
| gb\|EHA27889.1\| | 64% | *Aspergillus niger* ATCC 1015 | 394 |
| dbj\|BAA08123.1\| | 64% | *Aspergillus niger* | 394 |
| gb\|AAG34660.1\| | 61% | *Penicillium janthinellum* | 394 |
| ref\|XP_001401093.1\| | 64% | *Aspergillus niger* CBS 513.88 | 394 |
| dbj\|GAQ42734.1\| | 63% | *Aspergillus niger* | 394 |
| dbj\|GAD93189.1\| | 64% | *Byssochlamys spectabilis* No. 5 | 398 |
| emb\|CEJ58766.1\| | 63% | *Penicillium brasilianum* | 394 |
| gb\|AAB63942.1\| | 66% | *Penicillium janthinellum* | 394 |
| gb\|AAA78947.1\| | 63% | *Aspergillus awamori* | 394 |
| gb\|AAB35849.1\| | 65% | *Aspergillus oryzae,* M-9 | 390 |
| gb\|KGO67622.1\| | 64% | *Penicillium italicum* | 396 |
| dbj\|BAA08640.1\| | 63% | *Aspergillus niger* | 394 |
| gb\|EPS32966.1\| | 65% | *Penicillium oxalicum* 114-2 | 390 |
| ref\|XP_001274608.1\| | 63% | *Aspergillus clavatus* NRRL 1 | 394 |
| emb\|CRL26143.1\| | 63% | *Penicillium camemberti* | 396 |
| emb\|CRG86996.1\| | 65% | *Talaromyces islandicus* | 390 |
| gb\|KGO40965.1\| | 62% | *Penicillium expansum* | 396 |
| gb\|KOS47505.1\| | 63% | *Penicillium nordicum* | 396 |
| gb\|KJJ28210.1\| | 64% | *Penicillium solitum* | 396 |
| gb\|KNG87336.1\| | 60% | *Aspergillus nomius* NRRL 13137 | 418 |
| emb\|CRG92082.1\| | 62% | *Talaromyces islandicus* | 393 |
| emb\|CAA59972.1\| | 62% | *Penicillium roqueforti* | 397 |
| ref\|XP_014532363.1\| | 60% | *Penicillium digitatum* Pd1 | 396 |
| ref\|XP_002483414.1\| | 66% | *Talaromyces stipitatus* ATCC 10500 | 387 |
| emb\|CRG91238.1\| | 62% | *Talaromyces islandicus* | 681 |
| gb\|AAB07619.1\| | 62% | *Aspergillus fumigatus* | 393 |
| gb\|KUL83076.1\| | 65% | *Talaromyces verruculosus* | 390 |
| gb\|KIWO3891.1\| | 61% | *Verruconis gallopava* | 422 |
| dbj\|GAM38640.1\| | 64% | *Talaromyces cellulolyticus* | 390 |
| gb\|EME47325.1\| | 64% | *Dothistroma septosporum* NZE10 | 397 |
| gb\|EMF15316.1\| | 61% | *Sphaerulina musiva* SO2202 | 353 |
| gb\|EME85439.1\| | 64% | *Pseudocercospora fijiensis* CIRAD86 | 354 |
| dbj\|GAM83863.1\| | 61% | *Fungal sp.* No. 11243 | 325 |

| **Table 6. List of GcyPro1 homologs identified from the NCBI non-redundant protein database** | | | |
|---|---|---|---|
| **Accession#** | **PID to query** | **Organism** | **AA length of subject sequence** |
| gb\|KKK15635.1\| | 71% | *Aspergillus ochraceoroseus* | 390 |
| ref\|XP_013330359.1\| | 71% | *Rasamsonia emersonii* CBS 393.64 | 397 |
| emb\|CDM30996.1\| | 67% | *Penicillium roqueforti* FM164 | 396 |
| dbj\|GAO82919.1\| | 66% | *Neosartorya udagawae* | 395 |
| ref\|XP_002567415.1\| | 69% | *Penicillium rubens* Wisconsin 54-1255 | 396 |
| dbj\|GAQ09821.1\| | 65% | *Aspergillus lentulus* | 395 |
| emb\|CEJ57934.1\| | 66% | *Penicillium brasilianum* | 391 |
| gb\|KGO67622.1\| | 68% | *Penicillium italicum* | 396 |
| ref\|XP_753324.1\| | 64% | *Aspergillus fumigatus* Af293 | 395 |
| gb\|EHA27889.1\| | 64% | *Aspergillus niger* ATCC 1015 | 394 |
| dbj\|BAA08123.1\| | 64% | *Aspergillus niger* | 394 |
| dbj\|GAQ42734.1\| | 64% | *Aspergillus niger* | 394 |
| emb\|CEJ58766.1\| | 65% | *Penicillium brasilianum* | 394 |
| ref\|XP_001401093.1\| | 64% | *Aspergillus niger* CBS 513.88 | 394 |
| gb\|EPS32384.1\| | 68% | *Penicillium oxalicum* 114-2 | 395 |
| ref\|XP_001259355.1\| | 64% | *Neosartorya fischeri* NRRL 181 | 395 |
| dbj\|GAA90749.1\| | 64% | *Aspergillus kawachii* IFO 4308 | 394 |
| gb\|AAB63942.1\| | 65% | *Penicillium janthinellum* | 394 |
| gb\|AAB35849.1\| | 68% | *Aspergillus oryzae,* M-9 | 390 |
| emb\|CRL26143.1\| | 66% | *Penicillium camemberti* | 396 |
| dbj\|BAA08640.1\| | 64% | *Aspergillus niger* | 394 |
| gb\|AAA78947.1\| | 64% | *Aspergillus awamori* | 394 |
| ref\|XP_001824175.1\| | 61% | *Aspergillus oryzae* RIB40 | 404 |
| gb\|KJK68602.1\| | 61% | *Aspergillus parasiticus* SU-1 | 404 |
| gb\|KKK19892.1\| | 62% | *Aspergillus ochraceoroseus* | 396 |
| dbj\|BAA02994.1\| | 61% | *Aspergillus oryzae* | 404 |
| dbj\|BAC97797.1\| | 63% | *Monascus purpureus* | 396 |
| gb\|EPS32966.1\| | 63% | *Penicillium oxalicum* 114-2 | 390 |
| gb\|KGO40965.1\| | 68% | *Penicillium expansum* | gb\|EPS32384.1\| |
| gb\|KOS47505.1\| | 66% | *Penicillium nordicum* | 396 |
| emb\|CRG86996.1\| | 67% | *Talaromyces islandicus* | 390 |
| gb\|KJJ28210.1\| | 67% | *Penicillium solitum* | 396 |
| ref\|XP_002483414.1\| | 70% | *Talaromyces stipitatus* ATCC 10500 | 387 |
| dbj\|GAD93189.1\| | 65% | *Byssochlamys spectabilis* No. 5 | 398 |
| emb\|CAA59972.1\| | 64% | *Penicillium roqueforti* | 397 |
| gb\|AAG34660.1\| | 61% | *Penicillium janthinellum* | 394 |
| ref\|XP_001274608.1\| | 63% | *Aspergillus clavatus* NRRL 1 | 394 |
| gb\|KNG87336.1\| | 61% | *Aspergillus nomius* NRRL 13137 | 418 |
| ref\|XP_664492.1\| | 60% | *Aspergillus nidulans* FGSC A4 | 386 |
| emb\|CEL03520.1\| | 60% | *Aspergillus calidoustus* | 386 |
| ref\|XP_014532363.1\| | 66% | *Penicillium digitatum* Pd1 | 396 |
| emb\|CRG92082.1\| | 63% | *Talaromyces islandicus* | 393 |
| ref\|XP_003176622.1\| | 61% | *Microsporum gypseum* CBS 118893 | 403 |
| gb\|KUL83076.1\| | 67% | *Talaromyces verruculosus* | 390 |
| gb\|AAB07619.1\| | 61% | *Aspergillus fumigatus* | 393 |
| dbj\|GAM38640.1\| | 66% | *Talaromyces cellulolyticus* | 390 |
| emb\|CRG91238.1\| | 63% | *Talaromyces islandicus* | 681 |
| gb\|KlWO3891.1\| | 62% | *Verruconis gallopava* | 422 |
| emb\|CEJ60851.1\| | 60% | *Penicillium brasilianum* | 396 |
| ref\|XP_007922458.1\| | 64% | *Pseudocercospora fijiensis* CIRAD86 | 354 |
| ref\|XP_007744834.1\| | 61% | *Cladophialophora psammophila* CBS 110553 | 399 |
| gb\|EMF15316.1\| | 63% | *Sphaerulina musiva* SO2202 | 353 |
| gb\|KIW94714.1\| | 61% | *Cladophialophora bantiana* CBS 173.52 | 399 |
| gb\|EME47325.1\| | 64% | *Dothistroma septosporum* NZE10 | 397 |
| dbj\|GAM91036.1\| | 60% | *Fungal sp.* No.11243 | 328 |

| **Table 7. List of RcyPro2 homologs identified from Genome Quest Patent database** | | | |
|---|---|---|---|
| **Accession#** | **PID to query** | **Organism** | **AA length of subject sequence** |
| WO2014138983-1470 | 75.19% | *Paecilomyces byssochlamydoides* | 396 |
| US20150197760-0009 | 72.12% | *Rasamsonia emersonii* | 397 |
| US20140370546-0492 | 71.61% | *Penicillium chrysogenum* | 396 |
| US8815571-0007 | 69.57% | *Aspergillus oryzae* | 390 |
| WO2014202616-31569 | 68.54% | *Rasamsonia emersonii* | 377 |
| WO2015048332-25808 | 67.77% | *Talaromyces stipitatus* | 387 |
| US8815571-0001 | 66.75% | *Aspergillus saitoi* | 394 |
| WO2015004241-0493 | 66.50% | *Aspergillus niger* | 394 |
| US8815571-0003 | 66.50% | *Aspergillus niger* | 394 |
| US20150307562-0687 | 66.24% | *Aspergillus niger* | 394 |
| US20150307562-0686 | 66.24% | *Aspergillus niger* | 394 |
| US20150307562-0681 | 65.73% | *Aspergillus niger* | 394 |
| US8288517-0007 | 65.47% | Artificial Sequence | 394 |
| US20150307562-0682 | 65.47% | *Aspergillus clavatus* | 394 |
| US20150307562-0683 | 65.47% | *Neosartorya fumigata* | 395 |
| US20150307562-0691 | 65.47% | *Neosartorya fischeri* | 395 |
| US20150307562-20828 | 65.47% | *Penicillium roqueforti* | 397 |

| **Table 8. List of RcoPro2 homologs identified from Genome Quest Patent database** | | | |
|---|---|---|---|
| **Accession#** | **PID to query** | **Organism** | **AA length of subject sequence** |
| US20150197760-0009 | 94.19% | *Rasamsonia emersonii* | 397 |
| WO2014202616-31569 | 89.14% | *Rasamsonia emersonii* | 377 |
| WO2014138983-1470 | 80.3% | *Paecilomyces byssochlamydoides* | 396 |
| WO2015048332-25808 | 66.92% | *Talaromyces stipitatus* | 387 |
| US8815571-0007 | 66.67% | *Aspergillus oryzae* | 390 |
| US20140370546-0492 | 65.66% | *Penicillium chrysogenum* | 396 |

| **Table 9. List of GcyPro2 homologs identified from Genome Quest Patent database** | | | |
|---|---|---|---|
| **Accession#** | **PID to query** | **Organism** | **AA length of subject sequence** |
| WO2014138983-1470 | 77.69% | *Paecilomyces byssochlamydoides* | 396 |
| US20150197760-0009 | 74.1% | *Rasamsonia emersonii* | 397 |
| US20140370546-0492 | 69.23% | *Penicillium chrysogenum* | 396 |
| US8815571-0007 | 70.51% | *Aspergillus oryzae* | 390 |
| WO2014202616-31569 | 70.77% | *Rasamsonia emersonii* | 377 |
| WO2015048332-25808 | 71.03% | *Talaromyces stipitatus* | 387 |
| US8815571-0001 | 67.44% | *Aspergillus saitoi* | 394 |
| WO2015004241-0493 | 67.69% | *Aspergillus niger* | 394 |
| US8815571-0003 | 67.69% | *Aspergillus niger* | 394 |
| US20150307562-0687 | 66.92% | *Aspergillus niger* | 394 |
| US20150307562-0686 | 67.44% | *Aspergillus niger* | 394 |
| US20150307562-0681 | 67.18% | *Aspergillus niger* | 394 |
| US8288517-0007 | 66.92% | Artificial Sequence | 394 |
| US20150307562-0683 | 66.15% | *Neosartorya fumigata* | 395 |
| US20150307562-0691 | 66.15% | *Neosartorya fischeri* | 395 |
| US20150307562-20828 | 67.18% | *Penicillium roqueforti* | 397 |
| US8815571-0008 | 67.18% | *Penicillium janthinellum* | 394 |
| US8815571-0009 | 65.12% | *Monascus purpureus* | 395 |

### B. Multiple protein sequence alignment

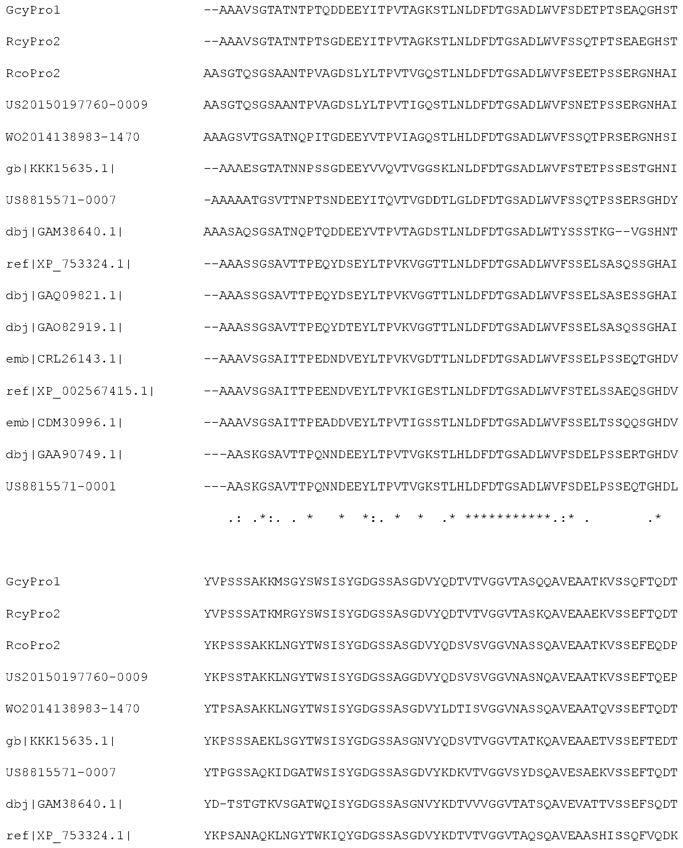

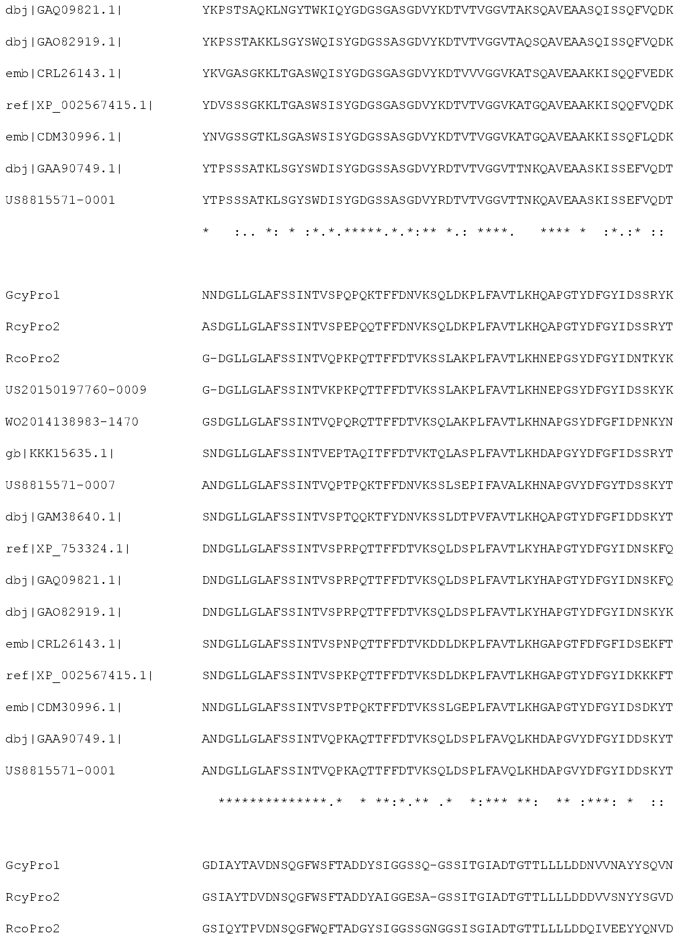

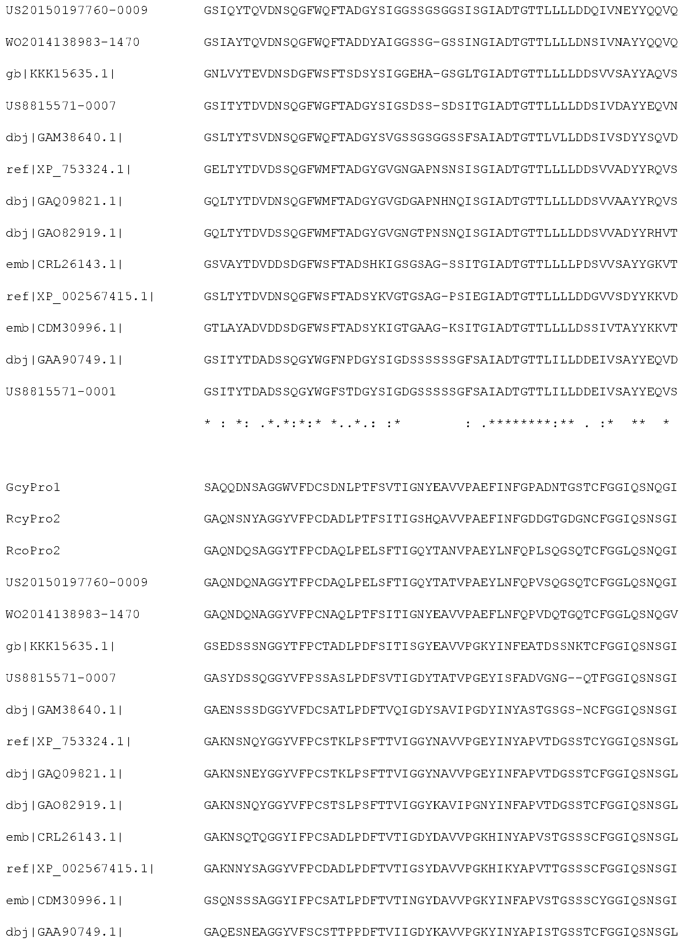

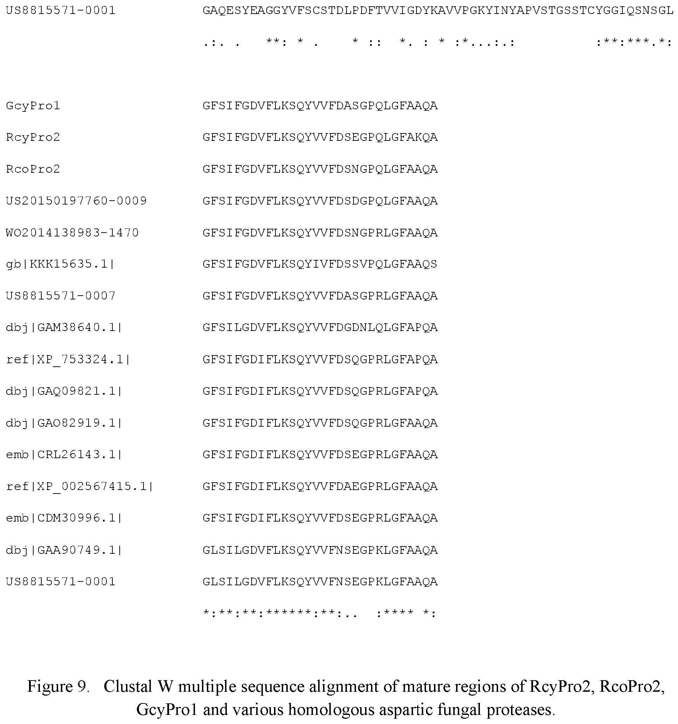

An alignment of the amino acid sequences of the predicted mature forms of RcyPro2 (SEQ ID NO:7), RcoPro2 (SEQ ID NO:8), GcyPro1 (SEQ ID NO:9), and the amino acid sequences of predicted mature form of multiple proteases: XP_753324.1 (SEQ ID NO: 10), GAA90749.1 (SEQ ID NO: 11), GAQ09821.1 (SEQ ID NO: 12), KKK15635.1 (SEQ ID NO: 13), US8815571-0007 (SEQ ID NO: 14), US8815571-0001 (SEQ ID NO: 15), GAO82919.1 (SEQ ID NO: 16), WO2014138983-1470 (SEQ ID NO: 17), CRL26143.1 (SEQ ID NO: 18), CDM30996.1 (SEQ ID NO: 19), XP_002567415.1 (SEQ ID NO: 20), US20150197760-0009 (SEQ ID NO: 21), and GAM38640.1 (SEQ ID NO: 22), listed in Tables 10 is shown in Figure 9. The sequences were aligned using CLUSTALW software (Thompson et al., Nucleic Acids Research, 22:4673-4680, 1994) with default parameters.

| **Table 10. Various fungal aspartic proteases identified by searches on NCBI and Genome Quest databases and used in sequence comparison with** RcyPro2, RcoPro2, and GcyPro1 | | |
|---|---|---|
| **accession number** | **Organism** | **SEQ ID NO** |
| ref\|XP_753324.1\| | *Aspergillus fumigatus* Af293 | 10 |
| dbj\|GAA90749.1\| | *Aspergillus kawachii* IFO 4308 | 11 |
| dbj\|GAQ09821.1\| | *Aspergillus lentulus* | 12 |
| gb\| KKK15635.1\| | *Aspergillus ochraceoroseus* | 13 |
| US8815571-0007 | *Aspergillus oryzae* | 14 |
| US8815571-0001 | *Aspergillus saitoi* | 15 |
| dbj\|GAO82919.1\| | *Neosartorya udagawae* | 16 |
| WO2014138983-1470 | *Paecilomyces byssochlamydoides* | 17 |
| emb\|CRL26143.1\| | *Penicillium camemberti* | 18 |
| emb\|CDM30996.1\| | *Penicillium roqueforti* FM164 | 19 |
| ref\|XP002567415.1\| | *Penicillium rubens* Wisconsin 54-1255 | 20 |
| US20150197760-0009 | *Rasamsonia emersonii* | 21 |
| dbj\|GAM38640.1\| | *Talaromyces cellulolyticus* | 22 |

### C. Phylogenetic Tree

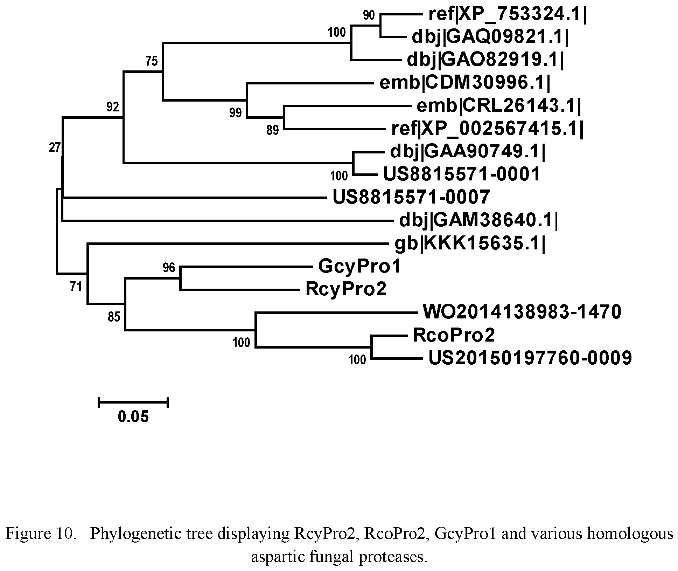

The phylogenetic tree set forth in Figure 10 was built using the amino acid sequences of the predicted mature forms of RcyPro2 (SEQ ID NO:7), RcoPro2 (SEQ ID NO:8), GcyPro1 (SEQ ID NO:9), and the predicted mature form of multiple proteases: XP_753324.1 (SEQ ID NO: 10), GAA90749.1 (SEQ ID NO: 11), GAQ09821.1 (SEQ ID NO: 12), KKK15635.1 (SEQ ID NO: 13), US8815571-0007 (SEQ ID NO: 14), US8815571-0001 (SEQ ID NO: 15), GAO82919.1 (SEQ ID NO: 16), WO2014138983-1470 (SEQ ID NO: 17), CRL26143.1 (SEQ ID NO: 18), CDM30996.1 (SEQ ID NO: 19), XP_002567415.1 (SEQ ID NO: 20), US20150197760-0009 (SEQ ID NO: 21), and GAM38640.1 (SEQ ID NO: 22), shown on Figure 9.

The phylogenetic tree was built using program MEGA 5 (Koichiro Tamura et al., (2011) Mol Biol Evol. 28(10): 2731-2739) which applies the Neighbor Joining method (NJ) (Saitou, N.; and Nei, M. (1987). The neighbor-joining (NJ) method: a new method for reconstructing Guide Trees. Mol. Biol. Evol. 4, 406-425). The NJ method works on a matrix of distances between all pairs of sequences to be analyzed. These distances are related to the degree of divergence between the sequences.

## Claims

1. A recombinant construct comprising a regulatory sequence functional in a production host operably linked to a nucleotide sequence encoding an aspartic protease selected from the group consisting of
(a) an aspartic protease having at least 90% sequence identity to SEQ ID NO:2 or SEQ ID NO:7; and
(b) an aspartic protease having at least 90% sequence identity to SEQ ID NO:6, or SEQ ID NO:9.

2. A production host; comprising the recombinant construct according to claim 1 wherein said host is selected from the group consisting of fungi, bacteria, yeast and algae, optionally
wherein the aspartic protease construct is chromosomally or extrachromosomally expressed.

3. A method for producing an aspartic protease comprising:
(a) transforming a production host with the recombinant construct of claim 1; and
(b) culturing the production host of step (a) under conditions whereby the aspartic protease is produced.

4. A method according to claim 3 wherein the aspartic protease is recovered from the production host.

5. An aspartic protease-containing culture supernatant obtained by the method of any of claims 3 or 4.

6. A recombinant microbial production host for expressing an aspartic protease, said recombinant microbial production host comprising the recombinant construct of claim 1, optionally wherein said host is selected from the group consisting of bacteria, fungi, yeast and algae.

7. A production host according to claim 6 wherein the aspartic protease construct is chromosomally or extrachromosomally expressed.

8. Use of an aspartic protease in feed, feedstuff, a feed additive composition or premix wherein the aspartic protease is selected from the group consisting of
(a) an aspartic protease having at least 90% sequence identity to SEQ ID NO:2 or SEQ ID NO:7; and
(b) an aspartic protease having at least 90% sequence identity to SEQ ID NO:6, or SEQ ID NO:9,
wherein the aspartic protease may be used (i) alone or (ii) in combination with a direct fed microbial comprising at least one bacterial strain or (iii) with at least one other enzyme or (iv) in combination with a direct fed microbial comprising at least one bacterial strain and at least one other enzyme.

9. Animal feed comprising an aspartic protease selected from the group consisting of:
(a) an aspartic protease having at least 90% sequence identity to SEQ ID NO:2 or SEQ ID NO:7; and
(b) an aspartic protease having at least 90% sequence identity to SEQ ID NO:6, or SEQ ID NO:9
wherein the aspartic protease is present in an amount from 1-20g/ton feed and further wherein the aspartic protease may be used (i) alone or (ii) in combination with a direct fed microbial comprising at least one bacterial strain or (iii) with at least one other enzyme or (iv) in combination with a direct fed microbial comprising at least one bacterial strain and at least one other enzyme.

10. An isolated polypeptide having protease activity, said polypeptide comprising an amino acid sequence of a protease selected from the group consisting of:
(a) an aspartic protease having at least 90% sequence identity to SEQ ID NO:2 or SEQ ID NO:7; and
(b) an aspartic protease having at least 90% sequence identity to SEQ ID NO:6, or SEQ ID NO:9.

11. A polynucleotide sequence encoding the polypeptide of claim 10.

12. A feed additive composition for use in animal feed comprising the polypeptide of claim 10 and at least one component selected from the group consisting of a protein, a peptide, sucrose, lactose, sorbitol, glycerol, propylene glycol, sodium chloride, sodium sulfate, sodium acetate, sodium citrate, sodium formate, sodium sorbate, potassium chloride, potassium sulfate, potassium acetate, potassium citrate, potassium formate, potassium acetate, potassium sorbate, magnesium chloride, magnesium sulfate, magnesium acetate, magnesium citrate, magnesium formate, magnesium sorbate, sodium metabisulfite, methyl paraben and propyl paraben.

13. A granulated feed additive composition for use in animal feed comprising the polypeptide of claim 10 wherein the granulated feed additive composition comprises particles produced by a process selected from the group consisting of high shear granulation, drum granulation, extrusion, spheronization, fluidized bed agglomeration, fluidized bed spray coating, spray drying, freeze drying, prilling, spray chilling, spinning disk atomization, coacervation, tableting, or any combination of the above processes, optionally wherein the mean diameter of the particles is greater than 50 microns and less than 2000 microns.

14. The feed additive composition of claim 13 wherein said composition is in a liquid form, optionally wherein said composition is in a liquid form suitable for spray-drying on a feed pellet.

## Patentansprüche

1. Rekombinantes Konstrukt umfassend eine regulatorische Sequenz, die in einem Produktionswirt funktionell ist, der funktionsfähig mit einer Nukleotidsequenz verknüpft ist, die für eine Aspartat-Protease codiert ausgewählt aus der Gruppe bestehend aus
(a) einer Aspartat-Protease, die mindestens 90 % Sequenzidentität mit SEQ ID NO: 2 oder SEQ ID NO: 7 aufweist; und
(b) einer Aspartat-Protease, die mindestens 90 % Sequenzidentität mit SEQ ID NO: 6 oder SEQ ID NO: 9 aufweist.

2. Produktionswirt umfassend das rekombinante Konstrukt nach Anspruch 1, wobei der Wirt aus der Gruppe ausgewählt wird bestehend aus Pilzen, Bakterien, Hefe und Algen, wahlweise
wobei das Konstrukt Aspartat-Protease chromosomal oder extrachromosomal ausgedrückt wird.

3. Verfahren für die Herstellung einer Aspartat-Protease, umfassend:
(a) Umwandeln eines Produktionswirts mit dem rekombinanten Konstrukt nach Anspruch 1; und
(b) Züchten des Produktionswirts aus Schritt (a) unter Bedingungen, wodurch die Aspartat-Protease hergestellt wird.

4. Verfahren nach Anspruch 3, wobei die Aspartat-Protease von dem Produktionswirt gewonnen wird.

5. Aspartat-Protease enthaltender Kulturüberstand, die durch das Verfahren nach einem der Ansprüche 3 oder 4 erhalten wird.

6. Rekombinanter mikrobieller Produktionswirt zum Exprimieren einer Aspartat-Protease, wobei der rekombinante mikrobielle Produktionswirt das rekombinante Konstrukt nach Anspruch 1 umfasst, wobei wahlweise der Wirt aus der Gruppe ausgewählt wird bestehend aus Bakterien, Pilzen, Hefe und Algen

7. Produktionswirt nach Anspruch 6, wobei das Konstrukt Aspartat-Protease chromosomal oder extrachromosomal ausgedrückt wird.

8. Verwendung einer Aspartat-Protease in Futter, Futtermittel, einer Futterzusatzzusammensetzung oder Vormischung, wobei die Aspartat-Protease aus der Gruppe ausgewählt wird bestehend aus
(a) einer Aspartat-Protease, die mindestens 90 % Sequenzidentität mit SEQ ID NO: 2 oder SEQ ID NO: 7 aufweist; und
(b) einer Aspartat-Protease, die mindestens 90 % Sequenzidentität mit SEQ ID NO: 6 oder SEQ ID NO: 9 aufweist,
wobei die Aspartat-Protease (i) allein oder (ii) in Kombination mit einer direkt zugeführten Mikrobe, die mindestens einen Bakterienstamm umfasst oder (iii) mit mindestens einem anderen Enzym oder (iv) in Kombination mit einer direkt zugeführten Mikrobe umfassend mindestens einen Bakterienstamm und mindestens ein anderes Enzym verwendet werden kann.

9. Tierfutter umfassend eine Aspartat-Protease ausgewählt aus der Gruppe bestehend aus:
(a) einer Aspartat-Protease, die mindestens 90 % Sequenzidentität mit SEQ ID NO: 2 oder SEQ ID NO: 7 aufweist; und
(b) einer Aspartat-Protease, die mindestens 90 % Sequenzidentität mit SEQ ID NO: 6 oder SEQ ID NO: 9 aufweist.
wobei die Aspartat-Protease in einer Menge von 1-20 g/Tonne Futter vorliegt und wobei ferner die Aspartat-Protease (i) allein oder (ii) in Kombination mit einer direkt zugeführten Mikrobe, die mindestens einen Bakterienstamm umfasst, oder (iii) mit mindestens einem anderen Enzym oder (iv) in Kombination mit einer direkt zugeführten Mikrobe umfassend mindestens einen Bakterienstamm und mindestens ein anderes Enzym verwendet werden kann.

10. Isoliertes Polypeptid, das Proteaseaktivität aufweist, wobei das Polypeptid eine Aminosäuresequenz einer Protease umfasst ausgewählt aus der Gruppe bestehend aus:
(a) einer Aspartat-Protease, die mindestens 90 % Sequenzidentität mit SEQ ID NO: 2 oder SEQ ID NO: 7 aufweist; und
(b) einer Aspartat-Protease, die mindestens 90 % Sequenzidentität mit SEQ ID NO: 6 oder SEQ ID NO: 9 aufweist.

11. Polynukleotidsequenz, die für das Polypeptid nach Anspruch 10 kodiert.

12. Futterzusatzzusammensetzung zur Verwendung in Tierfutter, umfassend das Polypeptid nach Anspruch 10 und mindestens eine Komponente ausgewählt aus der Gruppe bestehend aus einem Protein, einem Peptid, Sucrose, Lactose, Sorbit, Glycerin, Propylenglykol, Natriumchlorid, Natriumsulfat, Natriumacetat, Natriumcitrat, Natriumformiat, Natriumsorbat, Kaliumchlorid, Kaliumsulfat, Kaliumacetat, Kaliumcitrat, Kaliumformiat, Kaliumacetat, Kaliumsorbat, Magnesiumchlorid, Magnesiumsulfat, Magnesiumacetat, Magnesiumcitrat, Magnesiumformiat, Magnesiumsorbat, Natriummetabisulfit, Methylparaben und Propylparaben.

13. Granulierte Futterzusatzzusammensetzung zur Verwendung in Tierfutter, umfassend das Polypeptid nach Anspruch 10, wobei die granulierte Futterzusatzzusammensetzung Teilchen umfasst hergestellt durch ein Verfahren ausgewählt aus der Gruppe bestehend aus Hochschergranulierung, Trommelgranulierung, Extrusion, Sphäronisierung, Wirbelbettagglomeration, Wirbelbettspritzbeschichten, Sprühtrocknung, Gefriertrocknung, Sprühkristallisierung, Sprühkühlen, Schleuderscheibenatomisieren, Coazervieren, Tablettieren oder irgendeiner Kombination der obigen Verfahren, wobei wahlweise der mittlere Durchmesser der Teilchen größer als 50 Mikron und geringer als 2000 Mikron ist.

14. Futterzusatzzusammensetzung nach Anspruch 13, wobei die Zusammensetzung sich in einer flüssigen Form befindet, wobei wahlweise die Zusammensetzung sich in einer flüssigen Form befindet, die für Sprühtrocknen auf einem Futterpellet geeignet ist.

## Revendications

1. Produit de synthèse recombinant comprenant une séquence régulatrice fonctionnelle dans un hôte de production lié de manière opérationnelle à une séquence de nucléotides codant pour une protéase aspartique sélectionnée parmi le groupe constitué de
(a) une protéase aspartique ayant au moins 90 % d'identité de séquence avec la SEQ ID NO : 2 ou la SEQ ID NO : 7 ; et
(b) une protéase aspartique ayant au moins 90 % d'identité de séquence avec la SEQ ID NO : 6 ou la SEQ ID NO : 9.

2. Hôte de production : comprenant le produit de synthèse recombinant selon la revendication 1, dans lequel ledit hôte est sélectionné parmi le groupe constitué de champignons, bactéries, levures et algues, optionnellement
dans lequel le produit de synthèse de protéase aspartique est exprimé chromosomiquement ou extra-chromosomiquement.

3. Procédé de production d'une protéase aspartique, comprenant les étapes consistant à :
(a) transformer un hôte de production avec le produit de synthèse recombinant selon la revendication 1 ; et
(b) faire une culture de l'hôte de production de l'étape (a) dans des conditions par lesquelles la protéase aspartique est produite.

4. Procédé selon la revendication 3, dans lequel la protéase aspartique est récupérée de l'hôte de production.

5. Surnageant de culture contenant une protéase aspartique obtenu par le procédé selon l'une quelconque des revendications 3 ou 4.

6. Hôte de production microbien recombinant pour exprimer une protéase aspartique, ledit hôte de production microbien recombinant comprenant le produit de synthèse recombinant selon la revendication 1, optionnellement dans lequel ledit hôte est sélectionné parmi le groupe constitué de bactéries, champignons, levure et algues.

7. Hôte de production selon la revendication 6, dans lequel le produit de synthèse de protéase aspartique est exprimé chromosomiquement ou extra-chromosomiquement.

8. Utilisation d'une protéase aspartique dans un aliment pour animaux, fourrage, une composition additive pour alimentation animale ou un prémix, dans laquelle la protéase aspartique est sélectionnée parmi le groupe constitué de
(a) une protéase aspartique ayant au moins 90 % d'identité de séquence avec la SEQ ID NO : 2 ou la SEQ ID NO : 7 ; et
(b) une protéase aspartique ayant au moins 90 % d'identité de séquence avec la SEQ ID NO : 6 ou la SEQ ID NO : 9,
dans laquelle la protéase aspartique peut être utilisée (i) seule ou (ii) en combinaison avec un microbe administré directement comprenant au moins une souche bactérienne ou (iii) avec au moins une autre enzyme ou (iv) en combinaison avec un microbe administrés directement comprenant au moins une souche bactérienne et au moins une autre enzyme.

9. Aliment pour animaux comprenant une protéase aspartique sélectionnée parmi le groupe constitué de :
(a) une protéase aspartique ayant au moins 90 % d'identité de séquence avec la SEQ ID NO : 2 ou la SEQ ID NO : 7 ; et
(b) une protéase aspartique ayant au moins 90 % d'identité de séquence avec la SEQ ID NO : 6 ou la SEQ ID NO : 9,
dans lequel la protéase aspartique est présente en une quantité de 1-20 g/tonne d'aliment pour animaux et dans lequel en outre la protéase aspartique peut être utilisée (i) seule ou (ii) en combinaison avec un microbe administré directement comprenant au moins une souche bactérienne ou (iii) avec au moins une autre enzyme ou (iv) en combinaison avec un microbe administré directement comprenant au moins une souche bactérienne et au moins une autre enzyme.

10. Polypeptide isolé ayant une activité de protéase, ledit polypeptide comprenant une séquence d'acides aminés d'une protéase sélectionnée parmi le groupe constitué de :
(a) une protéase aspartique ayant au moins 90 % d'identité de séquence avec la SEQ ID NO : 2 ou la SEQ ID NO : 7 ; et
(b) une protéase aspartique ayant au moins 90 % d'identité de séquence avec la SEQ ID NO : 6 ou la SEQ ID NO : 9.

11. Séquence polynucléotidique codant pour le polypeptide selon la revendication 10.

12. Composition additive pour alimentation animale pour une utilisation dans un aliment pour animaux comprenant le polypeptide selon la revendication 10 et au moins un composant sélectionné parmi le groupe constitué d'une protéine, un peptide, saccharose, lactose, sorbitol, glycérol, propylène glycol, chlorure de sodium, sulfate de sodium, acétate de sodium, citrate de sodium, formiate de sodium, sorbate de sodium, chlorure de potassium, sulfate de potassium, acétate de potassium, citrate de potassium, formiate de potassium, acétate de potassium, sorbate de potassium, chlorure de magnésium, sulfate de magnésium, acétate de magnésium, citrate de magnésium, formiate de magnésium, sorbate de magnésium, métabisulfite de sodium, méthylparabène et propylparabène.

13. Composition additive granulée pour alimentation animale pour une utilisation dans un aliment pour animaux comprenant le polypeptide selon la revendication 10, dans laquelle la composition additive granulée pour alimentation animale comprend des particules produites par un procédé sélectionné parmi le groupe constitué de granulation à haut cisaillement, granulation en tambour, extrusion, sphéronisation, agglomération en lit fluidisé, enrobage par pulvérisation en lit fluidisé, séchage par atomisation, lyophilisation, grelonage, refroidissement par atomisation, atomisation sur disque tournant, coacervation, fabrication en comprimés, ou une combinaison quelconque des procédés ci-dessus, optionnellement dans laquelle le diamètre moyen des particules est supérieur à 50 microns et inférieur à 2000 microns.

14. Composition additive pour alimentation animale selon la revendication 13, dans laquelle ladite composition est sous une forme liquide, optionnellement dans laquelle ladite composition est sous une forme liquide convenant au séchage par pulvérisation sur un pellet d'aliment pour animaux.


### SEQUENCE LISTING

<110> DuPont Nutrition Biosciences
   Gu, Xiaogang
   Shipovskov, Stepan
   Yu, Shukun
   Zou, Zhengzheng
<120> ASPARTIC ACID PROTEASES
<130> NB40988PCT
<160> 22
<170> PatentIn version 3.5
<210> 1
   <211> 1349
   <212> DNA
   <213> Rasamsonia cylindrospora
<400> 1
   atgggcgtct ccaaagcggt cgtgactgct ctctctctgt gctcggcctt cgctgccgcc      60
   gcgccggctc cgccgcggac cttctcgctc caccaggtgc ccgtcccgcg gaccaagccg     120
   cgccccaatc cggcctccca gtatgcgcgc gcgctgggca agttccatgc ccccgtgccg    180
   gccaatgtcg ccgcggccgc cgtcagtggc accgcgacca acacgccgac ctccggcgac     240
   gaggagtaca tcacgccggt gacggccggc aagagcacgc tgaacctgga ctttgacacc     300
   ggatcggcgg atctgtaagt acggagacag ctggaccagg gaatgccttg ctgacaccct     360
   ccaggtgggt cttctcgtcg cagacgccca cgtcggaggc cgagggccac tccacctacg     420
   tgcccagctc gtcggccacg aagatgcgcg gctacagctg gagcatctcg tacggggatg     480
   gcagttcggc cagtggcgat gtctaccagg ataccgtcac ggtcggcggc gtgacggcct     540
   ccaagcaggc cgtggaggcc gcggagaagg tcagctccga gttcacccag gacacggcca     600
   gcgatggcct gctgggattg gcgttcagca gcatcaacac cggtgagtcg ctttcttctt     660
   ccatggcatc ccgggaacgg actccgtaac ggacttcgtg ctaacgggtg ggaatagtct     720
   cgccggaacc gcagcagacc ttcttcgaca acgtcaagtc gcagctggac aagcccctgt     780
   ttgcggtgac gctgaagcac caggcgcccg gcacctacga cttcgggtac atcgactcca     840
   gccgctacac gggcagcatc gcgtacaccg acgtcgacaa ctcgcaggga ttctggagct     900
   tcacggcgga tgactacgcg atcggcggcg agagcgcggg ttcctcgatc acggggattg     960
   ctggtacgtc cgcagaaaag gagaatatcc gtccgctcgc taaccagaca gacaccggca    1020
   ccacgctcct cctcctcgac gacgacgtcg tctccaacta ctacagcggc gtggacggcg    1080
   cgcagaactc caactacgcc ggcggctacg tcttcccctg tgacgccgac ctgccgacgt   1140
   tctcgatcac catcggcagc caccaggccg tcgtgccggc cgagttcatc aatttcggcg    1200
   acgacggcac cggcgacggc aactgcttcg gcggcatcca gtcgaactcg ggcatcggct    1260
   tttcgatctt cggcgacgtc ttcctcaaga gccagtacgt ggtgtttgac tcggagggac    1320
   ctcagctggg ctttgccaag caggcgtaa                                      1349
<210> 2
   <211> 391
   <212> PRT
   <213> Rasamsonia cylindrospora
<400> 2
   Met Gly Val Ser Lys Ala Val Val Thr Ala Leu Ser Leu Cys Ser Ala
   1               5                   10                  15
   Phe Ala Ala Ala Ala Pro Ala Pro Pro Arg Thr Phe Ser Leu His Gln
               20                  25                  30
   Val Pro Val Pro Arg Thr Lys Pro Arg Pro Asn Pro Ala Ser Gln Tyr
           35                  40                  45
   Ala Arg Ala Leu Gly Lys Phe His Ala Pro Val Pro Ala Asn Val Ala
       50                  55                  60
   Ala Ala Ala Val Ser Gly Thr Ala Thr Asn Thr Pro Thr Ser Gly Asp
   65                  70                  75                  80
   Glu Glu Tyr Ile Thr Pro Val Thr Ala Gly Lys Ser Thr Leu Asn Leu
                       85                  90                  95
   Asp Phe Asp Thr Gly Ser Ala Asp Leu Trp Val Phe Ser Ser Gln Thr
                   100                 105                 110
   Pro Thr Ser Glu Ala Glu Gly His Ser Thr Tyr Val Pro Ser Ser Ser
               115                 120                 125
   Ala Thr Lys Met Arg Gly Tyr Ser Trp Ser Ile Ser Tyr Gly Asp Gly
       130                 135                 140
   Ser Ser Ala Ser Gly Asp Val Tyr Gln Asp Thr Val Thr Val Gly Gly
   145                 150                 155                 160
   Val Thr Ala Ser Lys Gln Ala Val Glu Ala Ala Glu Lys Val Ser Ser
                   165                 170                 175
   Glu Phe Thr Gln Asp Thr Ala Ser Asp Gly Leu Leu Gly Leu Ala Phe
               180                 185                 190
   Ser Ser Ile Asn Thr Val Ser Pro Glu Pro Gln Gln Thr Phe Phe Asp
           195                 200                 205
   Asn Val Lys Ser Gln Leu Asp Lys Pro Leu Phe Ala Val Thr Leu Lys
       210                 215                 220
   His Gln Ala Pro Gly Thr Tyr Asp Phe Gly Tyr Ile Asp Ser Ser Arg
   225                 230                 235                 240
   Tyr Thr Gly Ser Ile Ala Tyr Thr Asp Val Asp Asn Ser Gln Gly Phe
                   245                 250                 255
   Trp Ser Phe Thr Ala Asp Asp Tyr Ala Ile Gly Gly Glu Ser Ala Gly
               260                 265                 270
   Ser Ser Ile Thr Gly Ile Ala Asp Thr Gly Thr Thr Leu Leu Leu Leu
           275                 280                 285
   Asp Asp Asp Val Val Ser Asn Tyr Tyr Ser Gly Val Asp Gly Ala Gln
       290                 295                 300
   Asn Ser Asn Tyr Ala Gly Gly Tyr Val Phe Pro Cys Asp Ala Asp Leu
   305                 310                 315                 320
   Pro Thr Phe Ser Ile Thr Ile Gly Ser His Gln Ala Val Val Pro Ala
                   325                 330                 335
   Glu Phe Ile Asn Phe Gly Asp Asp Gly Thr Gly Asp Gly Asn Cys Phe
               340                 345                 350
   Gly Gly Ile Gln Ser Asn Ser Gly Ile Gly Phe Ser Ile Phe Gly Asp
           355                 360                 365
   Val Phe Leu Lys Ser Gln Tyr Val Val Phe Asp Ser Glu Gly Pro Gln
       370                 375                 380
   Leu Gly Phe Ala Lys Gln Ala
   385                 390
<210> 3
   <211> 1294
   <212> DNA
   <213> Rasamsonia composticola
<400> 3
   atgggtctgt ccaaagcctt cgtgtctgca ctctcgctgt gctccgccgt cgccgtggcc      60
   gccccggcgc ctgctcccaa cgtgcagttc tctctgaagc aggtcgctgt gccccggagc     120
   aagcctcgtg ctcctccagc tgccgagtac gcgcgcgctc tagccaagta tggcgccccc     180
   gtgccgtcgt ccgtgcggac cgccgcgtcc ggcacgcaga gcggctctgc ggccaacacg     240
   cccgtcgccg gtgacagcct gtatctgacc cccgtcaccg tcggccagag cacgctcaac     300
   ctcgactttg acacgggttc tgcggatctg taagtattgt actagatcac ctgaagacga     360
   acgagaactg acccgtccag ctgggtcttc tccgaggaga cgccctccag cgagcgcggc     420
   aaccacgcca tctacaagcc cagctcctcg gccaagaagc tgaacggcta cacctggagc     480
   atctcgtacg gcgacggcag ctccgccagc ggtgatgtct accaggactc cgtttccgtc     540
   ggcggcgtca acgcgtccag ccaggcggtc gaggcggcca ccaaggtcag ctctgagttc     600
   gagcaggacc ccggcgacgg cctgctgggt ctggccttca gcagcatcaa caccgtccag     660
   cccaagccgc agaccacctt cttcgacacg gtcaagagct ccctcgccaa gccgctgttc     720
   gccgtcaccc taaagcacaa cgagcccggc agctacgact ttggctacat cgacaacacc     780
   aagtacaagg gcagcatcca gtacaccccg gtcgacaact cgcagggctt ctggcagttc     840
   actgcggacg gctactcgat tggcggctcc agcggcaacg gcggctccat ttctggcatt     900
   gctggtaagg acccctcaat ccaatagata aaagccccat atgctgatca ttccagacac     960
   cggcaccacc ctcctcctgc tcgacgacca gatcgtcgag gagtactacc agaacgtcga    1020
   cggcgcccag aacgaccaga gcgccggtgg ctacaccttc ccgtgcgacg cgcagctgcc    1080
   cgagctgagc ttcaccatcg gccagtacac cgccaacgtc cccgccgagt acctcaactt    1140
   ccagccgctg tcccagggca gccagacctg cttcggcggc ctgcagtcca accagggtat    1200
   tggcttctcc atcttcggcg acgtcttcct caagagccag tatgtcgtct ttgactcgaa    1260
   tggtcctcag ctgggctttg ctgctcaggc ttag                                1294
<210> 4
   <211> 396
   <212> PRT
   <213> Rasamsonia composticola
<400> 4
   Met Gly Leu Ser Lys Ala Phe Val Ser Ala Leu Ser Leu Cys Ser Ala
   1               5                   10                  15
   Val Ala Val Ala Ala Pro Ala Pro Ala Pro Asn Val Gln Phe Ser Leu
               20                  25                  30
   Lys Gln Val Ala Val Pro Arg Ser Lys Pro Arg Ala Pro Pro Ala Ala
           35                  40                  45
   Glu Tyr Ala Arg Ala Leu Ala Lys Tyr Gly Ala Pro Val Pro Ser Ser
       50                  55                  60
   Val Arg Thr Ala Ala Ser Gly Thr Gln Ser Gly Ser Ala Ala Asn Thr
   65                  70                  75                  80
   Pro Val Ala Gly Asp Ser Leu Tyr Leu Thr Pro Val Thr Val Gly Gln
                   85                  90                  95
   Ser Thr Leu Asn Leu Asp Phe Asp Thr Gly Ser Ala Asp Leu Trp Val
               100                 105                 110
   Phe Ser Glu Glu Thr Pro Ser Ser Glu Arg Gly Asn His Ala Ile Tyr
           115                 120                 125
   Lys Pro Ser Ser Ser Ala Lys Lys Leu Asn Gly Tyr Thr Trp Ser Ile
       130                 135                 140
   Ser Tyr Gly Asp Gly Ser Ser Ala Ser Gly Asp Val Tyr Gln Asp Ser
   145                 150                 155                 160
   Val Ser Val Gly Gly Val Asn Ala Ser Ser Gln Ala Val Glu Ala Ala
                   165                 170                 175
   Thr Lys Val Ser Ser Glu Phe Glu Gln Asp Pro Gly Asp Gly Leu Leu
               180                 185                 190
   Gly Leu Ala Phe Ser Ser Ile Asn Thr Val Gln Pro Lys Pro Gln Thr
           195                 200                 205
   Thr Phe Phe Asp Thr Val Lys Ser Ser Leu Ala Lys Pro Leu Phe Ala
       210                 215                 220
   Val Thr Leu Lys His Asn Glu Pro Gly Ser Tyr Asp Phe Gly Tyr Ile
   225                 230                 235                 240
   Asp Asn Thr Lys Tyr Lys Gly Ser Ile Gln Tyr Thr Pro Val Asp Asn
                   245                 250                 255
   Ser Gln Gly Phe Trp Gln Phe Thr Ala Asp Gly Tyr Ser Ile Gly Gly
               260                 265                 270
   Ser Ser Gly Asn Gly Gly Ser Ile Ser Gly Ile Ala Asp Thr Gly Thr
           275                 280                 285
   Thr Leu Leu Leu Leu Asp Asp Gln Ile Val Glu Glu Tyr Tyr Gln Asn
       290                 295                 300
   Val Asp Gly Ala Gln Asn Asp Gln Ser Ala Gly Gly Tyr Thr Phe Pro
   305                 310                 315                 320
   Cys Asp Ala Gln Leu Pro Glu Leu Ser Phe Thr Ile Gly Gln Tyr Thr
                   325                 330                 335
   Ala Asn Val Pro Ala Glu Tyr Leu Asn Phe Gln Pro Leu Ser Gln Gly
               340                 345                 350
   Ser Gln Thr Cys Phe Gly Gly Leu Gln Ser Asn Gln Gly Ile Gly Phe
           355                 360                 365
   Ser Ile Phe Gly Asp Val Phe Leu Lys Ser Gln Tyr Val Val Phe Asp
       370                 375                 380
   Ser Asn Gly Pro Gln Leu Gly Phe Ala Ala Gln Ala
   385                 390                 395
<210> 5
   <211> 1366
   <212> DNA
   <213> Geosmithia cylindrospora
<400> 5
   atgggtgtgt ccaaagctgt cgtgactgtg ctcctctgct ccgctttggc ggccgcagcc      60
   cctacttcga cgcagacctt ctcgttcaac caggtggccg ttccccgaac gaaacctcgt     120
   gctcctccgg ctgccgagta tgcgcgtgct ctgcgcaagt acaaggccac cgtgcctgca     180
   tccgtagccg ccgctgccgt cagtggaacg gcaaccaaca cgccgacgca agacgatgag     240
   gagtacatca ctccagtgac ggccggcaag agcactctga acctggactt tgacacggga     300
   tcggccgatc tgtaagcaca tatctaaaca cagctgctga agaggcttct tcgctgacac     360
   tacgtatcta ggtgggtgtt ttcggacgag actcccactt ccgaggccca gggccactcc     420
   acctacgtgc ccagctcgtc ggccaagaag atgagtggct acagctggag catctcctat     480
   ggcgacggca gctcggccag cggcgacgtc taccaggaca ccgtcaccgt cggcggcgtg     540
   acggcctccc agcaggctgt ggaggcggcc accaaggtca gctcccagtt cactcaggac     600
   acgaacaacg atggcctgct aggattggct tttagcagca tcaacacagg tgagctggct     660
   tccctctttt ttttttttcc tttcttctcg ttttatttgc tttatttcta atggatggta      720
   tatagtctcg cctcaaccgc agaagacctt cttcgacaac gtcaagtcac agctcgacaa     780
   gcccctattt gcggtcacgc tgaagcacca ggcccccggc acgtatgact ttggctacat     840
   cgactccagc cggtacaagg gcgacattgc ctacactgct gtcgacaact cgcagggctt     900
   ctggtccttc acggcggatg actactcgat tggtggctcc agtcagggca gctccattac     960
   tggcattgct ggtaggtcat ctcgcagaga aacaaaaata aataaatccg ggctaatgag    1020
   ttatgcagac actggcacca cccttctcct cctggacgac aacgtcgtca acgcctacta    1080
   ctcccaggtg aacagcgcgc agcaggacaa cagcgctggc ggctgggtat ttgactgcag    1140
   cgacaatctg ccaaccttct ccgtcaccat cggcaactac gaggccgtcg tgccggccga    1200
   gttcatcaac tttgggccgg ccgacaacac cggttccacc tgcttcggcg ggatccagtc    1260
   gaaccagggc atcggcttct ctatctttgg ggacgtcttc ctcaagagcc agtacgtcgt    1320
   gtttgacgcc agtgggcctc agctgggctt tgctgctcag gcgtga                  1366
<210> 6
   <211> 390
   <212> PRT
   <213> Geosmithia cylindrospora
<400> 6
   Met Gly Val Ser Lys Ala Val Val Thr Val Leu Leu Cys Ser Ala Leu
   1               5                   10                  15
   Ala Ala Ala Ala Pro Thr Ser Thr Gln Thr Phe Ser Phe Asn Gln Val
               20                  25                  30
   Ala Val Pro Arg Thr Lys Pro Arg Ala Pro Pro Ala Ala Glu Tyr Ala
           35                  40                  45
   Arg Ala Leu Arg Lys Tyr Lys Ala Thr Val Pro Ala Ser Val Ala Ala
       50                  55                  60
   Ala Ala Val Ser Gly Thr Ala Thr Asn Thr Pro Thr Gln Asp Asp Glu
   65                  70                  75                  80
   Glu Tyr Ile Thr Pro Val Thr Ala Gly Lys Ser Thr Leu Asn Leu Asp
                   85                  90                  95
   Phe Asp Thr Gly Ser Ala Asp Leu Trp Val Phe Ser Asp Glu Thr Pro
               100                 105                 110
   Thr Ser Glu Ala Gln Gly His Ser Thr Tyr Val Pro Ser Ser Ser Ala
           115                 120                 125
   Lys Lys Met Ser Gly Tyr Ser Trp Ser Ile Ser Tyr Gly Asp Gly Ser
       130                 135                 140
   Ser Ala Ser Gly Asp Val Tyr Gln Asp Thr Val Thr Val Gly Gly Val
   145                 150                 155                 160
   Thr Ala Ser Gln Gln Ala Val Glu Ala Ala Thr Lys Val Ser Ser Gln
                   165                 170                 175
   Phe Thr Gln Asp Thr Asn Asn Asp Gly Leu Leu Gly Leu Ala Phe Ser
               180                 185                 190
   Ser Ile Asn Thr Val Ser Pro Gln Pro Gln Lys Thr Phe Phe Asp Asn
           195                 200                 205
   Val Lys Ser Gln Leu Asp Lys Pro Leu Phe Ala Val Thr Leu Lys His
       210                 215                 220
   Gln Ala Pro Gly Thr Tyr Asp Phe Gly Tyr Ile Asp Ser Ser Arg Tyr
   225                 230                 235                 240
   Lys Gly Asp Ile Ala Tyr Thr Ala Val Asp Asn Ser Gln Gly Phe Trp
                   245                 250                 255
   Ser Phe Thr Ala Asp Asp Tyr Ser Ile Gly Gly Ser Ser Gln Gly Ser
               260                 265                 270
   Ser Ile Thr Gly Ile Ala Asp Thr Gly Thr Thr Leu Leu Leu Leu Asp
           275                 280                 285
   Asp Asn Val Val Asn Ala Tyr Tyr Ser Gln Val Asn Ser Ala Gln Gln
       290                 295                 300
   Asp Asn Ser Ala Gly Gly Trp Val Phe Asp Cys Ser Asp Asn Leu Pro
   305                 310                 315                 320
   Thr Phe Ser Val Thr Ile Gly Asn Tyr Glu Ala Val Val Pro Ala Glu
                   325                 330                 335
   Phe Ile Asn Phe Gly Pro Ala Asp Asn Thr Gly Ser Thr Cys Phe Gly
               340                 345                 350
   Gly Ile Gln Ser Asn Gln Gly Ile Gly Phe Ser Ile Phe Gly Asp Val
           355                 360                 365
   Phe Leu Lys Ser Gln Tyr Val Val Phe Asp Ala Ser Gly Pro Gln Leu
       370                 375                 380
   Gly Phe Ala Ala Gln Ala
   385                 390
<210> 7
   <211> 327
   <212> PRT
   <213> Rasamsonia cylindrospora
<400> 7
   Ala Ala Ala Val Ser Gly Thr Ala Thr Asn Thr Pro Thr Ser Gly Asp
   1               5                   10                  15
   Glu Glu Tyr Ile Thr Pro Val Thr Ala Gly Lys Ser Thr Leu Asn Leu
               20                  25                  30
   Asp Phe Asp Thr Gly Ser Ala Asp Leu Trp Val Phe Ser Ser Gln Thr
           35                  40                  45
   Pro Thr Ser Glu Ala Glu Gly His Ser Thr Tyr Val Pro Ser Ser Ser
       50                  55                  60
   Ala Thr Lys Met Arg Gly Tyr Ser Trp Ser Ile Ser Tyr Gly Asp Gly
   65                  70                  75                  80
   Ser Ser Ala Ser Gly Asp Val Tyr Gln Asp Thr Val Thr Val Gly Gly
                   85                  90                  95
   Val Thr Ala Ser Lys Gln Ala Val Glu Ala Ala Glu Lys Val Ser Ser
               100                 105                 110
   Glu Phe Thr Gln Asp Thr Ala Ser Asp Gly Leu Leu Gly Leu Ala Phe
           115                 120                 125
   Ser Ser Ile Asn Thr Val Ser Pro Glu Pro Gln Gln Thr Phe Phe Asp
       130                 135                 140
   Asn Val Lys Ser Gln Leu Asp Lys Pro Leu Phe Ala Val Thr Leu Lys
   145                 150                 155                 160
   His Gln Ala Pro Gly Thr Tyr Asp Phe Gly Tyr Ile Asp Ser Ser Arg
                   165                 170                 175
   Tyr Thr Gly Ser Ile Ala Tyr Thr Asp Val Asp Asn Ser Gln Gly Phe
               180                 185                 190
   Trp Ser Phe Thr Ala Asp Asp Tyr Ala Ile Gly Gly Glu Ser Ala Gly
           195                 200                 205
   Ser Ser Ile Thr Gly Ile Ala Asp Thr Gly Thr Thr Leu Leu Leu Leu
       210                 215                 220
   Asp Asp Asp Val Val Ser Asn Tyr Tyr Ser Gly Val Asp Gly Ala Gln
   225                 230                 235                 240
   Asn Ser Asn Tyr Ala Gly Gly Tyr Val Phe Pro Cys Asp Ala Asp Leu
                   245                 250                 255
   Pro Thr Phe Ser Ile Thr Ile Gly Ser His Gln Ala Val Val Pro Ala
               260                 265                 270
   Glu Phe Ile Asn Phe Gly Asp Asp Gly Thr Gly Asp Gly Asn Cys Phe
           275                 280                 285
   Gly Gly Ile Gln Ser Asn Ser Gly Ile Gly Phe Ser Ile Phe Gly Asp
       290                 295                 300
   Val Phe Leu Lys Ser Gln Tyr Val Val Phe Asp Ser Glu Gly Pro Gln
   305                 310                 315                 320
   Leu Gly Phe Ala Lys Gln Ala
                   325
<210> 8
   <211> 329
   <212> PRT
   <213> Rasamsonia composticola
<400> 8
   Ala Ala Ser Gly Thr Gln Ser Gly Ser Ala Ala Asn Thr Pro Val Ala
   1               5                   10                  15
   Gly Asp Ser Leu Tyr Leu Thr Pro Val Thr Val Gly Gln Ser Thr Leu
               20                  25                  30
   Asn Leu Asp Phe Asp Thr Gly Ser Ala Asp Leu Trp Val Phe Ser Glu
           35                  40                  45
   Glu Thr Pro Ser Ser Glu Arg Gly Asn His Ala Ile Tyr Lys Pro Ser
       50                  55                  60
   Ser Ser Ala Lys Lys Leu Asn Gly Tyr Thr Trp Ser Ile Ser Tyr Gly
   65                  70                  75                  80
   Asp Gly Ser Ser Ala Ser Gly Asp Val Tyr Gln Asp Ser Val Ser Val
                   85                  90                  95
   Gly Gly Val Asn Ala Ser Ser Gln Ala Val Glu Ala Ala Thr Lys Val
               100                 105                 110
   Ser Ser Glu Phe Glu Gln Asp Pro Gly Asp Gly Leu Leu Gly Leu Ala
           115                 120                 125
   Phe Ser Ser Ile Asn Thr Val Gln Pro Lys Pro Gln Thr Thr Phe Phe
       130                 135                 140
   Asp Thr Val Lys Ser Ser Leu Ala Lys Pro Leu Phe Ala Val Thr Leu
   145                 150                 155                 160
   Lys His Asn Glu Pro Gly Ser Tyr Asp Phe Gly Tyr Ile Asp Asn Thr
                   165                 170                 175
   Lys Tyr Lys Gly Ser Ile Gln Tyr Thr Pro Val Asp Asn Ser Gln Gly
               180                 185                 190
   Phe Trp Gln Phe Thr Ala Asp Gly Tyr Ser Ile Gly Gly Ser Ser Gly
           195                 200                 205
   Asn Gly Gly Ser Ile Ser Gly Ile Ala Asp Thr Gly Thr Thr Leu Leu
       210                 215                 220
   Leu Leu Asp Asp Gln Ile Val Glu Glu Tyr Tyr Gln Asn Val Asp Gly
   225                 230                 235                 240
   Ala Gln Asn Asp Gln Ser Ala Gly Gly Tyr Thr Phe Pro Cys Asp Ala
                   245                 250                 255
   Gln Leu Pro Glu Leu Ser Phe Thr Ile Gly Gln Tyr Thr Ala Asn Val
               260                 265                 270
   Pro Ala Glu Tyr Leu Asn Phe Gln Pro Leu Ser Gln Gly Ser Gln Thr
           275                 280                 285
   Cys Phe Gly Gly Leu Gln Ser Asn Gln Gly Ile Gly Phe Ser Ile Phe
       290                 295                 300
   Gly Asp Val Phe Leu Lys Ser Gln Tyr Val Val Phe Asp Ser Asn Gly
   305                 310                 315                 320
   Pro Gln Leu Gly Phe Ala Ala Gln Ala
                   325
<210> 9
   <211> 327
   <212> PRT
   <213> Geosmithia cylindrospora
<400> 9
   Ala Ala Ala Val Ser Gly Thr Ala Thr Asn Thr Pro Thr Gln Asp Asp
   1 5 10 15
   Glu Glu Tyr Ile Thr Pro Val Thr Ala Gly Lys Ser Thr Leu Asn Leu
   20 25 30
   Asp Phe Asp Thr Gly Ser Ala Asp Leu Trp Val Phe Ser Asp Glu Thr
   35 40 45
   Pro Thr Ser Glu Ala Gln Gly His Ser Thr Tyr Val Pro Ser Ser Ser
   50 55 60
   Ala Lys Lys Met Ser Gly Tyr Ser Trp Ser Ile Ser Tyr Gly Asp Gly
   65 70 75 80
   Ser Ser Ala Ser Gly Asp Val Tyr Gln Asp Thr Val Thr Val Gly Gly
   85 90 95
   Val Thr Ala Ser Gln Gln Ala Val Glu Ala Ala Thr Lys Val Ser Ser
   100 105 110
   Gln Phe Thr Gln Asp Thr Asn Asn Asp Gly Leu Leu Gly Leu Ala Phe
   115 120 125
   Ser Ser Ile Asn Thr Val Ser Pro Gln Pro Gln Lys Thr Phe Phe Asp
   130 135 140
   Asn Val Lys Ser Gln Leu Asp Lys Pro Leu Phe Ala Val Thr Leu Lys
   145 150 155 160
   His Gln Ala Pro Gly Thr Tyr Asp Phe Gly Tyr Ile Asp Ser Ser Arg
   165 170 175
   Tyr Lys Gly Asp Ile Ala Tyr Thr Ala Val Asp Asn Ser Gln Gly Phe
   180 185 190
   Trp Ser Phe Thr Ala Asp Asp Tyr Ser Ile Gly Gly Ser Ser Gln Gly
   195 200 205
   Ser Ser Ile Thr Gly Ile Ala Asp Thr Gly Thr Thr Leu Leu Leu Leu
   210 215 220
   Asp Asp Asn Val Val Asn Ala Tyr Tyr Ser Gln Val Asn Ser Ala Gln
   225 230 235 240
   Gln Asp Asn Ser Ala Gly Gly Trp Val Phe Asp Cys Ser Asp Asn Leu
   245 250 255
   Pro Thr Phe Ser Val Thr Ile Gly Asn Tyr Glu Ala Val Val Pro Ala
   260 265 270
   Glu Phe Ile Asn Phe Gly Pro Ala Asp Asn Thr Gly Ser Thr Cys Phe
   275 280 285
   Gly Gly Ile Gln Ser Asn Gln Gly Ile Gly Phe Ser Ile Phe Gly Asp
   290 295 300
   Val Phe Leu Lys Ser Gln Tyr Val Val Phe Asp Ala Ser Gly Pro Gln
   305 310 315 320
   Leu Gly Phe Ala Ala Gln Ala
   325
<210> 10
   <211> 328
   <212> PRT
   <213> Aspergillus fumigatus
<400> 10
   Ala Ala Ala Ser Ser Gly Ser Ala Val Thr Thr Pro Glu Gln Tyr Asp
   1               5                   10                  15
   Ser Glu Tyr Leu Thr Pro Val Lys Val Gly Gly Thr Thr Leu Asn Leu
               20                  25                  30
   Asp Phe Asp Thr Gly Ser Ala Asp Leu Trp Val Phe Ser Ser Glu Leu
           35                  40                  45
   Ser Ala Ser Gln Ser Ser Gly His Ala Ile Tyr Lys Pro Ser Ala Asn
       50                  55                  60
   Ala Gln Lys Leu Asn Gly Tyr Thr Trp Lys Ile Gln Tyr Gly Asp Gly
   65                  70                  75                  80
   Ser Ser Ala Ser Gly Asp Val Tyr Lys Asp Thr Val Thr Val Gly Gly
                   85                  90                  95
   Val Thr Ala Gln Ser Gln Ala Val Glu Ala Ala Ser His Ile Ser Ser
               100                 105                 110
   Gln Phe Val Gln Asp Lys Asp Asn Asp Gly Leu Leu Gly Leu Ala Phe
           115                 120                 125
   Ser Ser Ile Asn Thr Val Ser Pro Arg Pro Gln Thr Thr Phe Phe Asp
       130                 135                 140
   Thr Val Lys Ser Gln Leu Asp Ser Pro Leu Phe Ala Val Thr Leu Lys
   145                 150                 155                 160
   Tyr His Ala Pro Gly Thr Tyr Asp Phe Gly Tyr Ile Asp Asn Ser Lys
                   165                 170                 175
   Phe Gln Gly Glu Leu Thr Tyr Thr Asp Val Asp Ser Ser Gln Gly Phe
               180                 185                 190
   Trp Met Phe Thr Ala Asp Gly Tyr Gly Val Gly Asn Gly Ala Pro Asn
           195                 200                 205
   Ser Asn Ser Ile Ser Gly Ile Ala Asp Thr Gly Thr Thr Leu Leu Leu
       210                 215                 220
   Leu Asp Asp Ser Val Val Ala Asp Tyr Tyr Arg Gln Val Ser Gly Ala
   225                 230                 235                 240
   Lys Asn Ser Asn Gln Tyr Gly Gly Tyr Val Phe Pro Cys Ser Thr Lys
                   245                 250                 255
   Leu Pro Ser Phe Thr Thr Val Ile Gly Gly Tyr Asn Ala Val Val Pro
               260                 265                 270
   Gly Glu Tyr Ile Asn Tyr Ala Pro Val Thr Asp Gly Ser Ser Thr Cys
           275                 280                 285
   Tyr Gly Gly Ile Gln Ser Asn Ser Gly Leu Gly Phe Ser Ile Phe Gly
       290                 295                 300
   Asp Ile Phe Leu Lys Ser Gln Tyr Val Val Phe Asp Ser Gln Gly Pro
   305                 310                 315                 320
   Arg Leu Gly Phe Ala Pro Gln Ala
                   325
<210> 11
   <211> 327
   <212> PRT
   <213> Aspergillus kawachii
<400> 11
   Ala Ala Ser Lys Gly Ser Ala Val Thr Thr Pro Gln Asn Asn Asp Glu
   1               5                   10                  15
   Glu Tyr Leu Thr Pro Val Thr Val Gly Lys Ser Thr Leu His Leu Asp
               20                  25                  30
   Phe Asp Thr Gly Ser Ala Asp Leu Trp Val Phe Ser Asp Glu Leu Pro
           35                  40                  45
   Ser Ser Glu Arg Thr Gly His Asp Val Tyr Thr Pro Ser Ser Ser Ala
       50                  55                  60
   Thr Lys Leu Ser Gly Tyr Ser Trp Asp Ile Ser Tyr Gly Asp Gly Ser
   65                  70                  75                  80
   Ser Ala Ser Gly Asp Val Tyr Arg Asp Thr Val Thr Val Gly Gly Val
                   85                  90                  95
   Thr Thr Asn Lys Gln Ala Val Glu Ala Ala Ser Lys Ile Ser Ser Glu
               100                 105                 110
   Phe Val Gln Asp Thr Ala Asn Asp Gly Leu Leu Gly Leu Ala Phe Ser
           115                 120                 125
   Ser Ile Asn Thr Val Gln Pro Lys Ala Gln Thr Thr Phe Phe Asp Thr
       130                 135                 140
   Val Lys Ser Gln Leu Asp Ser Pro Leu Phe Ala Val Gln Leu Lys His
   145                 150                 155                 160
   Asp Ala Pro Gly Val Tyr Asp Phe Gly Tyr Ile Asp Asp Ser Lys Tyr
                   165                 170                 175
   Thr Gly Ser Ile Thr Tyr Thr Asp Ala Asp Ser Ser Gln Gly Tyr Trp
               180                 185                 190
   Gly Phe Asn Pro Asp Gly Tyr Ser Ile Gly Asp Ser Ser Ser Ser Ser
           195                 200                 205
   Ser Gly Phe Ser Ala Ile Ala Asp Thr Gly Thr Thr Leu Ile Leu Leu
       210                 215                 220
   Asp Asp Glu Ile Val Ser Ala Tyr Tyr Glu Gln Val Asp Gly Ala Gln
   225                 230                 235                 240
   Glu Ser Asn Glu Ala Gly Gly Tyr Val Phe Ser Cys Ser Thr Thr Pro
                   245                 250                 255
   Pro Asp Phe Thr Val Ile Ile Gly Asp Tyr Lys Ala Val Val Pro Gly
               260                 265                 270
   Lys Tyr Ile Asn Tyr Ala Pro Ile Ser Thr Gly Ser Ser Thr Cys Phe
           275                 280                 285
   Gly Gly Ile Gln Ser Asn Ser Gly Leu Gly Leu Ser Ile Leu Gly Asp
       290                 295                 300
   Val Phe Leu Lys Ser Gln Tyr Val Val Phe Asn Ser Glu Gly Pro Lys
   305                 310                 315                 320
   Leu Gly Phe Ala Ala Gln Ala
                   325
<210> 12
   <211> 328
   <212> PRT
   <213> Aspergillus lentulus
<400> 12
   Ala Ala Ala Ser Ser Gly Ser Ala Val Thr Thr Pro Glu Gln Tyr Asp
   1               5                   10                  15
   Ser Glu Tyr Leu Thr Pro Val Lys Val Gly Gly Thr Thr Leu Asn Leu
               20                  25                  30
   Asp Phe Asp Thr Gly Ser Ala Asp Leu Trp Val Phe Ser Ser Glu Leu
           35                  40                  45
   Ser Ala Ser Glu Ser Ser Gly His Ala Ile Tyr Lys Pro Ser Thr Ser
       50                  55                  60
   Ala Gln Lys Leu Asn Gly Tyr Thr Trp Lys Ile Gln Tyr Gly Asp Gly
   65                  70                  75                  80
   Ser Gly Ala Ser Gly Asp Val Tyr Lys Asp Thr Val Thr Val Gly Gly
                   85                  90                  95
   Val Thr Ala Lys Ser Gln Ala Val Glu Ala Ala Ser Gln Ile Ser Ser
               100                 105                 110
   Gln Phe Val Gln Asp Lys Asp Asn Asp Gly Leu Leu Gly Leu Ala Phe
           115                 120                 125
   Ser Ser Ile Asn Thr Val Ser Pro Arg Pro Gln Thr Thr Phe Phe Asp
       130                 135                 140
   Thr Val Lys Ser Gln Leu Asp Ser Pro Leu Phe Ala Val Thr Leu Lys
   145                 150                 155                 160
   Tyr His Ala Pro Gly Thr Tyr Asp Phe Gly Tyr Ile Asp Asn Ser Lys
                   165                 170                 175
   Phe Gln Gly Gln Leu Thr Tyr Thr Asp Val Asp Asn Ser Gln Gly Phe
               180                 185                 190
   Trp Met Phe Thr Ala Asp Gly Tyr Gly Val Gly Asp Gly Ala Pro Asn
           195                 200                 205
   His Asn Gln Ile Ser Gly Ile Ala Asp Thr Gly Thr Thr Leu Leu Leu
       210                 215                 220
   Leu Asp Asp Ser Val Val Ala Ala Tyr Tyr Arg Gln Val Ser Gly Ala
   225                 230                 235                 240
   Lys Asn Ser Asn Glu Tyr Gly Gly Tyr Val Phe Pro Cys Ser Thr Lys
                   245                 250                 255
   Leu Pro Ser Phe Thr Thr Val Ile Gly Gly Tyr Asn Ala Val Val Pro
               260                 265                 270
   Gly Glu Tyr Ile Asn Phe Ala Pro Val Thr Asp Gly Ser Ser Thr Cys
           275                 280                 285
   Phe Gly Gly Ile Gln Ser Asn Ser Gly Leu Gly Phe Ser Ile Phe Gly
       290                 295                 300
   Asp Ile Phe Leu Lys Ser Gln Tyr Val Val Phe Asp Ser Gln Gly Pro
   305                 310                 315                 320
   Arg Leu Gly Phe Ala Pro Gln Ala
                   325
<210> 13
   <211> 327
   <212> PRT
   <213> Aspergillus ochraceoroseus
<400> 13
   Ala Ala Ala Glu Ser Gly Thr Ala Thr Asn Asn Pro Ser Ser Gly Asp
   1               5                   10                  15
   Glu Glu Tyr Val Val Gln Val Thr Val Gly Gly Ser Lys Leu Asn Leu
               20                  25                  30
   Asp Phe Asp Thr Gly Ser Ala Asp Leu Trp Val Phe Ser Thr Glu Thr
           35                  40                  45
   Pro Ser Ser Glu Ser Thr Gly His Asn Ile Tyr Lys Pro Ser Ser Ser
       50                  55                  60
   Ala Glu Lys Leu Ser Gly Tyr Thr Trp Ser Ile Ser Tyr Gly Asp Gly
   65                  70                  75                  80
   Ser Ser Ala Ser Gly Asn Val Tyr Gln Asp Ser Val Thr Val Gly Gly
                   85                  90                  95
   Val Thr Ala Thr Lys Gln Ala Val Glu Ala Ala Glu Thr Val Ser Ser
               100                 105                 110
   Glu Phe Thr Glu Asp Thr Ser Asn Asp Gly Leu Leu Gly Leu Ala Phe
           115                 120                 125
   Ser Ser Ile Asn Thr Val Glu Pro Thr Ala Gln Ile Thr Phe Phe Asp
       130                 135                 140
   Thr Val Lys Thr Gln Leu Ala Ser Pro Leu Phe Ala Val Thr Leu Lys
   145                 150                 155                 160
   His Asp Ala Pro Gly Tyr Tyr Asp Phe Gly Phe Ile Asp Ser Ser Arg
                   165                 170                 175
   Tyr Thr Gly Asn Leu Val Tyr Thr Glu Val Asp Asn Ser Asp Gly Phe
               180                 185                 190
   Trp Ser Phe Thr Ser Asp Ser Tyr Ser Ile Gly Gly Glu His Ala Gly
           195                 200                 205
   Ser Gly Leu Thr Gly Ile Ala Asp Thr Gly Thr Thr Leu Leu Leu Leu
       210                 215                 220
   Asp Asp Ser Val Val Ser Ala Tyr Tyr Ala Gln Val Ser Gly Ser Glu
   225                 230                 235                 240
   Asp Ser Ser Ser Asn Gly Gly Tyr Thr Phe Pro Cys Thr Ala Asp Leu
                   245                 250                 255
   Pro Asp Phe Ser Ile Thr Ile Ser Gly Tyr Glu Ala Val Val Pro Gly
               260                 265                 270
   Lys Tyr Ile Asn Phe Glu Ala Thr Asp Ser Ser Asn Lys Thr Cys Phe
           275                 280                 285
   Gly Gly Ile Gln Ser Asn Ser Gly Ile Gly Phe Ser Ile Phe Gly Asp
       290                 295                 300
   Val Phe Leu Lys Ser Gln Tyr Ile Val Phe Asp Ser Ser Val Pro Gln
   305                 310                 315                 320
   Leu Gly Phe Ala Ala Gln Ser
                   325
<210> 14
   <211> 326
   <212> PRT
   <213> Aspergillus oryzae
<400> 14
   Ala Ala Ala Ala Ala Thr Gly Ser Val Thr Thr Asn Pro Thr Ser Asn
   1               5                   10                  15
   Asp Glu Glu Tyr Ile Thr Gln Val Thr Val Gly Asp Asp Thr Leu Gly
               20                  25                  30
   Leu Asp Phe Asp Thr Gly Ser Ala Asp Leu Trp Val Phe Ser Ser Gln
               35                  40                  45
   Thr Pro Ser Ser Glu Arg Ser Gly His Asp Tyr Tyr Thr Pro Gly Ser
       50                  55                  60
   Ser Ala Gln Lys Ile Asp Gly Ala Thr Trp Ser Ile Ser Tyr Gly Asp
   65                  70                  75                  80
   Gly Ser Ser Ala Ser Gly Asp Val Tyr Lys Asp Lys Val Thr Val Gly
                   85                  90                  95
   Gly Val Ser Tyr Asp Ser Gln Ala Val Glu Ser Ala Glu Lys Val Ser
               100                 105                 110
   Ser Glu Phe Thr Gln Asp Thr Ala Asn Asp Gly Leu Leu Gly Leu Ala
           115                 120                 125
   Phe Ser Ser Ile Asn Thr Val Gln Pro Thr Pro Gln Lys Thr Phe Phe
       130                 135                 140
   Asp Asn Val Lys Ser Ser Leu Ser Glu Pro Ile Phe Ala Val Ala Leu
   145                 150                 155                 160
   Lys His Asn Ala Pro Gly Val Tyr Asp Phe Gly Tyr Thr Asp Ser Ser
                   165                 170                 175
   Lys Tyr Thr Gly Ser Ile Thr Tyr Thr Asp Val Asp Asn Ser Gln Gly
               180                 185                 190
   Phe Trp Gly Phe Thr Ala Asp Gly Tyr Ser Ile Gly Ser Asp Ser Ser
           195                 200                 205
   Ser Asp Ser Ile Thr Gly Ile Ala Asp Thr Gly Thr Thr Leu Leu Leu
       210                 215                 220
   Leu Asp Asp Ser Ile Val Asp Ala Tyr Tyr Glu Gln Val Asn Gly Ala
   225                 230                 235                 240
   Ser Tyr Asp Ser Ser Gln Gly Gly Tyr Val Phe Pro Ser Ser Ala Ser
                   245                 250                 255
   Leu Pro Asp Phe Ser Val Thr Ile Gly Asp Tyr Thr Ala Thr Val Pro
               260                 265                 270
   Gly Glu Tyr Ile Ser Phe Ala Asp Val Gly Asn Gly Gln Thr Phe Gly
           275                 280                 285
   Gly Ile Gln Ser Asn Ser Gly Ile Gly Phe Ser Ile Phe Gly Asp Val
       290                 295                 300
   Phe Leu Lys Ser Gln Tyr Val Val Phe Asp Ala Ser Gly Pro Arg Leu
   305                 310                 315                 320
   Gly Phe Ala Ala Gln Ala
                   325
<210> 15
   <211> 327
   <212> PRT
   <213> Aspergillus saitoi
<400> 15
   Ala Ala Ser Lys Gly Ser Ala Val Thr Thr Pro Gln Asn Asn Asp Glu
   1               5                   10                  15
   Glu Tyr Leu Thr Pro Val Thr Val Gly Lys Ser Thr Leu His Leu Asp
               20                  25                  30
   Phe Asp Thr Gly Ser Ala Asp Leu Trp Val Phe Ser Asp Glu Leu Pro
           35                  40                  45
   Ser Ser Glu Gln Thr Gly His Asp Leu Tyr Thr Pro Ser Ser Ser Ala
       50                  55                  60
   Thr Lys Leu Ser Gly Tyr Ser Trp Asp Ile Ser Tyr Gly Asp Gly Ser
   65                  70                  75                  80
   Ser Ala Ser Gly Asp Val Tyr Arg Asp Thr Val Thr Val Gly Gly Val
                   85                  90                  95
   Thr Thr Asn Lys Gln Ala Val Glu Ala Ala Ser Lys Ile Ser Ser Glu
               100                 105                 110
   Phe Val Gln Asp Thr Ala Asn Asp Gly Leu Leu Gly Leu Ala Phe Ser
           115                 120                 125
   Ser Ile Asn Thr Val Gln Pro Lys Ala Gln Thr Thr Phe Phe Asp Thr
       130                 135                 140
   Val Lys Ser Gln Leu Asp Ser Pro Leu Phe Ala Val Gln Leu Lys His
   145                 150                 155                 160
   Asp Ala Pro Gly Val Tyr Asp Phe Gly Tyr Ile Asp Asp Ser Lys Tyr
                   165                 170                 175
   Thr Gly Ser Ile Thr Tyr Thr Asp Ala Asp Ser Ser Gln Gly Tyr Trp
               180                 185                 190
   Gly Phe Ser Thr Asp Gly Tyr Ser Ile Gly Asp Gly Ser Ser Ser Ser
           195                 200                 205
   Ser Gly Phe Ser Ala Ile Ala Asp Thr Gly Thr Thr Leu Ile Leu Leu
       210                 215                 220
   Asp Asp Glu Ile Val Ser Ala Tyr Tyr Glu Gln Val Ser Gly Ala Gln
   225                 230                 235                 240
   Glu Ser Tyr Glu Ala Gly Gly Tyr Val Phe Ser Cys Ser Thr Asp Leu
                   245                 250                 255
   Pro Asp Phe Thr Val Val Ile Gly Asp Tyr Lys Ala Val Val Pro Gly
               260                 265                 270
   Lys Tyr Ile Asn Tyr Ala Pro Val Ser Thr Gly Ser Ser Thr Cys Tyr
           275                 280                 285
   Gly Gly Ile Gln Ser Asn Ser Gly Leu Gly Leu Ser Ile Leu Gly Asp
       290                 295                 300
   Val Phe Leu Lys Ser Gln Tyr Val Val Phe Asn Ser Glu Gly Pro Lys
   305                 310                 315                 320
   Leu Gly Phe Ala Ala Gln Ala
                   325
<210> 16
   <211> 328
   <212> PRT
   <213> Neosartorya udagawae
<400> 16
   Ala Ala Ala Ser Ser Gly Ser Ala Val Thr Thr Pro Glu Gln Tyr Asp
   1               5                   10                  15
   Thr Glu Tyr Leu Thr Pro Val Lys Val Gly Gly Thr Thr Leu Asn Leu
               20                  25                  30
   Asp Phe Asp Thr Gly Ser Ala Asp Leu Trp Val Phe Ser Ser Glu Leu
           35                  40                  45
   Ser Ala Ser Gln Ser Ser Gly His Ala Ile Tyr Lys Pro Ser Ser Thr
       50                  55                  60
   Ala Lys Lys Leu Ser Gly Tyr Ser Trp Gln Ile Ser Tyr Gly Asp Gly
   65                  70                  75                  80
   Ser Ser Ala Ser Gly Asp Val Tyr Lys Asp Thr Val Thr Val Gly Gly
                   85                  90                  95
   Val Thr Ala Gln Ser Gln Ala Val Glu Ala Ala Ser Gln Ile Ser Ser
               100                 105                 110
   Gln Phe Val Gln Asp Lys Asp Asn Asp Gly Leu Leu Gly Leu Ala Phe
           115                 120                 125
   Ser Ser Ile Asn Thr Val Ser Pro Arg Pro Gln Thr Thr Phe Phe Asp
       130                 135                 140
   Thr Val Lys Ser Gln Leu Asp Ser Pro Leu Phe Ala Val Thr Leu Lys
   145                 150                 155                 160
   Tyr His Ala Pro Gly Thr Tyr Asp Phe Gly Tyr Ile Asp Asn Ser Lys
                   165                 170                 175
   Tyr Lys Gly Gln Leu Thr Tyr Thr Asp Val Asp Ser Ser Gln Gly Phe
               180                 185                 190
   Trp Met Phe Thr Ala Asp Gly Tyr Gly Val Gly Asn Gly Thr Pro Asn
           195                 200                 205
   Ser Asn Gln Ile Ser Gly Ile Ala Asp Thr Gly Thr Thr Leu Leu Leu
       210                 215                 220
   Leu Asp Asp Ser Val Val Ala Asp Tyr Tyr Arg His Val Thr Gly Ala
   225                 230                 235                 240
   Lys Asn Ser Asn Gln Tyr Gly Gly Tyr Val Phe Pro Cys Ser Thr Ser
                   245                 250                 255
   Leu Pro Ser Phe Thr Thr Val Ile Gly Gly Tyr Lys Ala Val Ile Pro
               260                 265                 270
   Gly Asn Tyr Ile Asn Phe Ala Pro Val Thr Asp Gly Ser Ser Thr Cys
           275                 280                 285
   Phe Gly Gly Ile Gln Ser Asn Ser Gly Leu Gly Phe Ser Ile Phe Gly
       290                 295                 300
   Asp Ile Phe Leu Lys Ser Gln Tyr Val Val Phe Asp Ser Gln Gly Pro
   305                 310                 315                 320
   Arg Leu Gly Phe Ala Ala Gln Ala
                   325
<210> 17
   <211> 329
   <212> PRT
   <213> Paecilomyces byssochlamydoides
<400> 17
   Ala Ala Ala Gly Ser Val Thr Gly Ser Ala Thr Asn Gln Pro Ile Thr
   1               5                   10                  15
   Gly Asp Glu Glu Tyr Val Thr Pro Val Ile Ala Gly Gln Ser Thr Leu
               20                  25                  30
   His Leu Asp Phe Asp Thr Gly Ser Ala Asp Leu Trp Val Phe Ser Ser
           35                  40                  45
   Gln Thr Pro Arg Ser Glu Arg Gly Asn His Ser Ile Tyr Thr Pro Ser
       50                  55                  60
   Ala Ser Ala Lys Lys Leu Asn Gly Tyr Thr Trp Ser Ile Ser Tyr Gly
   65                  70                  75                  80
   Asp Gly Ser Ser Ala Ser Gly Asp Val Tyr Leu Asp Thr Ile Ser Val
                   85                  90                  95
   Gly Gly Val Asn Ala Ser Ser Gln Ala Val Glu Ala Ala Thr Gln Val
               100                 105                 110
   Ser Ser Glu Phe Thr Gln Asp Thr Gly Ser Asp Gly Leu Leu Gly Leu
           115                 120                 125
   Ala Phe Ser Ser Ile Asn Thr Val Gln Pro Gln Arg Gln Thr Thr Phe
       130                 135                 140
   Phe Asp Thr Val Lys Ser Gln Leu Ala Lys Pro Leu Phe Ala Val Thr
   145                 150                 155                 160
   Leu Lys His Asn Ala Pro Gly Ser Tyr Asp Phe Gly Phe Ile Asp Pro
                   165                 170                 175
   Asn Lys Tyr Asn Gly Ser Ile Ala Tyr Thr Gln Val Asp Asn Ser Gln
               180                 185                 190
   Gly Phe Trp Gln Phe Thr Ala Asp Asp Tyr Ala Ile Gly Gly Ser Ser
           195                 200                 205
   Gly Gly Ser Ser Ile Asn Gly Ile Ala Asp Thr Gly Thr Thr Leu Leu
       210                 215                 220
   Leu Leu Asp Asn Ser Ile Val Asn Ala Tyr Tyr Gln Gln Val Gln Gly
   225                 230                 235                 240
   Ala Gln Asn Asp Gln Asn Ala Gly Gly Tyr Val Phe Pro Cys Asn Ala
                   245                 250                 255
   Gln Leu Pro Thr Phe Ser Ile Thr Ile Gly Asn Tyr Glu Ala Val Val
               260                 265                 270
   Pro Ala Glu Phe Leu Asn Phe Gln Pro Val Asp Gln Thr Gly Gln Thr
           275                 280                 285
   Cys Phe Gly Gly Leu Gln Ser Asn Gln Gly Val Gly Phe Ser Ile Phe
       290                 295                 300
   Gly Asp Val Phe Leu Lys Ser Gln Tyr Val Val Phe Asp Ser Asn Gly
   305                 310                 315                 320
   Pro Arg Leu Gly Phe Ala Ala Gln Ala
                   325
<210> 18
   <211> 327
   <212> PRT
   <213> Penicillium camemberti
<400> 18
   Ala Ala Ala Val Ser Gly Ser Ala Ile Thr Thr Pro Glu Asp Asn Asp
   1               5                   10                  15
   Val Glu Tyr Leu Thr Pro Val Lys Val Gly Asp Thr Thr Leu Asn Leu
               20                  25                  30
   Asp Phe Asp Thr Gly Ser Ala Asp Leu Trp Val Phe Ser Ser Glu Leu
           35                  40                  45
   Pro Ser Ser Glu Gln Thr Gly His Asp Val Tyr Lys Val Gly Ala Ser
       50                  55                  60
   Gly Lys Lys Leu Thr Gly Ala Ser Trp Gln Ile Ser Tyr Gly Asp Gly
   65                  70                  75                  80
   Ser Gly Ala Ser Gly Asp Val Tyr Lys Asp Thr Val Val Val Gly Gly
                   85                  90                  95
   Val Lys Ala Thr Ser Gln Ala Val Glu Ala Ala Lys Lys Ile Ser Gln
               100                 105                 110
   Gln Phe Val Glu Asp Lys Ser Asn Asp Gly Leu Leu Gly Leu Ala Phe
           115                 120                 125
   Ser Ser Ile Asn Thr Val Ser Pro Asn Pro Gln Thr Thr Phe Phe Asp
       130                 135                 140
   Thr Val Lys Asp Asp Leu Asp Lys Pro Leu Phe Ala Val Thr Leu Lys
   145                 150                 155                 160
   His Gly Ala Pro Gly Thr Phe Asp Phe Gly Phe Ile Asp Ser Glu Lys
                   165                 170                 175
   Phe Thr Gly Ser Val Ala Tyr Thr Asp Val Asp Asp Ser Asp Gly Phe
               180                 185                 190
   Trp Ser Phe Thr Ala Asp Ser His Lys Ile Gly Ser Gly Ser Ala Gly
           195                 200                 205
   Ser Ser Ile Thr Gly Ile Ala Asp Thr Gly Thr Thr Leu Leu Leu Leu
       210                 215                 220
   Pro Asp Ser Val Val Ser Ala Tyr Tyr Gly Lys Val Thr Gly Ala Lys
   225                 230                 235                 240
   Asn Ser Gln Thr Gln Gly Gly Tyr Ile Phe Pro Cys Ser Ala Asp Leu
                   245                 250                 255
   Pro Asp Phe Thr Val Thr Ile Gly Asp Tyr Asp Ala Val Val Pro Gly
               260                 265                 270
   Lys His Ile Asn Tyr Ala Pro Val Ser Thr Gly Ser Ser Ser Cys Phe
           275                 280                 285
   Gly Gly Ile Gln Ser Asn Ser Gly Leu Gly Phe Ser Ile Phe Gly Asp
       290                 295                 300
   Ile Phe Leu Lys Ser Gln Tyr Val Val Phe Asp Ser Glu Gly Pro Arg
   305                 310                 315                 320
   Leu Gly Phe Ala Ala Gln Ala
                   325
<210> 19
   <211> 327
   <212> PRT
   <213> Penicillium roqueforti
<400> 19
   Ala Ala Ala Val Ser Gly Ser Ala Ile Thr Thr Pro Glu Ala Asp Asp
   1               5                   10                  15
   Val Glu Tyr Leu Thr Pro Val Thr Ile Gly Ser Ser Thr Leu Asn Leu
               20                  25                  30
   Asp Phe Asp Thr Gly Ser Ala Asp Leu Trp Val Phe Ser Ser Glu Leu
           35                  40                  45
   Thr Ser Ser Gln Gln Ser Gly His Asp Val Tyr Asn Val Gly Ser Ser
       50                  55                  60
   Gly Thr Lys Leu Ser Gly Ala Ser Trp Ser Ile Ser Tyr Gly Asp Gly
   65                  70                  75                  80
   Ser Ser Ala Ser Gly Asp Val Tyr Lys Asp Thr Val Thr Val Gly Gly
                   85                  90                  95
   Val Lys Ala Thr Gly Gln Ala Val Glu Ala Ala Lys Lys Ile Ser Ser
               100                 105                 110
   Gln Phe Leu Gln Asp Lys Asn Asn Asp Gly Leu Leu Gly Leu Ala Phe
           115                 120                 125
   Ser Ser Ile Asn Thr Val Ser Pro Thr Pro Gln Lys Thr Phe Phe Asp
       130                 135                 140
   Thr Val Lys Ser Ser Leu Gly Glu Pro Leu Phe Ala Val Thr Leu Lys
   145                 150                 155                 160
   His Gly Ala Pro Gly Thr Tyr Asp Phe Gly Tyr Ile Asp Ser Asp Lys
                   165                 170                 175
   Tyr Thr Gly Thr Leu Ala Tyr Ala Asp Val Asp Asp Ser Asp Gly Phe
               180                 185                 190
   Trp Ser Phe Thr Ala Asp Ser Tyr Lys Ile Gly Thr Gly Ala Ala Gly
           195                 200                 205
   Lys Ser Ile Thr Gly Ile Ala Asp Thr Gly Thr Thr Leu Leu Leu Leu
       210                 215                 220
   Asp Ser Ser Ile Val Thr Ala Tyr Tyr Lys Lys Val Thr Gly Ser Gln
   225                 230                 235                 240
   Asn Ser Ser Ser Ala Gly Gly Tyr Ile Phe Pro Cys Ser Ala Thr Leu
                   245                 250                 255
   Pro Asp Phe Thr Val Thr Ile Asn Gly Tyr Asp Ala Val Val Pro Gly
               260                 265                 270
   Lys Tyr Ile Asn Phe Ala Pro Val Ser Thr Gly Ser Ser Ser Cys Tyr
           275                 280                 285
   Gly Gly Ile Gln Ser Asn Ser Gly Ile Gly Phe Ser Ile Phe Gly Asp
       290                 295                 300
   Ile Phe Leu Lys Ser Gln Tyr Val Val Phe Asp Ser Glu Gly Pro Arg
   305                 310                 315                 320
   Leu Gly Phe Ala Ala Gln Ala
                   325
<210> 20
   <211> 327
   <212> PRT
   <213> Penicillium rubens Wisconsin
<400> 20
   Ala Ala Ala Val Ser Gly Ser Ala Ile Thr Thr Pro Glu Glu Asn Asp
   1               5                   10                  15
   Val Glu Tyr Leu Thr Pro Val Lys Ile Gly Glu Ser Thr Leu Asn Leu
               20                  25                  30
   Asp Phe Asp Thr Gly Ser Ala Asp Leu Trp Val Phe Ser Thr Glu Leu
           35                  40                  45
   Ser Ser Ala Glu Gln Ser Gly His Asp Val Tyr Asp Val Ser Ser Ser
       50                  55                  60
   Gly Lys Lys Leu Thr Gly Ala Ser Trp Ser Ile Ser Tyr Gly Asp Gly
   65                  70                  75                  80
   Ser Gly Ala Ser Gly Asp Val Tyr Lys Asp Thr Val Thr Val Gly Gly
                   85                  90                  95
   Val Lys Ala Thr Gly Gln Ala Val Glu Ala Ala Lys Lys Ile Ser Gln
               100                 105                 110
   Gln Phe Val Gln Asp Lys Ser Asn Asp Gly Leu Leu Gly Leu Ala Phe
           115                 120                 125
   Ser Ser Ile Asn Thr Val Ser Pro Lys Pro Gln Thr Thr Phe Phe Asp
       130                 135                 140
   Thr Val Lys Ser Asp Leu Asp Lys Pro Leu Phe Ala Val Thr Leu Lys
   145                 150                 155                 160
   His Gly Ala Pro Gly Thr Tyr Asp Phe Gly Tyr Ile Asp Lys Lys Lys
                   165                 170                 175
   Phe Thr Gly Ser Leu Thr Tyr Thr Asp Val Asp Asn Ser Gln Gly Phe
               180                 185                 190
   Trp Ser Phe Thr Ala Asp Ser Tyr Lys Val Gly Thr Gly Ser Ala Gly
           195                 200                 205
   Pro Ser Ile Glu Gly Ile Ala Asp Thr Gly Thr Thr Leu Leu Leu Leu
       210                 215                 220
   Asp Asp Gly Val Val Ser Asp Tyr Tyr Lys Lys Val Asp Gly Ala Lys
   225                 230                 235                 240
   Asn Asn Tyr Ser Ala Gly Gly Tyr Val Phe Pro Cys Asp Ala Asp Leu
                   245                 250                 255
   Pro Asp Phe Thr Val Thr Ile Gly Ser Tyr Asp Ala Val Val Pro Gly
               260                 265                 270
   Lys His Ile Lys Tyr Ala Pro Val Thr Thr Gly Ser Ser Ser Cys Phe
           275                 280                 285
   Gly Gly Ile Gln Ser Asn Ser Gly Ile Gly Phe Ser Ile Phe Gly Asp
       290                 295                 300
   Ile Phe Leu Lys Ser Gln Tyr Val Val Phe Asp Ala Glu Gly Pro Arg
   305                 310                 315                 320
   Leu Gly Phe Ala Ala Gln Ala
                   325
<210> 21
   <211> 329
   <212> PRT
   <213> Rasamsonia emersonii
<400> 21
   Ala Ala Ser Gly Thr Gln Ser Gly Ser Ala Ala Asn Thr Pro Val Ala
   1               5                   10                  15
   Gly Asp Ser Leu Tyr Leu Thr Pro Val Thr Ile Gly Gln Ser Thr Leu
               20                  25                  30
   Asn Leu Asp Phe Asp Thr Gly Ser Ala Asp Leu Trp Val Phe Ser Asn
           35                  40                  45
   Glu Thr Pro Ser Ser Glu Arg Gly Asn His Ala Ile Tyr Lys Pro Ser
       50                  55                  60
   Ser Thr Ala Lys Lys Leu Asn Gly Tyr Thr Trp Ser Ile Ser Tyr Gly
   65                  70                  75                  80
   Asp Gly Ser Ser Ala Gly Gly Asp Val Tyr Gln Asp Ser Val Ser Val
                   85                  90                  95
   Gly Gly Val Asn Ala Ser Asn Gln Ala Val Glu Ala Ala Thr Lys Val
               100                 105                 110
   Ser Ser Glu Phe Thr Gln Glu Pro Gly Asp Gly Leu Leu Gly Leu Ala
           115                 120                 125
   Phe Ser Ser Ile Asn Thr Val Lys Pro Lys Pro Gln Thr Thr Phe Phe
       130                 135                 140
   Asp Thr Val Lys Ser Ser Leu Ala Lys Pro Leu Phe Ala Val Thr Leu
   145                 150                 155                 160
   Lys His Asn Glu Pro Gly Ser Tyr Asp Phe Gly Tyr Ile Asp Ser Ser
                   165                 170                 175
   Lys Tyr Lys Gly Ser Ile Gln Tyr Thr Gln Val Asp Asn Ser Gln Gly
               180                 185                 190
   Phe Trp Gln Phe Thr Ala Asp Gly Tyr Ser Ile Gly Gly Ser Ser Gly
           195                 200                 205
   Ser Gly Gly Ser Ile Ser Gly Ile Ala Asp Thr Gly Thr Thr Leu Leu
       210                 215                 220
   Leu Leu Asp Asp Gln Ile Val Asn Glu Tyr Tyr Gln Gln Val Gln Gly
   225                 230                 235                 240
   Ala Gln Asn Asp Gln Asn Ala Gly Gly Tyr Thr Phe Pro Cys Asp Ala
                   245                 250                 255
   Gln Leu Pro Glu Leu Ser Phe Thr Ile Gly Gln Tyr Thr Ala Thr Val
               260                 265                 270
   Pro Ala Glu Tyr Leu Asn Phe Gln Pro Val Ser Gln Gly Ser Gln Thr
           275                 280                 285
   Cys Phe Gly Gly Leu Gln Ser Asn Gln Gly Ile Gly Phe Ser Ile Phe
       290                 295                 300
   Gly Asp Val Phe Leu Lys Ser Gln Tyr Val Val Phe Asp Ser Asp Gly
   305                 310                 315                 320
   Pro Gln Leu Gly Phe Ala Ala Gln Ala
                   325
<210> 22
   <211> 326
   <212> PRT
   <213> Talaromyces cellulolyticus
<400> 22
   Ala Ala Ala Ser Ala Gln Ser Gly Ser Ala Thr Asn Gln Pro Thr Gln
   1               5                   10                  15
   Asp Asp Glu Glu Tyr Val Thr Pro Val Thr Ala Gly Asp Ser Thr Leu
               20                  25                  30
   Asn Leu Asp Phe Asp Thr Gly Ser Ala Asp Leu Trp Thr Tyr Ser Ser
           35                  40                  45
   Ser Thr Lys Gly Val Gly Ser His Asn Thr Tyr Asp Thr Ser Thr Gly
       50                  55                  60
   Thr Lys Val Ser Gly Ala Thr Trp Gln Ile Ser Tyr Gly Asp Gly Ser
   65                  70                  75                  80
   Ser Ala Ser Gly Asn Val Tyr Lys Asp Thr Val Val Val Gly Gly Val
                   85                  90                  95
   Thr Ala Thr Ser Gln Ala Val Glu Val Ala Thr Thr Val Ser Ser Glu
               100                 105                 110
   Phe Ser Gln Asp Thr Ser Asn Asp Gly Leu Leu Gly Leu Ala Phe Ser
           115                 120                 125
   Ser Ile Asn Thr Val Ser Pro Thr Gln Gln Lys Thr Phe Tyr Asp Asn
       130                 135                 140
   Val Lys Ser Ser Leu Asp Thr Pro Val Phe Ala Val Thr Leu Lys His
   145                 150                 155                 160
   Gln Ala Pro Gly Thr Tyr Asp Phe Gly Phe Ile Asp Asp Ser Lys Tyr
                   165                 170                 175
   Thr Gly Ser Leu Thr Tyr Thr Ser Val Asp Asn Ser Gln Gly Phe Trp
               180                 185                 190
   Gln Phe Thr Ala Asp Gly Tyr Ser Val Gly Ser Ser Gly Ser Gly Gly
           195                 200                 205
   Ser Ser Phe Ser Ala Ile Ala Asp Thr Gly Thr Thr Leu Val Leu Leu
       210                 215                 220
   Asp Asp Ser Ile Val Ser Asp Tyr Tyr Ser Gln Val Asp Gly Ala Glu
   225                 230                 235                 240
   Asn Ser Ser Ser Asp Gly Gly Tyr Val Phe Asp Cys Ser Ala Thr Leu
                   245                 250                 255
   Pro Asp Phe Thr Val Gln Ile Gly Asp Tyr Ser Ala Val Ile Pro Gly
               260                 265                 270
   Asp Tyr Ile Asn Tyr Ala Ser Thr Gly Ser Gly Ser Asn Cys Phe Gly
           275                 280                 285
   Gly Ile Gln Ser Asn Ser Gly Ile Gly Phe Ser Ile Leu Gly Asp Val
       290                 295                 300
   Phe Leu Lys Ser Gln Tyr Val Val Phe Asp Gly Asp Asn Leu Gln Leu
   305                 310                 315                 320
   Gly Phe Ala Pro Gln Ala
                   325